(19) Europäisches Patentamt
European Patent Office
Office européen des brevets

(11) EP 3 104 426 B1

(12) # EUROPEAN PATENT SPECIFICATION

(45) Date of publication and mention of the grant of the patent: **16.08.2023 Bulletin 2023/33**

(21) Application number: **15746426.4**

(22) Date of filing: **30.01.2015**

(51) International Patent Classification (IPC):
***C07D 493/04*** (2006.01) ***C07D 495/04*** (2006.01)
***H10K 85/10*** (2023.01) ***H10K 85/60*** (2023.01)
*H10K 10/46* (2023.01)

(52) Cooperative Patent Classification (CPC):
**C07D 493/04; C07D 495/04; C07F 7/08; C07F 7/0838; H10K 85/6574; H10K 85/6576;**
H10K 10/484

(86) International application number:
**PCT/JP2015/052604**

(87) International publication number:
**WO 2015/119045 (13.08.2015 Gazette 2015/32)**

(54) **ORGANIC TRANSISTOR, COMPOUND, ORGANIC SEMICONDUCTOR MATERIAL FOR NON-LUMINESCENT ORGANIC SEMICONDUCTOR DEVICE, MATERIAL FOR ORGANIC TRANSISTOR, COATING SOLUTION FOR NON-LUMINESCENT ORGANIC SEMICONDUCTOR DEVICE, AND ORGANIC SEMICONDUCTOR FILM FOR NON-LUMINESCENT ORGANIC SEMICONDUCTOR DEVICE**

ORGANISCHER TRANSISTOR, VERBINDUNG, ORGANISCHES HALBLEITERMATERIAL EINES NICHTLUMINESZENTEN ORGANISCHEN HALBLEITERBAUELEMENTS, MATERIAL FÜR EINEN ORGANISCHEN TRANSISTOR, BESCHICHTUNGSLÖSUNG FÜR NICHTLUMINESZENTES ORGANISCHES HALBLEITERBAUELEMENT UND ORGANISCHE HALBLEITERFOLIE EINES NICHTLUMINESZENTEN ORGANISCHEN HALBLEITERBAUELEMENTS

TRANSISTOR ORGANIQUE, COMPOSÉ, MATÉRIAU SEMICONDUCTEUR ORGANIQUE POUR DISPOSITIF SEMICONDUCTEUR ORGANIQUE NON LUMINESCENT, MATÉRIAU POUR TRANSISTOR ORGANIQUE, SOLUTION D'ENDUCTION POUR DISPOSITIF SEMICONDUCTEUR ORGANIQUE NON LUMINESCENT, ET FILM SEMICONDUCTEUR ORGANIQUE POUR DISPOSITIF SEMICONDUCTEUR ORGANIQUE NON LUMINESCENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.02.2014 JP 2014021329**

(43) Date of publication of application:
**14.12.2016 Bulletin 2016/50**

(73) Proprietor: **FUJIFILM Corporation**
**Tokyo 106-8620 (JP)**

(72) Inventors:
- **HIRAI Yuki**
**Ashigarakami-gun**
**Kanagawa 258-8577 (JP)**
- **MASUI Kensuke**
**Ashigarakami-gun**
**Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 251 342** **EP-A1- 2 301 926**
**JP-A- 2008 545 630** **JP-A- 2009 190 999**
**US-A1- 2008 220 285** **US-A1- 2012 138 915**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- MASAKI SHIMIZU ET AL: "Palladium-Catalyzed Annulation of vic-Bis(pinacolatoboryl)alkenes and -phenanthrenes with 2,2'-Dibromobiaryls: Facile Synthesis of Functionalized Phenanthrenes and Dibenzo[g,p]chrysenes", ANGEWANDTE CHEMIE. INTERNATIONAL EDITION, VCH VERLAG, WEINHEIM, DE, vol. 47, no. 42, 6 October 2008 (2008-10-06), pages 8096-8099, XP002664293, ISSN: 0570-0833, DOI: 10.1002/ANIE.200803213 [retrieved on 2008-09-15]
- WEIFENG ZHANG ET AL.: 'Novel Butterfly-Shaped Fused Heteroacenes: Synthesis, Properties, and Device Performance of Solution-Processed Field- Effect Transistors' ORGANIC LETTERS vol. 14, no. 17, 21 August 2012, pages 4382 - 4385, XP055219005

## Description

### BACKGROUND OF THE INVENTION

1. Field of the Invention

The present invention relates to an organic transistor, a compound, an organic semiconductor material for a non-light-emitting organic semiconductor device, a material for an organic transistor, a coating solution for a non-light-emitting organic semiconductor device, an organic semiconductor film for a non-light-emitting organic semiconductor device, and the like. Specifically, the present invention relates to a compound having a condensed cyclic skeleton including at least two heterocyclic rings, a compound which is an intermediate compound of the above compound, an organic transistor, an organic semiconductor material for a non-light-emitting organic semiconductor device, a material for an organic transistor, a coating solution for a non-light-emitting organic semiconductor device, and an organic semiconductor film for a non-light-emitting organic semiconductor device.

2. Description of the Related Art

The devices using organic semiconductor materials are drawing great attention because they are expected to be superior in various aspects to the devices using inorganic semiconductor materials of the related art such as silicon. Examples of the devices using organic semiconductor materials include a photoelectric conversion element such as an organic thin film solar cell or a solid-state imaging element using organic semiconductor materials as photoelectric conversion materials, an organic transistor (referred to as an organic thin-film transistor in some cases) having non-light-emitting properties (in the present specification, "non-light-emitting" refers to properties by which a luminous efficiency of equal to or less than 1 lm/W is obtained in a case where electric currents are applied to a device at a current density of 0.1 $mW/cm^2$ at room temperature in the atmosphere; non-light-emitting organic semiconductor devices mean organic semiconductor devices excluding light-emitting organic semiconductor devices such as organic electroluminescence elements), and the like. The devices using organic semiconductor materials are likely to make it possible to prepare large area elements at lower temperature and lower costs compared to the devices using inorganic semiconductor materials. Furthermore, the characteristics of the materials can be easily changed by varying the molecular structure thereof. Therefore, the materials show a wide variation and can realize functions or elements that cannot be obtained by inorganic semiconductor materials.

For example, EP2251342A1 describes that if a polymer using a low-molecular weight compound such as a benzobisbenzothiophene derivative is used in an organic transistor, sufficient charge transport properties can be exhibited, and the compound exhibits excellent solubility in a solvent. However, EP2251342A1 describes neither the specific structure of the low-molecular weight compound such as a benzobisbenzothiophene derivative nor the intermediate thereof.

EP2301926A1 and EP2301921A1 describe that if a benzobisbenzothiophene derivative or the like is used as a material of an organic electro-luminescence (organic EL) element, luminescence efficiency becomes high, and the element has long service life. However, in EP2301926A1 and EP2301921A1, an investigation into the application of the benzobisbenzothiophene derivative or the like to an organic transistor is not carried out.

KR10-2012-0120886A describes that if a benzobisbenzothiophene derivative or the like is used in an organic EL element or an organic photoelectric conversion element, luminescence efficiency becomes high, driving voltage is lowered, and color is excellently developed. However, in KR10-2012-0120886A, an investigation into the application of the benzobisbenzothiophene derivative or the like to an organic transistor is not carried out.

Tetrahedron Lett., 1973 (17), 1561 discloses dimethyl-substituted benzobisbenzothiophene. However, in the document, only the generation of the compound by a photoreaction is confirmed, and the application of the compound to an organic transistor is not described.

Rao, Tilak, Journal of Scientific and Industrial Research, 1958, vol. 17 B, pp. 260-265 and Kudo, Hirotaka; Tedjamulia, Marvin L.; Castle, Raymond N.; Lee, Milton L., Journal of Heterocyclic Chemistry, 1984, vol. 21, pp. 185-192 disclose unsubstituted benzobisbenzothiophene.

US2008220285 and US2012138915 describe organic field-effect transistors comprising a benzobisbenzothiophene derivative or a benzobisbenzofuran derivative.

### SUMMARY OF THE INVENTION

Being useful as an organic EL element material does not mean being useful as a semiconductor material for an organic transistor, because the characteristics required for the organic compound vary between the organic EL element and the organic transistor. In the organic EL element, charges need to be transported in the film thickness

direction (generally, several nanometers to hundreds of nanometers) of a general film. In contrast, in the organic transistor, charges (carriers) need to be transported a long distance between electrodes (generally, several micrometers to hundreds of micrometers) in the film plane direction, and hence extremely high carrier mobility is required. Accordingly, as the semiconductor material for an organic transistor, an organic compound showing highly ordered molecular arrangement and having high crystallinity is required. Furthermore, for the expression of high carrier mobility, the $\pi$-conjugate plane thereof is preferably upright against a substrate. On the other hand, for the organic EL element, in order to improve the luminous efficiency, an element having high luminous efficiency and uniformly emitting light in plane is required. Generally, an organic compound having high crystallinity causes luminescence defectiveness such as nonuniform in-plane electric field intensity, nonuniform luminescence, and quenching of luminescence. Therefore, as the material for an organic EL element, those having low crystallinity but having high amorphousness are desirable. Consequently, the use of the organic compound constituting the organic EL element material as an organic semiconductor material does not ensure that excellent transistor characteristics can be obtained.

In addition, likewise, being useful as an organic photoelectric conversion element does not mean being useful as a semiconductor material for an organic transistor for which extremely high carrier mobility is required.

Under the circumstances described above, the inventors of the present invention conducted an investigation into the use of the compounds described in the aforementioned documents into a semiconductor active layer of an organic transistor. As a result, the inventors found that the compounds show low carrier mobility in most cases, exhibit low solubility in an organic solvent, and do not enable the formation of the semiconductor active layer of the organic transistor by a solution film formation method.

Therefore, in order to solve the aforementioned problems of the related art, the inventors of the present invention continued the investigation. An object of the present invention is to provide an organic transistor using a compound which results in high carrier mobility when being used in a semiconductor active layer of an organic transistor and exhibits high solubility in an organic solvent.

As a result of conducting thorough investigation for achieving the above objects, the inventors of the present invention obtained knowledge that by investigating various types of substituents for a condensed cyclic skeleton, that is, a mother nucleus, substituting the mother nucleus with at least one of the substituents represented by Formula (W) in particular, and limiting the substituents to specific substituents in a case where substituents ($R^5$ and $R^6$ in Formula (1) of the present invention) are present in a short axis direction of a molecule, a compound is obtained which results in high carrier mobility when being used in a semiconductor active layer of an organic transistor and exhibits high solubility in an organic solvent. Based on the knowledge, the inventors accomplished the present invention.

The present invention as specific means for achieving the aforementioned object is constituted as below.

[1] An organic transistor comprising a compound represented by the following Formula (1) in a semiconductor active layer wherein said compound is a polymer compound having a repeating structure which is a $\pi$-conjugated polymer having a repeating structure in which the compound represented by Formula (1) represents at least one or more arylene groups or heteroarylene groups, or a pendant-type polymer in which the compound represented by Formula (1) is bonded to the main chain of the polymer via the side chain:

Formula (1)

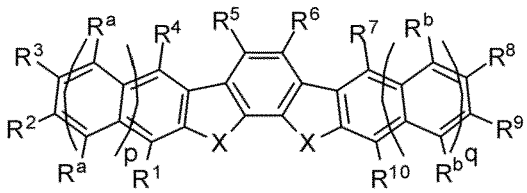

in Formula (1),

each X independently represents an O atom, a S atom, or a Se atom,

each of p and q independently represents an integer of 0 to 2,

each of $R^1$ to $R^{10}$, $R^a$, and $R^b$ independently represents a hydrogen atom, a halogen atom, or a substituent represented by the following Formula (W), at least one of $R^1$, $R^2$, $R^3$, $R^4$, $R^5$, $R^6$, $R^7$, $R^8$, $R^9$, $R^{10}$, $R^a$, or $R^b$ is a substituent represented by Formula (W), and in a case where at least one of $R^5$ or $R^6$ is a substituent represented by Formula (W), L in Formula (W) represented by $R^5$ and $R^6$ is a divalent linking group represented by the following Formula (L-2) or (L-3);

-L-R Formula (W)

in Formula (W),
L represents a divalent linking group represented by any one of the following Formulae (L-1) to (L-5), (L-13), (L-17) and (L-18) or a divalent linking group in which two or more divalent linking groups represented by any one of the following Formulae (L-1) to (L-5), (L-13), (L-17) and (L-18) are bonded to each other, and
R represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group;

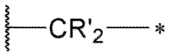
(L-1)

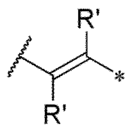
(L-2)

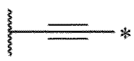
(L-3)

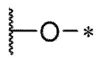
(L-4)

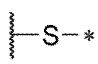
(L-5)

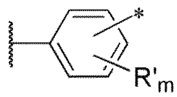
(L-13)

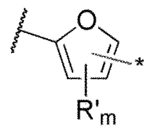
(L-17)

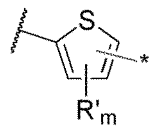
(L-18)

in Formulae (L-1) to (L-5), (L-13), (L-17) and (L-18),
the portion of a wavy line represents a position of bonding to a condensed cyclic skeleton,
m in Formula (L-13) represents 4, m in Formulae (L-17) to (L-18) represents 2,
each R' in Formulae (L-1), (L-2), (L-13), (L-17) and (L-18) independently represents a hydrogen atom or a substituent as defined in claim 1, and in a case where p=q=0 and a substituent represented by Formula (W) is an alkyl group, the substituent represented by Formula (W) is a linear alkyl group having 4 to 18 carbon atoms or a branched alkyl group having 4 or more carbon atoms, and,
* represents a position of bonding to any portion of a wavy line and
R in a divalent linking group represented by any of the Formulas (L-1) to (L-5), (L-13), (L-17) and (L-18); in a case where L represents a divalent linking group in which two or more divalent linking groups represented by any one of Formulae (L-1) to (L-5), (L-13), (L-17) and (L-18) are bonded to each other, * represents a position of bonding to the portion of a wavy line of a divalent linking group represented by any of Formulae (L-1) to (L-5), (L-13), (L-17) and (L-18).

Preferred features are as described in the dependent claims.

According to the present invention, it is possible to provide an organic transistor using a compound which results in high carrier mobility when being used in a semiconductor active layer of the organic transistor and exhibits high solubility in an organic solvent.

**BRIEF DESCRIPTION OF THE DRAWINGS**

Fig. 1 is a schematic view showing a section of an exemplary structure of an organic transistor of the present invention.
Fig. 2 is a schematic view showing a section of a structure of the organic transistor manufactured as a substrate for measuring FET characteristics in examples of the present invention.

## DESCRIPTION OF THE PREFERRED EMBODIMENTS

Hereinafter, the present invention will be specifically described. The constituents described below will be explained based on representative embodiments or specific examples, but the present invention is not limited to the embodiments. In the present specification, a range of numerical values described using "to" means a range including the numerical values listed before and after "to" as a lower limit and an upper limit respectively.

In the present invention, unless otherwise specified, a hydrogen atom used in the description of each formula represents a hydrogen atom including an isotope (deuterium atom or the like). Furthermore, an atom constituting a substituent represents an atom including an isotope thereof.

[Organic transistor]

An organic transistor of the present invention contains a compound represented by the following Formula (1) in a semiconductor active layer.

Formula (1)

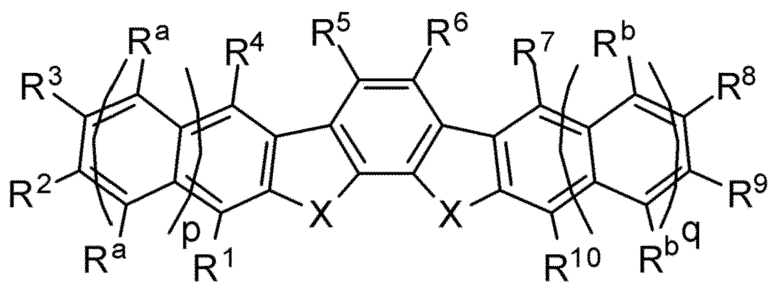

in Formula (1),

each X independently represents an O atom, a S atom, or a Se atom,

each of p and q independently represents an integer of 0 to 2,

each of $R^1$ to $R^{10}$, $R^a$, and $R^b$ independently represents a hydrogen atom, a halogen atom, or a substituent represented by the following Formula (W), at least one of $R^1$, $R^2$, $R^3$, $R^4$, $R^5$, $R^6$, $R^7$, $R^8$, $R^9$, $R^{10}$, $R^a$, or $R^b$ is a substituent represented by Formula (W), and in a case where at least one of $R^5$ or $R^6$ is a substituent represented by Formula (W), L in Formula (W) represented by $R^5$ and $R^6$ is a divalent linking group represented by the following Formula (L-2) or (L-3);

-L-R Formula (W)

in Formula (W),

L represents a divalent linking group represented by any one of the following Formulae (L-1) to (L-5), (L-13), (L-17) and (L-18) or a divalent linking group in which two or more divalent linking groups represented by any one of the following Formulae (L-1) to (L-5), (L-13), (L-17) and (L-18) are bonded to each other, and

R represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group;

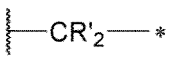
(L-1)

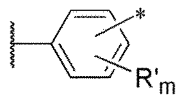
(L-13)

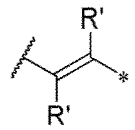
(L-2)

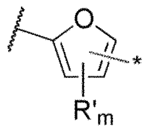
(L-17)

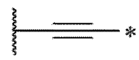
(L-3)

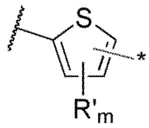
(L-18)

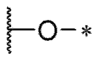
(L-4)

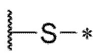
(L-5)

in Formulae (L-1) to (L-5), (L-13), (L-17) and (L-18),
the portion of a wavy line represents a position of bonding to a condensed cyclic skeleton,
m in Formula (L-13) represents 4, m in Formulae (L-17) to (L-18) represents 2,
each R' in Formulae (L-1), (L-2), (L-13) and (L-17) and (L-18) independently represents a hydrogen atom or a substituent as defined in claim

The compound represented by Formula (1) having the structure described above exhibits high solubility in an organic solvent. By containing the compound represented by Formula (1) in a semiconductor active layer, the organic transistor of the present invention has high carrier mobility.

Herein, EP2251342A1 (or JP2009-190999A) mainly focuses on a polymer having the skeleton of a benzobisbenzothiophene derivative or the like as a constitutional unit. However, EP2251342A1 does not have descriptions or examples showing the specific structure of the low-molecular weight compound. Furthermore, in Claim 1 of EP2251342A1, a compound is described which essentially has at least one substituent having a specific structure (an alkyl group, an alkoxy group, an alkylthio group, an alkylamino group, an alkoxycarbonyl group in which an alkyl moiety has 3 or more carbon atoms, an aryl group which may have a substituent, or a monovalent heterocyclic group or cyano group which may have a substituent) in a short axis direction of the molecule ($R^{11}$ and $R^{12}$ in Formula (1) of EP2251342A1). However, in such a compound, the intermolecular distance increases when the compound is crystallized, and thus overlapping of HOMO does not sufficiently occur between molecules. In addition, although Example 4 of EP2251342A1 describes the use of such a compound in an organic transistor, it does not disclose the results obtained by actually measuring carrier mobility. According to the investigation conducted by the inventors of the present invention, with the low-molecular weight compound described in EP2251342A1, it is difficult to obtain sufficient transistor characteristics, that is, high carrier mobility.

EP2301926A1 mainly focuses on a polycyclic condensed compound for an organic EL element, and does not describe a case where the compound described in the examples of EP2301926A1 is used in an organic transistor. Even if the compound is used, because the compound is an OLED material supposed to be subjected to a vapor deposition process, the solubility thereof in a general organic solvent would be low. In addition, according to the investigation conducted by the inventors of the present invention, carrier mobility of the compound is low.

In contrast, regarding the compound represented by Formula (1), by investigating various types of substituents for a condensed cyclic skeleton, that is, a mother nucleus such that overlapping of HOMO sufficiently occurs between molecules at the time of crystallization, substituting the compound with at least one of the groups represented by Formula (W) in particular, and limiting the substituents to specific substituents in a case where the substituents ($R^5$ and $R^6$ Formula (1) of the present invention) are present in a short axis direction of the molecule, the compound results in high carrier mobility when being used in a semiconductor active layer of an organic transistor and exhibits high solubility in an organic solvent. In order to improve the solubility in a general organic solvent, it is effective to introduce a group represented by Formula (W) into the compound, and in this way, the accomplishment of both of high mobility and high solubility that has been difficult so far can be realized.

It is preferable that the organic transistor of the present invention using the compound represented by Formula (1) shows only a slight threshold voltage shift after repeated driving. In order to reduce the threshold voltage shift after repeated driving, HOMO of the organic semiconductor material needs not to be too shallow or too deep. Furthermore, the chemical stability (particularly, resistance against air oxidation and stability against oxidation and reduction) of the organic semiconductor material, the heat stability of the film state, the high film density which makes it difficult for air or moisture to permeate the film, the film quality by which the film has small defectiveness such that charge accumulation

does not easily occur, and the like are required. It is considered that because the compound represented by Formula (1) satisfies the aforementioned requirements, the organic transistor shows only a slight threshold voltage shift after repeated driving. That is, in the organic transistor showing only a slight threshold voltage shift after repeated driving, the semiconductor active layer has high chemical stability, high film density, and the like, and thus the organic transistor can effectively function as a transistor over a long period of time.

Hereinafter, preferred aspects of the compound of the present invention, the organic transistor of the present invention, and the like will be described.

<Compound represented by Formula (1)>

The organic transistor of the present invention contains the compound represented by Formula (1) in a semiconductor active layer which will be described later. Among compounds represented by Formula (1), a compound represented by Formula (2-1) which will be described later and a compound represented by Formula (2-2) which will be described later are novel compounds. The compound represented by Formula (2-1) and the compound represented by Formula (2-2) are collectively called the compound of the present invention. The compound represented by Formula (1), particularly, the compound of the present invention is contained in a semiconductor active layer, which will be described later, in the organic transistor of the present invention. That is, the compound represented by Formula (1), particularly, the compound of the present invention can be used as a material for an organic transistor.

Formula (1)

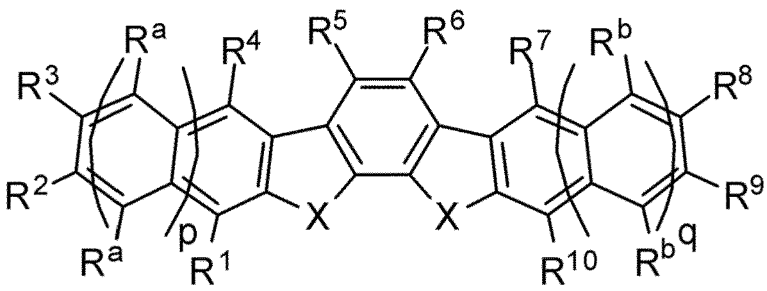

In Formula (1), each X independently represents an O atom, a S atom, or a Se atom. each X is preferably independently an O atom or a S atom. From the viewpoint of improving the carrier mobility, it is more preferable that at least one of X's is a S atom. X's are preferably the same linking group. It is particularly preferable that all of X's are a S atom.

In Formula (1), each of p and q independently represents an integer of 0 to 2. From the viewpoint of accomplishing both of mobility and solubility, it is preferable that each of p and q is 0 or 1. It is more preferable that p and q satisfy p = q = 0 or p = q = 1.

In Formula (1), each of $R^1$ to $R^{10}$, $R^a$, and $R^b$ independently represents a hydrogen atom, a halogen atom, or a substituent represented by the following Formula (W), at least one of $R^1$, $R^2$, $R^3$, $R^4$, $R^5$, $R^6$, $R^7$, $R^8$, $R^9$, $R^{10}$, $R^a$, or $R^b$ is a substituent represented by Formula (W), and in a case where at least one of $R^5$ or $R^6$ is a substituent represented by Formula (W), L in Formula (w) represented by $R^5$ and $R^6$ is a divalent linking group represented by the following Formula (L-2) or (L-3);

-L-R          Formula (W)

in Formula (W),

L represents a divalent linking group represented by any one of the following Formulae (L-1) to (L-5), (L-13), (L-17) and (L-18) or a divalent linking group in which two or more divalent linking groups represented by any one of the following Formulae (L-1) to (L-5), (L-13), (L-17) and (L-18) are bonded to each other, and

R represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group;

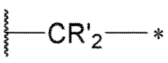 (L-1)

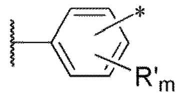 (L-13)

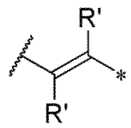 (L-2)

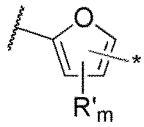 (L-17)

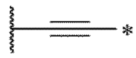 (L-3)

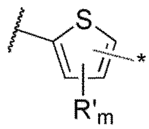 (L-18)

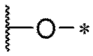 (L-4)

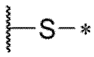 (L-5)

in Formulae (L-1) to (L-5), (L-13), (L-17) and (L-18),
the portion of a wavy line represents a position of bonding to a condensed cyclic skeleton,
m in Formula (L-13) represents 4, m in Formulae (L-17) to (L-18) represents 2,
each R' in Formulae (L-1), (L-2), (L-6), and (L-13), (L-17) to (L-18) independently represents a hydrogen atom or a substituent as defined in claim 1.

In Formula (1), each of $R^1$ to $R^{10}$, $R^a$, and $R^b$ independently represents a hydrogen atom, a halogen atom, or a substituent represented by the following Formula (W), at least one of $R^1$, $R^2$, $R^3$, $R^4$, $R^5$, $R^6$, $R^7$, $R^8$, $R^9$, $R^{10}$, $R^a$, and $R^b$ represents a group represented by Formula (W), and in a case where at least one of $R^5$ or $R^6$ is a substituent represented by Formula (W), L in Formula (W) represented by $R^5$ and $R^6$ is a divalent linking group represented by the following Formula (L-2) or (L-3).

The case where at least one of $R^5$ or $R^6$ is a substituent represented by Formula (W) corresponds to a case where none of $R^5$ and $R^6$ are a hydrogen atom or a halogen atom.

In a case where at least one of $R^5$ or $R^6$ is a substituent represented by Formula (W), L in Formula (W) represented by $R^5$ and $R^6$ is preferably a divalent linking group represented by the following Formula (L-3).

In a case where at least one of $R^5$ or $R^6$ is a substituent represented by Formula (W), both of $R^5$ and $R^6$ are preferably a substituent represented by Formula (W).

In a case where both of $R^5$ and $R^6$ are a hydrogen atom or a halogen atom, each of $R^1$ to $R^4$, $R^7$ to $R^{10}$, $R^a$, and $R^b$ is a hydrogen atom, a halogen atom, or a substituent represented by Formula (W), and at least one or more of $R^1$, $R^2$, $R^3$, $R^4$, $R^7$, $R^8$, $R^9$, $R^{10}$, $R^a$, or $R^b$ are a substituent represented by Formula (W).

In Formula (1), examples of the halogen atom represented by $R^1$ to $R^{10}$, $R^a$, and $R^b$ include a fluorine atom, a chlorine atom, a bromine atom, and an iodine atom. The halogen atom is preferably a fluorine atom, a chlorine atom, or a bromine atom, more preferably a fluorine atom or a chlorine atom, and particularly preferably a fluorine atom.

In the compound represented by Formula (1), the number of halogen atoms represented by $R^1$ to $R^{10}$, $R^a$, and $R^b$ is preferably 0 to 4, more preferably 0 to 2, particularly preferably 0 or 1, and more particularly preferably 0.

The group represented by Formula (W) that $R^1$ to $R^{10}$, $R^a$, and $R^b$ in Formula (1) represent will be described.

-L-R　　　Formula (W)

in Formula (W), L represents a divalent linking group represented by any one of the following Formulae (L-1) to (L-5), (L-13), (L-17) and (L-18) or a divalent linking group in which two or more divalent linking groups represented by any one of the following Formulae (L-1) to (L-5), (L-13), (L-17) and (L-18) are bonded to each other, and
R represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group:

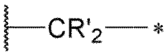 (L-1)

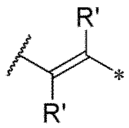 (L-2)

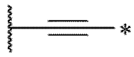 (L-3)

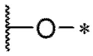 (L-4)

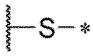 (L-5)

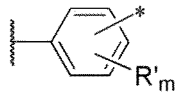 (L-13)

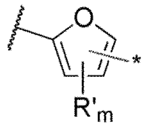 (L-17)

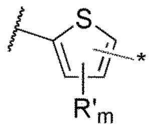 (L-18)

in Formulae (L-1) to (L-5), (L-13), (L-17) and (L-18), the portion of a wavy line represents a position of bonding to a condensed cyclic skeleton,

m in Formula (L-13) represents 4, m in Formulae (L-17) to (L-18) represents 2,

each R' in Formulae (L-1), (L-2), (L-13) and (L-17) to (L-18) independently represents a hydrogen atom or a substituent as defined below.

In Formula (W), L is a divalent linking group represented by any one of the following Formulae (L-1) to (L-5), (L-13), (L-17) and (L-18) or a divalent linking group in which two or more divalent linking groups represented by any of the following Formulae (L-1) to (L-5), (L-13), (L-17) and (L-18) are bonded to each other.

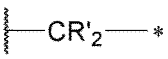 (L-1)

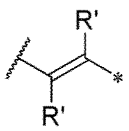 (L-2)

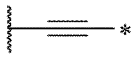 (L-3)

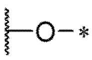 (L-4)

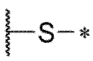 (L-5)

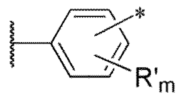 (L-13)

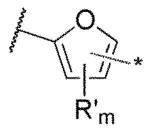 (L-17)

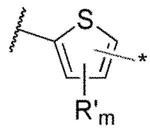 (L-18)

In Formulae (L-1) to (L-5), (L-13), (L-17) and (L-18), the portion of a wavy line represents a position of bonding to a condensed cyclic skeleton. Here, in a case where L represents a divalent linking group in which two or more divalent linking groups represented by any one of Formulae (L-1) to (L-5), (L-13), (L-17) and (L-18) are bonded to each other, the portion of a wavy line represents a position of bonding to * of a divalent linking group represented by any of Formulae (L-1) to (L-5), (L-13), (L-17) and (L-18).

In Formulae (L-1) to (L-5), (L-13), (L-17) and (L-18), * represents a position of bonding to any one of the portion of a wavy line and R in a divalent linking group represented by any of Formulae (L-1) to (L-5), (L-13), (L-17) and (L-18).

m in Formula (L-13) represents 4, m in Formulae (L-17) to (L-18) represents 2,

Each R' in Formulae (L-1), (L-2), (L-13) and (L-17) to (L-18) independently represents a hydrogen atom or a substituent.

In Formulae (L-1) to (L-2), each R' may form a condensed ring by being bonded to R adjacent to L.

A divalent linking group represented by any of Formulae (L-17) to (L-18) is more preferably a divalent linking group represented by any of the following Formulae (L-17A) to (L-18A).

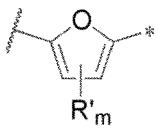

(L-17A)

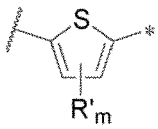

(L-18A)

In a case where a substituted or unsubstituted alkyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group is present on the terminal of the substituent represented by Formula (W), the substituent can be interpreted as either a substituent consisting of only -R in Formula (W) or a substituent consisting of -R-L in Formula (W).

In the present invention, in a case where a substituted or unsubstituted alkyl group whose main chain consists of N carbon atoms is present on the terminal of the substituent represented by Formula (W), the substituent is interpreted as -L-R in Formula (W) by including as much linking groups represented by -CRR2- as possible in R from the terminal of the substituent represented by Formula (W), and is not interpreted as a substituent composed solely of -R. Herein, $R^R$ represents a hydrogen atom or a substituent. Specifically, the substituent is interpreted as a substituent in which "one (L-1) corresponding to L in Formula (W)" is bonded to "a substituted or unsubstituted alkyl group corresponding to R in Formula (W) having a main chain consisting of (N - 1) carbon atoms". For example, in a case where a n-octyl group as an alkyl group having 8 carbon atoms is present on the terminal of the substituent, the substituent is interpreted as a substituent in which one (L-1), wherein two R's are hydrogen atoms, is boned to a n-heptyl group having 7 carbon atoms. In a case where the substituent represented by Formula (W) is an alkyl group, for example, how to make a differentiation between L and R is as follows, and L represents a divalent linking group represented by Formula (L-1).

Main chain of terminal (N carbon atoms)

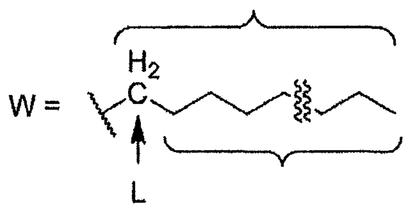

R(N - 1 carbon atoms)

In a case where the substituent represented by Formula (W) is an alkoxy group having 8 carbon atoms, the alkoxy group is interpreted as a substituent in which one linking group represented by Formula (L-4) that is -O-, one linking group represented by Formula (L-1) in which two R's are hydrogen atoms, and a n-heptyl group having 7 carbon atoms are bonded to each other. In a case where the substituent represented by Formula (W) is a group other than an alkyl group, how to make a differentiation between L and R is as follows, and L represents a divalent linking group in which "a divalent linking group other than the linking group represented by Formula (L-1)" and "a divalent linking group represented by Formula (L-1)" are bonded to each other.

Main chain of terminal (N carbon atoms)

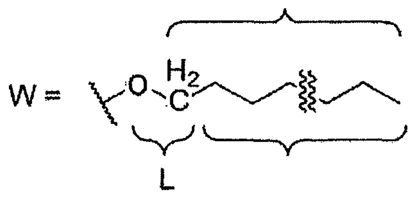

R (N - 1 carbon atoms)

In contrast, in the present invention, in a case where an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group is present on the terminal of the substituent represented by Formula (W), the substituent is interpreted as a substituent consisting solely of R in Formula (W) by including as much linking groups as possible in R from the terminal of the substituent represented by Formula (W), but is not interpreted as -L-R. For example, in a case where a $-(OCH_2CH_2)-(OCH_2CH_2)-OCH_3$ group is present on the terminal of the substituent, the substituent is interpreted as a substituent consisting of only an oligo-oxyethylene group in which the repetition number v of an oxyethylene unit is 2. In a case where an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group is present on the terminal of the substituent represented by Formula (W), how to make a differentiation between L and R is as follows, for example.

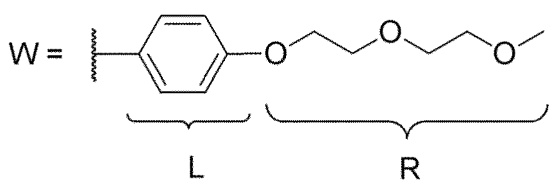

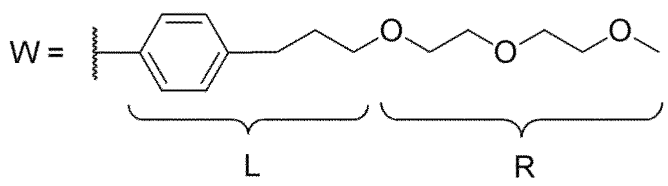

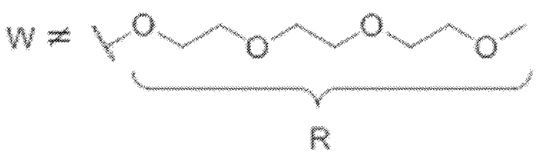

In a case where L forms a linking group in which divalent linking groups represented by any one of Formulae (L-1) to (L-5), (L-13), (L17) and (L-18) are bonded to each other, the number of the divalent linking groups bonded to each other represented by any one of Formulae (L-1) to (L-5), (L-13), (L17) and (L-18) is preferably 2 to 4, and more preferably 2 or 3.

The substituent R' in Formulae (L-1), (L-2), (L-13), and (L-17) to (L-18) includes a halogen atom, an alkyl group (including an alkyl group having 1 to 40 carbon atoms such as a methyl group, an ethyl group, a propyl group, a butyl group, a pentyl group, a hexyl group, a heptyl group, an octyl group, a nonyl group, a decyl group, an undecyl group, a dodecyl group, a tridecyl group, a tetradecyl group, or a pentadecyl group; here, the alkyl group also includes a 2,6-dimethyloctyl group, a 2-decyltetradecyl group, a 2-hexyldodecyl group, a 2-ethyloctyl group, a 2-decyltetradecyl group, a 2-butyldecyl group, a 1-octylnonyl group, a 2-ethyloctyl group, a 2-octyltetradecyl group, a 2-ethylhexyl group, a cycloalkyl group, a bicycloalkyl group, a tricycloalkyl group), an alkenyl group (including a 1-pentenyl group, a cycloalkenyl group, a bicycloalkenyl group), an alkynyl group (including a 1-pentynyl group, a trimethylsilylethynyl group, a triethylsilylethynyl group, a tri-i-propylsilylethynyl group, a 2-p-propylphenylethynyl group), an aryl group (including an aryl group having 6 to 20 carbon atoms such as a phenyl group, a naphthyl group, a p-pentylphenyl group, a 3,4-dipentylphenyl group, a p-heptoxyphenyl group, a 3,4-diheptoxyphenyl group), a hetero ring group (may be referred to as a heterocyclic group as well, including a 2-hexylfuranyl group), a cyano group, a hydroxyl group, a nitro group, an acyl group (including

a hexanoyl group, a benzoyl group ), an alkoxy group (including a butoxy group), an aryloxy group, a silyloxy group, a heterocyclic oxy group, an acyloxy group, a carbamoyloxy group, an amino group (including an anilino group), an acylamino group, an aminocarbonylamino group (including a ureide group), alkoxy- and aryloxycarbonylamino groups, alkyl- and aryl sulfonylamino groups, a mercapto group, alkyl- and arylthio groups (including a methylthio group, an octylthio group), a heterocyclic thio group, a sulfamoyl group, a sulfo group, alkyl- and aryl sulfinyl groups, alkyl- and aryl sulfonyl groups, alkyloxy- and aryloxycarbonyl groups, a carbamoyl group, aryl- and heterocyclic azo group, an imide group, a phosphino group, a phosphinyl group, a phosphinyloxy group, a phosphinylamino group, a phosphono group, a silyl group (a ditrimethylsiloxy methylbutoxy group), a hydrazino group, a ureide group, a boronic acid group ($-B(OH)_2$), a phosphate group ($-OPO(OH)_2$), a sulfate group ($-OSO_3H$).

These substituents may further have the above substituents. In addition, in a case where the compound represented by Formula (1) is a polymer compound having a repeating structure, each of $R^1$ to $R^8$ may have a group derived from a polymerizable group.

In a case where L includes divalent linking groups represented by Formulae (L-13), (L-17) to (L-18), any one of the divalent linking groups represented by Formulae (L-13), (L-17) to (L-18) is preferably bonded to R via the divalent linking group represented by Formula (L-1).

L is a divalent linking group represented by any one of Formulae (L-1) to (L-5), (L-13), (L-17), and (L-18) or a divalent linking group in which two or more divalent linking groups represented by any one of Formulae (L-1) to (L-5), (L-13), (L-17), and (L-18) are bonded to each other, preferably a divalent linking group represented by any one of Formulae (L-1) to (L-4) or a divalent linking group in which a divalent linking group represented by any one of Formulae (L-2) to (L-4) is bonded to a divalent linking group represented by Formula (L-1), and particularly preferably a divalent linking group represented by Formula (L-1). It is preferable that the divalent linking group represented by Formula (L-1) is bonded to the R side of a divalent linking group in which the divalent linking group represented by any one of Formulae (L-2) to (L-4) is bonded to the divalent linking group represented by Formula (L-1).

From the viewpoint of the chemical stability and the carrier transport properties, L is particularly preferably a divalent linking group including the divalent linking group represented by Formula (L-1), more particularly preferably a divalent linking group represented by Formula (L-1).

In Formula (W), R represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted silyl group.

In Formula (W), in a case where L adjacent to R is a divalent linking group represented by Formula (L-1), R is preferably a substituted or unsubstituted alkyl group, an oxyethylene group, an oligo-oxyethylene group in which a repetition number v of an oxyethylene unit is equal to or greater than 2, a siloxane group, or an oligosiloxane group having two or more silicon atoms, and more preferably a substituted or unsubstituted alkyl group.

In Formula (W), in a case where L adjacent to R is a divalent linking group represented by any of Formulae (L-2) and (L-4), (L-13), (L-17) or (L-18), R is more preferably a substituted or unsubstituted alkyl group.

In Formula (W), in a case where L adjacent to R is a divalent linking group represented by Formula (L-3), R is preferably a substituted or unsubstituted alkyl group or a substituted or unsubstituted silyl group.

In a case where R is a substituted or unsubstituted alkyl group, the number of carbon atoms thereof is preferably 4 to 18, more preferably 6 to 15 from the viewpoint of the chemical stability and the carrier transport properties, and even more preferably 6 to 12. If R is a long-chain alkyl group within the above range, the alkyl group is particularly preferably a long-chain linear alkyl group because then the linearity of the molecule is improved, and hence the carrier mobility can be improved.

In a case where R represents an alkyl group, the alkyl group may be a linear, branched, or cyclic alkyl group. However, the alkyl group is preferably a linear alkyl group because then the linearity of the molecule is improved, and hence the carrier mobility can be improved.

In contrast, from the viewpoint of improving the solubility in an organic solvent, R is preferably a branched alkyl group.

In a case where R is an alkyl group having a substituent, examples of the substituent include a halogen atom and the like (at this time, R becomes a haloalkyl group), and the halogen atom is preferably a fluorine atom. In a case where R is an alkyl group having a fluorine atom, a perfluoroalkyl group may be formed by the substitution of all of the hydrogen atoms with the fluorine atom. Here, R is preferably an unsubstituted alkyl group.

In the present specification, in a case where the R is an ethyleneoxy group or an oligo-ethyleneoxy group in which a repetition number v of an oxyethylene group is equal to or greater than 2, the "oligo-oxyethylene group" represented by R refers to a group represented by $-(OCH_2CH_2)_vOY$ (the repetition number v of an oxyethylene unit represents an integer of equal to or greater than 2, and Y on the terminal represents a hydrogen atom or a substituent). In a case where Y on the terminal of the oligo-oxyethylene group is a hydrogen atom, the terminal becomes a hydroxy group. The repetition number v of the oxyethylene unit is preferably 2 to 4, and more preferably 2 or 3. It is preferable that the

hydroxy group on the terminal of the oligo-oxyethylene group is sealed. That is, it is preferable that Y represents a substituent. In this case, the hydroxy group is preferably sealed with an alkyl group having 1 to 3 carbon atoms. In other words, Y is preferably an alkyl group having 1 to 3 carbon atoms, more preferably a methyl group or an ethyl group, and particularly preferably a methyl group.

In a case where R is a siloxane group or an oligosiloxane group having two or more silicon atoms, the repetition number of the siloxane unit is preferably 2 to 4, and more preferably 2 or 3. Furthermore, it is preferable that hydrogen atoms or alkyl groups are bonded to the Si atoms. In a case where alkyl groups are bonded to the Si atoms, the number of carbon atoms of each alkyl group is preferably 1 to 3. For example, it is preferable that methyl groups or ethyl groups are bonded to the Si atoms. The same alkyl groups may be bonded to the Si atoms, or different alkyl groups or hydrogen atoms may be bonded to the Si atoms. In addition, all of the siloxane units constituting the oligosiloxane group may be the same as or different from each other, but it is preferable that they are the same as each other.

In a case where L adjacent to R is a divalent linking group represented by Formula (L-3), R is preferably a substituted or unsubstituted silyl group. In a case where R is a substituted or unsubstituted silyl group, a substituted silyl group is preferable as R. The substituent of the silyl group is not particularly limited. However, the substituent is preferably a substituted or unsubstituted alkyl group, and more preferably a branched alkyl group. In a case where R is a trialkylsilyl group, the number of carbon atoms of each alkyl group bonded to the Si atoms is preferably 1 to 3. For example, it is preferable that methyl groups, ethyl groups, or isopropyl groups are bonded to the Si atoms. The same alkyl groups or different alkyl groups may be bonded to the Si atoms. In a case where R is a trialkylsilyl group further having a substituent on the alkyl group, the substituent is not particularly limited.

The total number of carbon atoms contained in the group represented by Formula (W), that is, the total number of carbon atoms contained in L and R is not particularly limited and can be 2 to 20, for example. However, the total number of carbon atoms is preferably 4 to 18. If the total number of carbon atoms contained in L and R is equal to or greater than the lower limit of the above range, the carrier mobility is improved, and the solubility is improved. If the total number of carbon atoms contained in L and R is equal to or less than the upper limit of the above range, the driving voltage is lowered.

The total number of carbon atoms contained in L and R is preferably 4 to 24, more preferably 6 to 21, and particularly preferably 6 to 18.

In the present invention, in a case where p = q = 0 in Formula (1), the total number of carbon atoms contained in the group represented by Formula (W) is preferably 4 to 24, more preferably 6 to 21, and particularly preferably 6 to 18.

Particularly, in a case where p = q = 0 in Formula (1), and the substituent represented by Formula (W) is an alkyl group, the substituent represented by Formula (W) is a linear alkyl group having 4 to 18 carbon atoms or a branched alkyl group having 4 or more carbon atoms, preferably a linear alkyl group having 4 to 14 carbon atoms or a branched alkyl group having 4 to 14 carbon atoms, particularly preferably a linear alkyl group having 5 to 14 carbon atoms or a branched alkyl group having 5 to 14 carbon atoms, more particularly preferably a linear alkyl group having 5 to 10 carbon atoms or a branched alkyl group having 6 to 12 carbon atoms, and even more particularly preferably a linear alkyl group having 6 to 10 obtained or a branched alkyl group having 8 to 12 carbon atoms.

In the present invention, in a case where p = q = 1 in Formula (1), the total number of carbon atoms contained in the group represented by Formula (W) is preferably 4 to 24, more preferably 4 to 21, particularly preferably 5 to 18, more particularly preferably 6 to 18, and even more particularly preferably 6 to 16.

Particularly, in a case where p = q = 1 in Formula (1), and the substituent represented by Formula (W) is an alkyl group, the substituent represented by Formula (W) is not particularly limited. However, the substituent is preferably a linear alkyl group having 4 to 18 carbon atoms or a branched alkyl group having 4 to 18 carbon atoms, more preferably a linear alkyl group having 4 to 14 carbon atoms or a branched alkyl group having 4 to 14 carbon atoms, particularly preferably a linear alkyl group having 5 to 12 carbon atoms or a branched alkyl group having 5 to 12 carbon atoms, more particularly preferably a linear alkyl group having 6 to 12 carbon atoms or a branched alkyl group having 6 to 12 carbon atoms, and even more particularly preferably a linear alkyl group having 6 to 10 carbon atoms or a branched alkyl group having 6 to 10 carbon atoms.

In a case where L includes divalent linking groups represented by Formulae (L-13), (L-17) to (L-18), R and L in Formula (W) are preferably combined such that R is an alkyl group, and L is any one of the divalent linking groups represented by Formulae (L-13), (L-17) to (L-18) and bonded to R via a divalent linking group represented by Formula (L-1).

Especially, R and L in Formula (W) are preferably combined such that L is a divalent linking group represented by any one of Formulae (L-1) to (L-5), (L-13), (L-17), and (L-18) or a divalent linking group in which two or more divalent linking groups represented by any one of Formulae (L-1) to (L-5), (L-13), (L-17), and (L-18) are bonded to each other, and R is a substituted or unsubstituted alkyl group; more preferably combined such that L is a divalent linking group represented by any one of Formulae (L-1) to (L-4) or a divalent linking group in which a divalent linking group represented by any one of Formulae (L-2) to (L-4) is bonded to a divalent linking group represented by Formula (L-1), and R is a substituted or unsubstituted alkyl group; and particularly preferably combined such that L is a divalent linking group

represented by Formula (L-1), and R is a substituted or unsubstituted alkyl group.

In the compound represented by Formula (1), the number of groups adopted as one of $R^1$ to $R^{10}$, $R^a$, and $R^b$ and represented by Formula (W) is preferably 1 to 4 from the viewpoint of improving the carrier mobility and the solubility in an organic solvent, more preferably 1 or 2, and particularly preferably 2.

The group represented by Formula (W) is positioned in any of $R^1$ to $R^{10}$, $R^a$, and $R^b$ without particular limitation. In the present invention, from the viewpoint of improving the carrier mobility and improving the solubility in an organic solvent, it is preferable that in Formula (1), each of $R^1$, $R^4$ to $R^7$, $R^{10}$, $R^a$, and $R^b$ is independently a hydrogen atom or a halogen atom, each of $R^2$, $R^3$, $R^8$, and $R^9$ is independently a hydrogen atom, a halogen atom, or a substituent represented by Formula (W), and at least one of $R^2$, $R^3$, $R^8$, or $R^9$ is a substituent represented by Formula (W).

In the present invention, it is more preferable that each of $R^1$, $R^3$ to $R^8$, and $R^{10}$ independently represents a hydrogen atom or a halogen atom, each of $R^2$ and $R^9$ is independently a hydrogen atom, a halogen atom, or a substituent represented by the following Formula (W), and at least one of $R^2$ or $R^9$ is a substituent represented by Formula (W).

In the present invention, it is particularly preferable that both of $R^2$ and $R^9$ are a substituent represented by Formula (W), and both of $R^3$ and $R^8$ are a hydrogen atom or a halogen atom. Alternatively, it is particularly preferable that both of $R^3$ and $R^8$ are a substituent represented by Formula (W), and both of $R^2$ and $R^9$ are a hydrogen atom or a halogen atom.

In the present invention, it is more particularly preferable that both of $R^2$ and $R^9$ are a substituent represented by Formula (W), and both of $R^3$ and $R^8$ are a hydrogen atom or a halogen atom. Alternatively, it is more particularly preferable that both of $R^3$ and $R^8$ are a substituent represented by Formula (W), and both of $R^2$ and $R^9$ are a hydrogen atom or a halogen atom.

In Formula (1), two or more groups represented by $R^1$ to $R^{10}$, $R^a$, and $R^b$ may or may not form a ring by being bonded to each other. However, it is preferable that they do not form a ring by being bonded to each other.

The compound represented by Formula (1) is preferably a compound represented by the following Formula (2-1) or (2-2), and particularly preferably a compound represented by Formula (2-1) from the viewpoint of accomplishing both the high carrier mobility and the high solubility.

First, a case where the compound represented by Formula (1) is a compound represented by the following Formula (2-1) will be described.

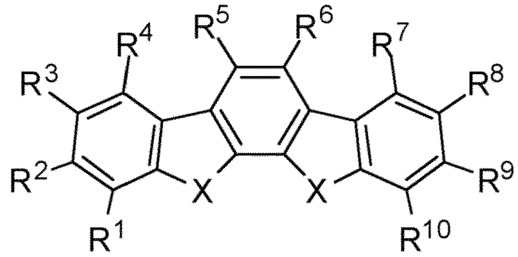

In Formula (2-1),

each of $R^1$, $R^3$ to $R^8$, and $R^{10}$ independently represents a hydrogen atom or a halogen atom,
each of $R^2$ and $R^9$ is independently a hydrogen atom, a halogen atom, or a substituent represented by the following Formula (W), at least one of $R^2$ or $R^9$ is a substituent represented by Formula (W), and in a case where the substituent represented by Formula (W) is an alkyl group, the substituent represented by Formula (W) is limited to a linear alkyl group having 4 to 18 carbon atoms or a branched alkyl group having 4 or more carbon atoms.

-L-R Formula (W)

in Formula (W) L and R are as defined herein.

In Formula (2-1), each X independently represents an O atom, a S atom, or a Se atom. The preferred range of X in Formula (2-1) is the same as the preferred range of X in Formula (1).

In Formula (2-1), each of $R^1$, $R^3$ to $R^8$, and $R^{10}$ independently represents a hydrogen atom or a halogen atom. The preferred range of $R^1$, $R^3$ to $R^8$, and $R^{10}$ in Formula (2-1) is the same as the preferred range of $R^1$, $R^3$ to $R^8$, and $R^{10}$ in Formula (1).

In Formula (2-1), each of $R^2$ and $R^9$ is independently s hydrogen atom, a halogen atom, or a substituent represented by Formula (W), and at least one of $R^2$ or $R^9$ is a substituent represented by Formula (W). Here, in a case where the substituent represented by Formula (W) is an alkyl group, the substituent represented by Formula (W) is limited to a linear alkyl group having 4 to 18 carbon atoms or a branched alkyl group having 4 or more carbon atoms. The preferred range of $R^2$ and $R^9$ in Formula (2-1) is the same as the preferred range of $R^2$ and $R^9$ in a case where p = q = 0 in Formula (1).

Next, a case where the compound represented by Formula (1) is a compound represented by the following Formula (2-2) will be described.

Formula (2-2)

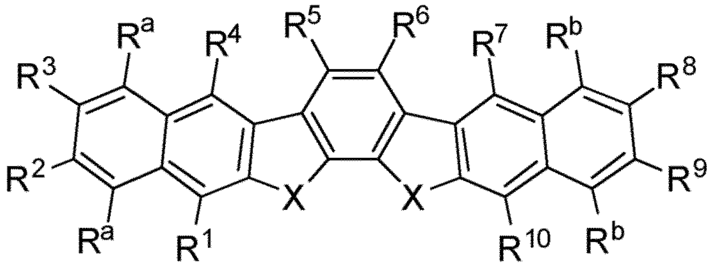

In Formula (2-2),

each X independently represents an O atom, a S atom, or a Se atom,
each of $R^1$, $R^4$ to $R^7$, $R^{10}$, $R^a$, and $R^b$ is independently a hydrogen atom or a halogen atom,
each of $R^2$, $R^3$, $R^8$, and $R^9$ independently represents a hydrogen atom, a halogen atom, or a substituent represented by the following Formula (W), and at least one of $R^2$, $R^3$, $R^8$, or $R^9$ represents a substituent represented by Formula (W).

-L-R Formula (W)

in Formula (W) L and R are as defined herein.

In Formula (2-2), each X independently represents an O atom, a S atom, or a Se atom. The preferred range of X in Formula (2-2) is the same as the preferred range of X in Formula (1).

In Formula (2-2), each of $R^1$, $R^4$ to $R^7$, $R^{10}$, $R^a$, and $R^b$ is independently a hydrogen atom or a halogen atom. The preferred range of $R^1$, $R^4$ to $R^7$, $R^{10}$, $R^a$, and $R^b$ in Formula (2-2) is the same as the preferred range of $R^1$, $R^4$ to $R^7$, $R^{10}$, $R^a$, and $R^b$ in Formula (1).

In Formula (2-2), each of $R^2$, $R^3$, $R^8$, and $R^9$ independently represents a hydrogen atom, a halogen atom, or a substituent represented by the following Formula (W), and at least one of $R^2$, $R^3$, $R^8$, or $R^9$ represents a substituent represented by Formula (W). The preferred range of $R^2$, $R^3$, $R^8$, and $R^9$ in Formula (2-2) is the same as the preferred range of $R^2$, $R^3$, $R^8$, and $R^9$ in a case where p = q = 1 in Formula (1).

Specific examples of the compound represented by Formula (1) will be shown below. However, the compound represented by Formula (1) that can be used in the present invention is not limited to the specific examples. Compound examples 1, 427, and 428 are not part of the invention.

Formula (1')

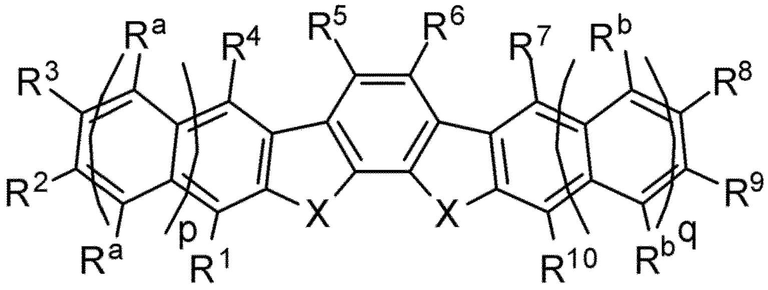

In Formula (1'), each of $R^a$ and $R^b$ independently represents a hydrogen atom, and each of X, p, q, and $R^1$ to $R^{10}$ represents the following groups. In the following tables, Ph represents a phenyl group or a phenylene group.

[Table 1]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 1 (representative) | S | 0 | 0 | H | C3H7 | H | H | H | H | H | H | C3H7 | H |
| Compound example 2 | S | 0 | 0 | H | C4H9 | H | H | H | H | H | H | C4H9 | H |
| Compound example 3 | S | 0 | 0 | H | C5H11 | H | H | H | H | H | H | C5H11 | H |
| Compound example 4 | S | 0 | 0 | H | C6H13 | H | H | H | H | H | H | C6H13 | H |
| Compound example 5 | S | 0 | 0 | H | C7H15 | H | H | H | H | H | H | C7H15 | H |
| Compound example 6 | S | 0 | 0 | H | C8H17 | H | H | H | H | H | H | C8H17 | H |
| Compound example 7 | S | 0 | 0 | H | C9H19 | H | H | H | H | H | H | C9H19 | H |
| Compound example 8 | S | 0 | 0 | H | C10H21 | H | H | H | H | H | H | C10H21 | H |
| Compound example 9 | S | 0 | 0 | H | C11H23 | H | H | H | H | H | H | C11H23 | H |
| Compound example 10 | S | 0 | 0 | H | C12H25 | H | H | H | H | H | H | C12H25 | H |
| Compound example 11 | S | 0 | 0 | H | C13H27 | H | H | H | H | H | H | C13H27 | H |
| Compound example 12 | S | 0 | 0 | H | C14H29 | H | H | H | H | H | H | C14H29 | H |
| Compound example 13 | S | 0 | 0 | H | C15H31 | H | H | H | H | H | H | C15H31 | H |
| Compound example 14 | S | 0 | 0 | H | C16H33 | H | H | H | H | H | H | C16H33 | H |
| Compound example 15 | S | 0 | 0 | H | C17H35 | H | H | H | H | H | H | C17H35 | H |
| Compound example 16 | S | 0 | 0 | H | C18H37 | H | H | H | H | H | H | C18H37 | H |
| Compound example 17 | S | 1 | 1 | H | C5H11 | H | H | H | H | H | H | C5H11 | H |
| Compound example 18 | S | 1 | 1 | H | C6H13 | H | H | H | H | H | H | C6H13 | H |
| Compound example 19 | S | 1 | 1 | H | C7H15 | H | H | H | H | H | H | C7H15 | H |
| Compound example 20 | S | 1 | 1 | H | C8H17 | H | H | H | H | H | H | C8H17 | H |
| Compound example 21 | S | 1 | 1 | H | C9H19 | H | H | H | H | H | H | C9H19 | H |
| Compound example 22 | S | 1 | 1 | H | C10H21 | H | H | H | H | H | H | C10H21 | H |
| Compound example 23 | S | 1 | 1 | H | C11H23 | H | H | H | H | H | H | C11H23 | H |
| Compound example 24 | S | 1 | 1 | H | C12H25 | H | H | H | H | H | H | C12H25 | H |
| Compound example 25 | S | 1 | 1 | H | C13H27 | H | H | H | H | H | H | C13H27 | H |
| Compound example 26 | S | 1 | 1 | H | C14H29 | H | H | H | H | H | H | C14H29 | H |

(continued)

| | X | p | q | R1 | R2 | R3 | R4 | R5 | R6 | R7 | R8 | R9 | R10 |
|---|---|---|---|---|---|---|---|---|---|---|---|---|---|
| Compound example 27 | S | 1 | 1 | H | C15H31 | H | H | H | H | H | H | C15H31 | H |
| Compound example 28 | S | 1 | 1 | H | C16H33 | H | H | H | H | H | H | C16H33 | H |
| Compound example 29 | S | 1 | 1 | H | C17H35 | H | H | H | H | H | H | C17H35 | H |
| Compound example 30 | S | 1 | 1 | H | C18H37 | H | H | H | H | H | H | C18H37 | H |

[Table 2]

| | X | p | 9 | R1 | R2 | R3 | R4 | R5 | R6 | R7 | R8 | R9 | R10 |
|---|---|---|---|---|---|---|---|---|---|---|---|---|---|
| Compound example 31 | S | 0 | 0 | H | p-C5H11-Ph-* | H | H | H | H | H | H | p-C5H11-Ph-* | H |
| Compound example 32 | S | 0 | 0 | H | p-C6H13-Ph-* | H | H | H | H | H | H | p-C6H13-Ph-* | H |
| Compound example 33 | S | 0 | 0 | H | p-C7H15-Ph-* | H | H | H | H | H | H | p-C7H15 Ph-* | H |
| Compound example 34 | S | 0 | 0 | H | p-C8H17-Ph-* | H | H | H | H | H | H | p-C8H17-Ph-* | H |
| Compound example 35 | S | 0 | 0 | H | p-C9H19 Ph-* | H | H | H | H | H | H | p-C9H19-Ph-* | H |
| Compound example 36 | S | 0 | 0 | H | p-C10H21-Ph-* | H | H | H | H | H | H | p-C10H21-Ph-* | H |
| Compound example 37 | S | 0 | 0 | H | p-C11H23-Ph-* | H | H | H | H | H | H | p-CHH23-Ph-* | H |
| Compound example 38 | S | 0 | 0 | H | p-C12H25-Ph-* | H | H | H | H | H | H | p-C12H25-Ph-* | H |
| Compound example 39 | S | 0 | 0 | H | p-C13H27-Ph-* | H | H | H | H | H | H | p-C13H27-Ph-* | H |
| Compound example 40 | S | 0 | 0 | H | p-C14H29-Ph-* | H | H | H | H | H | H | p-C14H29-Ph-* | H |
| Compound example 41 | S | 0 | 0 | H | p-C15H31-Ph-* | H | H | H | H | H | H | p-C15H31-Ph-* | H |
| Compound example 42 | S | 0 | 0 | H | p-C16H33-Ph-* | H | H | H | H | H | H | p-C16H33-Ph-* | H |
| Compound example 43 | S | 0 | 0 | H | p-C17H35-Ph-* | H | H | H | H | H | H | p-C17H35-Ph-* | H |
| Compound example 44 | S | 0 | 0 | H | p-C18H37-Ph-* | H | H | H | H | H | H | p-C18H37-Ph-* | H |
| Compound example 45 | S | 1 | 1 | H | p-C5H11-Ph-* | H | H | H | H | H | H | p-C5H11-Ph-* | H |
| Compound example 46 | S | 1 | 1 | H | p-C6H13-Ph-* | H | H | H | H | H | H | p-C6H13-Ph-* | H |
| Compound example 47 | S | 1 | 1 | H | p-C7H15-Ph-* | H | H | H | H | H | H | p-C7H15-Ph-* | H |
| Compound example 48 | S | 1 | 1 | H | p-C8H17-Ph-* | H | H | H | H | H | H | p-C8H17-Ph-* | H |

(continued)

| | X | p | 9 | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 49 | S | 1 | 1 | H | p-C9H19-Ph-* | H | H | H | H | H | H | p-C9H19-Ph-* | H |
| Compound example 50 | S | 1 | 1 | H | p-C10H21-Ph-* | H | H | H | H | H | H | p-C10H21-Ph-* | H |
| Compound example 51 | S | 1 | 1 | H | p-C11H23-Ph-* | H | H | H | H | H | H | p-C11H23-Ph-* | H |
| Compound example 52 | S | 1 | 1 | H | p-C12H25-Ph-* | H | H | H | H | H | H | p-C12H25-Ph-* | H |
| Compound example 53 | S | 1 | 1 | H | p-C13H27-Ph-* | H | H | H | H | H | H | p-C13H27-Ph-* | H |
| Compound example 54 | S | 1 | 1 | H | p-C14H29-Ph-* | H | H | H | H | H | H | p-C14H29-Ph-* | H |
| Compound example 55 | S | 1 | 1 | H | p-C15H31-Ph-* | H | H | H | H | H | H | p-C15H31-Ph-* | H |
| Compound example 56 | S | 1 | 1 | H | p-C16H33-Ph-* | H | H | H | H | H | H | p-C16H33-Ph-* | H |
| Compound example 57 | S | 1 | 1 | H | p-C17H35-Ph-* | H | H | H | H | H | H | p-C17H35-Ph-* | H |
| Compound example 58 | S | 1 | 1 | H | p-C18H37-Ph-* | H | H | H | H | H | H | p-C18H37-Ph-* | H |

[Table 3]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 59 | S | 0 | 0 | H | 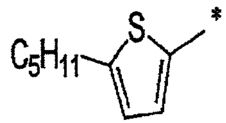 | H | H | H | H | H | H | 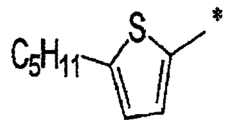 | H |
| Compound example 60 | S | 0 | 0 | H | 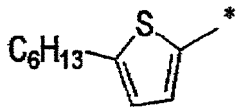 | H | H | H | H | H | H | 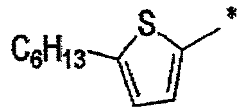 | H |
| Compound example 61 | S | 0 | 0 | H | 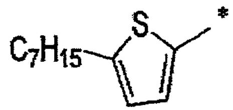 | H | H | H | H | H | H | 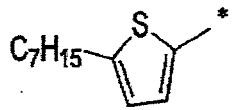 | H |
| Compound example 62 | S | 0 | 0 | H | 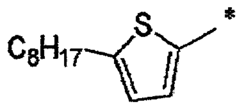 | H | H | H | H | H | H | 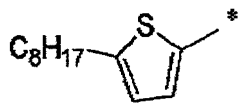 | H |
| Compound example 63 | S | 0 | 0 | H | 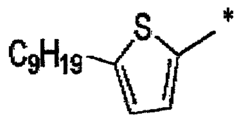 | H | H | H | H | H | H | 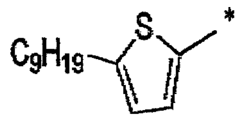 | H |
| Compound example 64 | S | 0 | 0 | H | 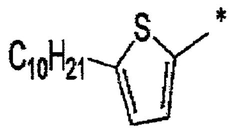 | H | H | H | H | H | H | 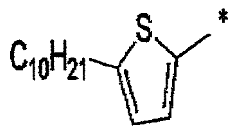 | H |
| Compound example 65 | S | 0 | 0 | H | 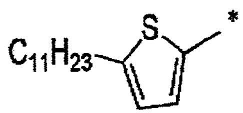 | H | H | H | H | H | H | 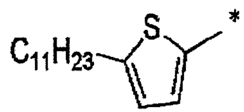 | H |
| Compound example 66 | S | 0 | 0 | H | 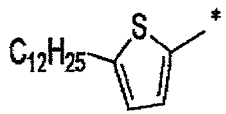 | H | H | H | H | H | H | 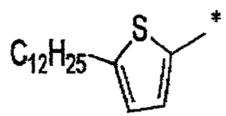 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 67 | S | 0 | 0 | H | 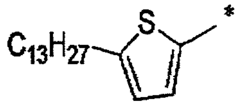 | H | H | H | H | H | H | 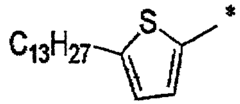 | H |
| Compound example 68 | S | 0 | 0 | H | 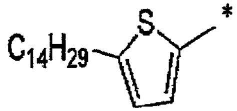 | H | H | H | H | H | H | 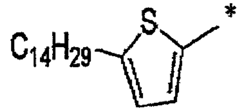 | H |

[Table 4]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 69 | S | 0 | 0 | H | 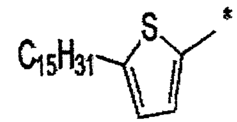 | H | H | H | H | H | H | 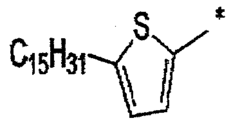 | H |
| Compound example 70 | S | 0 | 0 | H | 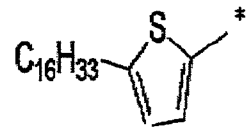 | H | H | H | H | H | H | 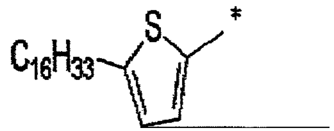 | H |
| Compound example 71 | S | 0 | 0 | H | 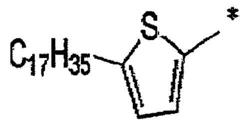 | H | H | H | H | H | H | 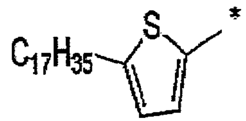 | H |
| Compound example 72 | S | 0 | 0 | H | 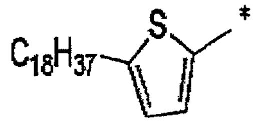 | H | H | H | H | H | H | 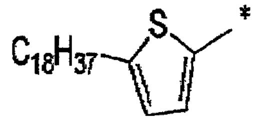 | H |
| Compound example 73 | S | 1 | 1 | H | 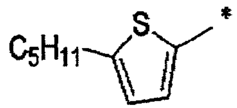 | H | H | H | H | H | H | 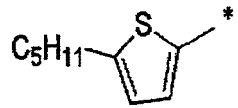 | H |
| Compound example 74 | S | 1 | 1 | H | 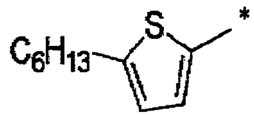 | H | H | H | H | H | H | 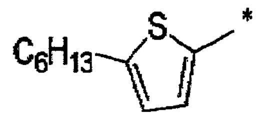 | H |
| Compound example 75 | S | 1 | 1 | H | 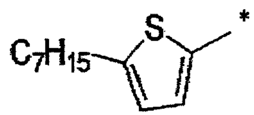 | H | H | H | H | H | H | 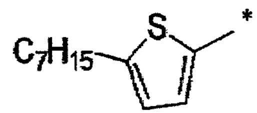 | H |
| Compound example 76 | S | 1 | 1 | H | 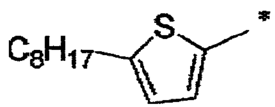 | H | H | H | H | H | H | 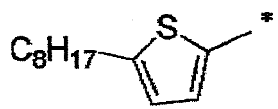 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 77 | S | 1 | 1 | H | 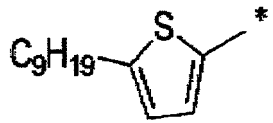 | H | H | H | H | H | H | 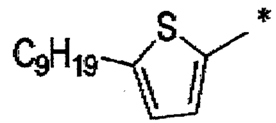 | H |
| Compound example 78 | S | 1 | 1 | H | 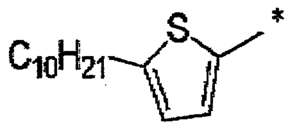 | H | H | H | H | H | H | 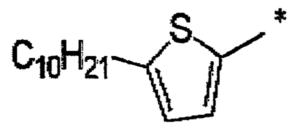 | H |

[Table 5]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 79 | S | 1 | 1 | H | 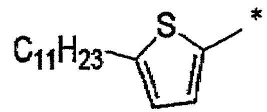 | H | H | H | H | H | H | 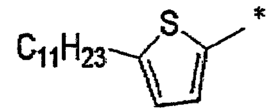 | H |
| Compound example 80 | S | 1 | 1 | H | 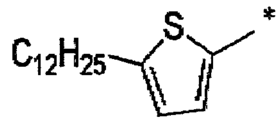 | H | H | H | H | H | H | 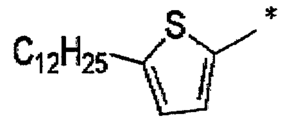 | H |
| Compound example 81 | S | 1 | 1 | H | 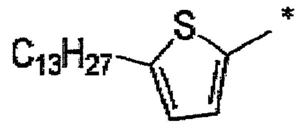 | H | H | H | H | H | H | 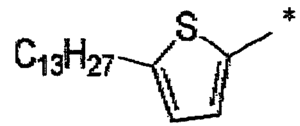 | H |
| Compound example 82 | S | 1 | 1 | H | 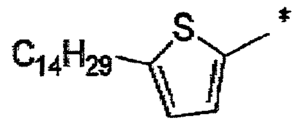 | H | H | H | H | H | H | 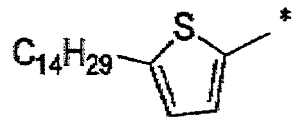 | H |
| Compound example 83 | S | 1 | 1 | H | 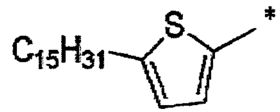 | H | H | H | H | H | H | 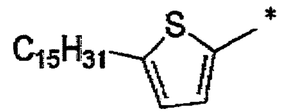 | H |
| Compound example 84 | S | 1 | 1 | H | 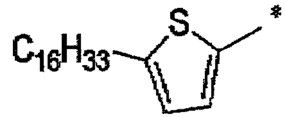 | H | H | H | H | H | H | 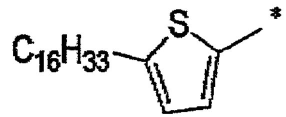 | H |
| Compound example 85 | S | 1 | 1 | H | 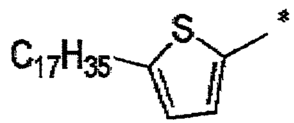 | H | H | H | H | H | H | 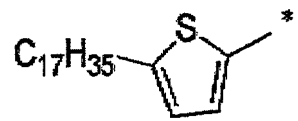 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 86 | S | 1 | 1 | H | 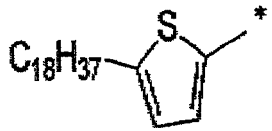 | H | H | H | H | H | H | 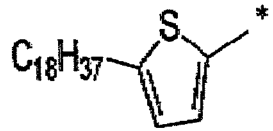 | H |

[Table 6]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 87 | S | 0 | 0 | H | C5H11 | H | H | H | H | H | H | C6H13 | H |
| Compound example 88 | S | 0 | 0 | H | C5H11 | H | H | H | H | H | H | C7H15 | H |
| Compound example 89 | S | 0 | 0 | H | C5H11 | H | H | H | H | H | H | C8H17 | H |
| Compound example 90 | S | 0 | 0 | H | C5H11 | H | H | H | H | H | H | C9H19 | H |
| Compound example 91 | S | 0 | 0 | H | C5H11 | H | H | H | H | H | H | C10H21 | H |
| Compound example 92 | S | 0 | 0 | H | C5H11 | H | H | H | H | H | H | C11H23 | H |
| Compound example 93 | S | 0 | 0 | H | C5H11 | H | H | H | H | H | H | C12H25 | H |
| Compound example 94 | S | 0 | 0 | H | C5H11 | H | H | H | H | H | H | C13H27 | H |
| Compound example 95 | S | 0 | 0 | H | C5H11 | H | H | H | H | H | H | C14H29 | H |
| Compound example 96 | S | 0 | 0 | H | C5H11 | H | H | H | H | H | H | C15H31 | H |
| Compound example 97 | S | 0 | 0 | H | C5H11 | H | H | H | H | H | H | C16H33 | H |
| Compound example 98 | S | 0 | 0 | H | C5H11 | H | H | H | H | H | H | C17H35 | H |
| Compound example 99 | S | 0 | 0 | H | C5H11 | H | H | H | H | H | H | C18H37 | H |
| Compound example 100 | S | 0 | 0 | H | C6H13 | H | H | H | H | H | H | C5H11 | H |
| Compound example 101 | S | 0 | 0 | H | C6H13 | H | H | H | H | H | H | C7H15 | H |
| Compound example 102 | S | 0 | 0 | H | C6H13 | H | H | H. | H | H | H | C8H17 | H |
| Compound example 103 | S | 0 | 0 | H | C6H13 | H | H | H | H | H | H | C9H19 | H |
| Compound example 104 | S | 0 | 0 | H | C6H13 | H | H | H | H | H | H | C10H21 | H |
| Compound example 105 | S | 0 | 0 | H | C6H13 | H | H | H | H | H | H | C11H23 | H |
| Compound example 106 | S | 0 | 0 | H | C6H13 | H | H | H | H | H | H | C12H25 | H |
| Compound example 107 | S | 0 | 0 | H | C6H13 | H | H | H | H | H | H | C13H27 | H |
| Compound example 108 | S | 0 | 0 | H | C6H13 | H | H | H | H | H | H | C14H29 | H |
| Compound example 109 | S | 0 | 0 | H | C6H13 | H | H | H | H | H | H | C15H31 | H |
| Compound example 110 | S | 0 | 0 | H | C6H13 | H | H | H | H | H | H | C16H33 | H |
| Compound example 111 | S | 0 | 0 | H | C6H13 | H | H | H | H | H | H | C17H35 | H |
| Compound example 112 | S | 0 | 0 | H | C6H13 | H | H | H | H | H | H | C18H37 | H |

[Table 7]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 113 | S | 0 | 0 | H | C7H15 | H | H | H | H | H | H | C5H11 | H |
| Compound example 114 | S | 0 | 0 | H | C7H15 | H | H | H | H | H | H | C6H13 | H |
| Compound example 115 | S | 0 | 0 | H | C7H15 | H | H | H | H | H | H | C8H17 | H |
| Compound example 116 | S | 0 | 0 | H | C7H15 | H | H | H | H | H | H | C9H19 | H |
| Compound example 117 | S | 0 | 0 | H | C7H15 | H | H | H | H | H | H | C10H21 | H |
| Compound example 118 | S | 0 | 0 | H | C7H15 | H | H | H | H | H | H | C11H23 | H |
| Compound example 119 | S | 0 | 0 | H | C7H15 | H | H | H | H | H | H | C12H25 | H |
| Compound example 120 | S | 0 | 0 | H | C7H15 | H | H | H | H | H | H | C13H27 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 121 | S | 0 | 0 | H | C7H15 | H | H | H | H | H | H | C14H29 | H |
| Compound example 122 | S | 0 | 0 | H | C7H15 | H | H | H | H | H | H | C15H31 | H |
| Compound example 123 | S | 0 | 0 | H | C7H15 | H | H | H | H | H | H | C16H33 | H |
| Compound example 124 | S | 0 | 0 | H | C7H15 | H | H | H | H | H | H | C17H35 | H |
| Compound example 125 | S | 0 | 0 | H | C7H15 | H | H | H | H | H | H | C18H37 | H |
| Compound example 126 | S | 0 | 0 | H | C8H17 | H | H | H | H | H | H | C5H11 | H |
| Compound example 127 | S | 0 | 0 | H | C8H17 | H | H | H | H | H | H | C6H13 | H |
| Compound example 128 | S | 0 | 0 | H | C8H17 | H | H | H | H | H | H | C7H15 | H |
| Compound example 129 | S | 0 | 0 | H | C8H17 | H | H | H | H | H | H | C9H19 | H |
| Compound example 130 | S | 0 | 0 | H | C8H17 | H | H | H | H | H | H | C10H21 | H |
| Compound example 131 | S | 0 | 0 | H | C8H17 | H | H | H | H | H | H | C11H23 | H |
| Compound example 132 | S | 0 | 0 | H | C8H17 | H | H | H | H | H | H | C12H25 | H |
| Compound example 133 | S | 0 | 0 | H | C8H17 | H | H | H | H | H | H | C13H27 | H |
| Compound example 134 | S | 0 | 0 | H | C8H17 | H | H | H | H | H | H | C14H29 | H |
| Compound example 135 | S | 0 | 0 | H | C8H17 | H | H | H | H | H | H | C15H31 | H |
| Compound example 136 | S | 0 | 0 | H | C8H17 | H | H | H | H | H | H | C16H33 | H |
| Compound example 137 | S | 0 | 0 | H | C8H17 | H | H | H | H | H | H | C17H35 | H |
| Compound example 138 | S | 0 | 0 | H | C8H17 | H | H | H | H | H | H | C18H37 | H |

[Table 8]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 139 | S | 0 | 0 | H | C9H19 | H | H | H | H | H | H | C5H11 | H |
| Compound example 140 | S | 0 | 0 | H | C9H19 | H | H | H | H | H | H | C6H13 | H |
| Compound example 141 | S | 0 | 0 | H | C9H19 | H | H | H | H | H | H | C7H15 | H |
| Compound example 142 | S | 0 | 0 | H | C9H19 | H | H | H | H | H | H | C8H17 | H |
| Compound example 143 | S | 0 | 0 | H | C9H19 | H | H | H | H | H | H | C10H21 | H |
| Compound example 144 | S | 0 | 0 | H | C9H19 | H | H | H | H | H | H | C11H23 | H |
| Compound example 145 | S | 0 | 0 | H | C9H19 | H | H | H | H | H | H | C12H25 | H |
| Compound example 146 | S | 0 | 0 | H | C9H19 | H | H | H | H | H | H | C13H27 | H |
| Compound example 147 | S | 0 | 0 | H | C9H19 | H | H | H | H | H | H | C14H29 | H |

(continued)

| | X | p | q | R1 | R2 | R3 | R4 | R5 | R6 | R7 | R8 | R9 | R10 |
|---|---|---|---|---|---|---|---|---|---|---|---|---|---|
| Compound example 148 | S | 0 | 0 | H | C9H19 | H | H | H | H | H | H | C15H31 | H |
| Compound example 149 | S | 0 | 0 | H | C9H19 | H | H | H | H | H | H | C16H33 | H |
| Compound example 150 | S | 0 | 0 | H | C9H19 | H | H | H | H | H | H | C17H35 | H |
| Compound example 151 | S | 0 | 0 | H | C9H19 | H | H | H | H | H | H | C18H37 | H |
| Compound example 152 | S | 0 | 0 | H | C10H21 | H | H | H | H | H | H | C5H11 | H |
| Compound example 153 | S | 0 | 0 | H | C10H21 | H | H | H | H | H | H | C6H13 | H |
| Compound example 154 | S | 0 | 0 | H | C10H21 | H | H | H | H | H | H | C7H15 | H |
| Compound example 155 | S | 0 | 0 | H | C10H21 | H | H | H | H | H | H | C8H17 | H |
| Compound example 156 | S | 0 | 0 | H | C10H21 | H | H | H | H | H | H | C9H19 | H |
| Compound example 157 | S | 0 | 0 | H | C10H21 | H | H | H | H | H | H | C11H23 | H |
| Compound example 158 | S | 0 | 0 | H | C10H21 | H | H | H | H | H | H | C12H25 | H |
| Compound example 159 | S | 0 | 0 | H | C10H21 | H | H | H | H | H | H | C13H27 | H |
| Compound example 160 | S | 0 | 0 | H | C10H21 | H | H | H | H | H | H | C14H29 | H |
| Compound example 161 | S | 0 | 0 | H | C10H21 | H | H | H | H | H | H | C15H31 | H |
| Compound example 162 | S | 0 | 0 | H | C10H21 | H | H | H | H | H | H | C16H33 | H |
| Compound example 163 | S | 0 | 0 | H | C10H21 | H | H | H | H | H | H | C17H35 | H |
| Compound example 164 | S | 0 | 0 | H | C10H21 | H | H | H | H | H | H | C18H37 | H |

[Table 9]

| | X | p | q | R1 | R2 | R3 | R4 | R5 | R6 | R7 | R8 | R9 | R10 |
|---|---|---|---|---|---|---|---|---|---|---|---|---|---|
| Compound example 165 | S | 0 | 0 | H | C11H23 | H | H | H | H | H | H | C5Hll | H |
| Compound example 166 | S | 0 | 0 | H | C11H23 | H | H | H | H | H | H | C6H13 | H |
| Compound example 167 | S | 0 | 0 | H | C11H23 | H | H | H | H | H | H | C7H15 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 168 | S | 0 | 0 | H | C11H23 | H | H | H | H | H | H | C8H17 | H |
| Compound example 169 | S | 0 | 0 | H | C11H23 | H | H | H | H | H | H | C9H19 | H |
| Compound example 170 | S | 0 | 0 | H | C11H23 | H | H | H | H | H | H | C10H21 | H |
| Compound example 171 | S | 0 | 0 | H | C11H23 | H | H | H | H | H | H | C12H25 | H |
| Compound example 172 | S | 0 | 0 | H | C11H23 | H | H | H | H | H | H | C13H27 | H |
| Compound example 173 | S | 0 | 0 | H | C11H23 | H | H | H | H | H | H | C14H29 | H |
| Compound example 174 | S | 0 | 0 | H | C11H23 | H | H | H | H | H | H | C15H31 | H |
| Compound example 175 | S | 0 | 0 | H | C11H23 | H | H | H | H | H | H | C16H33 | H |
| Compound example 176 | S | 0 | 0 | H | C11H23 | H | H | H | H | H | H | C17H35 | H |
| Compound example 177 | S | 0 | 0 | H | C11H23 | H | H | H | H | H | H | C18H37 | H |
| Compound example 178 | S | 0 | 0 | H | C12H25 | H | H | H | H | H | H | C5Hll | H |
| Compound example 179 | S | 0 | 0 | H | C12H25 | H | H | H | H | H | H | C6H13 | H |
| Compound example 180 | S | 0 | 0 | H | C12H25 | H | H | H | H | H | H | C7H15 | H |
| Compound example 181 | S | 0 | 0 | H | C12H25 | H | H | H | H | H | H | C8H17 | H |
| Compound example 182 | S | 0 | 0 | H | C12H25 | H | H | H | H | H | H | C9H19 | H |
| Compound example 183 | S | 0 | 0 | H | C12H25 | H | H | H | H | H | H | C10H21 | H |
| Compound example 184 | S | 0 | 0 | H | C12H25 | H | H | H | H | H | H | C11H23 | H |
| Compound example 185 | S | 0 | 0 | H | C12H25 | H | H | H | H | H | H | C13H27 | H |
| Compound example 186 | S | 0 | 0 | H | C12H25 | H | H | H | H | H | H | C14H29 | H |
| Compound example 187 | S | 0 | 0 | H | C12H25 | H | H | H | H | H | H | C15H31 | H |
| Compound example 188 | S | 0 | 0 | H | C12H25 | H | H | H | H | H | H | C16H33 | H |
| Compound example 189 | S | 0 | 0 | H | C12H25 | H | H | H | H | H | H | C17H35 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 190 | S | 0 | 0 | H | C12H25 | H | H | H | H | H | H | C18H37 | H |

[Table 10]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 191 | S | 0 | 0 | H | C13H27 | H | H | H | H | H | H | C5H11 | H |
| Compound example 192 | S | 0 | 0 | H | C13H27 | H | H | H | H | H | H | C6H13 | H |
| Compound example 193 | S | 0 | 0 | H | C13H27 | H | H | H | H | H | H | C7H15 | H |
| Compound example 194 | S | 0 | 0 | H | C13H27 | H | H | H | H | H | H | C8H17 | H |
| Compound example 195 | S | 0 | 0 | H | C13H27 | H | H | H | H | H | H | C9H19 | H |
| Compound example 196 | S | 0 | 0 | H | C13H27 | H | H | H | H | H | H | C10H21 | H |
| Compound example 197 | S | 0 | 0 | H | C13H27 | H | H | H | H | H | H | C11H23 | H |
| Compound example 198 | S | 0 | 0 | H | C13H27 | H | H | H | H | H | H | C12H25 | H |
| Compound example 199 | S | 0 | 0 | H | C13H27 | H | H | H | H | H | H | C14H29 | H |
| Compound example 200 | S | 0 | 0 | H | C13H27 | H | H | H | H | H | H | C15H31 | H |
| Compound example 201 | S | 0 | 0 | H | C13H27 | H | H | H | H | H | H | C16H33 | H |
| Compound example 202 | S | 0 | 0 | H | C13H27 | H | H | H | H | H | H | C17H35 | H |
| Compound example 203 | S | 0 | 0 | H | C13H27 | H | H | H | H | H | H | C18H37 | H |
| Compound example 204 | S | 0 | 0 | H | C14H29 | H | H | H | H | H | H | C5H11 | H |
| Compound example 205 | S | 0 | 0 | H | C14H29 | H | H | H | H | H | H | C6H13 | H |
| Compound example 206 | S | 0 | 0 | H | C14H29 | H | H | H | H | H | H | C7H15 | H |
| Compound example 207 | S | 0 | 0 | H | C14H29 | H | H | H | H | H | H | C8H17 | H |
| Compound example 208 | S | 0 | 0 | H | C14H29 | H | H | H | H | H | H | C9H19 | H |
| Compound example 209 | S | 0 | 0 | H | C14H29 | H | H | H | H | H | H | C10H21 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 210 | S | 0 | 0 | H | C14H29 | H | H | H | H | H | H | C11H23 | H |
| Compound example 211 | S | 0 | 0 | H | C14H29 | H | H | H | H | H | H | C12H25 | H |
| Compound example 212 | S | 0 | 0 | H | C14H29 | H | H | H | H | H | H | C13H27 | H |
| Compound example 213 | S | 0 | 0 | H | C14H29 | H | H | H | H | H | H | C15H31 | H |
| Compound example 214 | S | 0 | 0 | H | C14H29 | H | H | H | H | H | H | C16H33 | H |
| Compound example 215 | S | 0 | 0 | H | C14H29 | H | H | H | H | H | H | C17H35 | H |
| Compound example 216 | S | 0 | 0 | H | C14H29 | H | H | H | H | H | H | C18H37 | H |

[Table 11]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 217 | S | 0 | 0 | H | C15H31 | H | H | H | H | H | H | C5H11 | H |
| Compound example 218 | S | 0 | 0 | H | C15H31 | H | H | H | H | H | H | C6H13 | H |
| Compound example 219 | S | 0 | 0 | H | C15H31 | H | H | H | H | H | H | C7H15 | H |
| Compound example 220 | S | 0 | 0 | H | C15H31 | H | H | H | H | H | H | C8H17 | H |
| Compound example 221 | S | 0 | 0 | H | C15H31 | H | H | H | H | H | H | C9H19 | H |
| Compound example 222 | S | 0 | 0 | H | C15H31 | H | H | H | H | H | H | C10H21 | H |
| Compound example 223 | S | 0 | 0 | H | C15H31 | H | H | H | H | H | H | C11H23 | H |
| Compound example 224 | S | 0 | 0 | H | C15H31 | H | H | H | H | H | H | C12H25 | H |
| Compound example 225 | S | 0 | 0 | H | C15H31 | H | H | H | H | H | H | C13H27 | H |
| Compound example 226 | S | 0 | 0 | H | C15H31 | H | H | H | H | H | H | C14H29 | H |
| Compound example 227 | S | 0 | 0 | H | C15H31 | H | H | H | H | H | H | C16H33 | H |
| Compound example 228 | S | 0 | 0 | H | C15H31 | H | H | H | H | H | H | C17H35 | H |
| Compound example 229 | S | 0 | 0 | H | C15H31 | H | H | H | H | H | H | C18H37 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 230 | S | 0 | 0 | H | C16H33 | H | H | H | H | H | H | C5H11 | H |
| Compound example 231 | S | 0 | 0 | H | C16H33 | H | H | H | H | H | H | C6H13 | H |
| Compound example 232 | S | 0 | 0 | H | C16H33 | H | H | H | H | H | H | C7H15 | H |
| Compound example 233 | S | 0 | 0 | H | C16H33 | H | H | H | H | H | H | C8H17 | H |
| Compound example 234 | S | 0 | 0 | H | C16H33 | H | H | H | H | H | H | C9H19 | H |
| Compound example 235 | S | 0 | 0 | H | C16H33 | H | H | H | H | H | H | C10H21 | H |
| Compound example 236 | S | 0 | 0 | H | C16H33 | H | H | H | H | H | H | C11H23 | H |
| Compound example 237 | S | 0 | 0 | H | C16H33 | H | H | H | H | H | H | C12H25 | H |
| Compound example 238 | S | 0 | 0 | H | C16H33 | H | H | H | H | H | H | C13H27 | H |
| Compound example 239 | S | 0 | 0 | H | C16H33 | H | H | H | H | H | H | C14H29 | H |
| Compound example 240 | S | 0 | 0 | H | C16H33 | H | H | H | H | H | H | C15H31 | H |
| Compound example 241 | S | 0 | 0 | H | C16H33 | H | H | H | H | H | H | C17H35 | H |
| Compound example 242 | S | 0 | 0 | H | C16H33 | H | H | H | H | H | H | C18H37 | H |

[Table 12]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 243 | S | 0 | 0 | H | C17H35 | H | H | H | H | H | H | C5H11 | H |
| Compound example 244 | S | 0 | 0 | H | C17H35 | H | H | H | H | H | H | C6H13 | H |
| Compound example 245 | S | 0 | 0 | H | C17H35 | H | H | H | H | H | H | C7H15 | H |
| Compound example 246 | S | 0 | 0 | H | C17H35 | H | H | H | H | H | H | C8H17 | H |
| Compound example 247 | S | 0 | 0 | H | C17H35 | H | H | H | H | H | H | C9H19 | H |
| Compound example 248 | S | 0 | 0 | H | C17H35 | H | H | H | H | H | H | C10H21 | H |
| Compound example 249 | S | 0 | 0 | H | C17H35 | H | H | H | H | H | H | C11H23 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 250 | S | 0 | 0 | H | C17H35 | H | H | H | H | H | H | C12H25 | H |
| Compound example 251 | S | 0 | 0 | H | C17H35 | H | H | H | H | H | H | C13H27 | H |
| Compound example 252 | S | 0 | 0 | H | C17H35 | H | H | H | H | H | H | C14H29 | H |
| Compound example 253 | S | 0 | 0 | H | C17H35 | H | H | H | H | H | H | C15H31 | H |
| Compound example 254 | S | 0 | 0 | H | C17H35 | H | H | H | H | H | H | C16H33 | H |
| Compound example 255 | S | 0 | 0 | H | C17H35 | H | H | H | H | H | H | C18H37 | H |
| Compound example 256 | S | 0 | 0 | H | C18H37 | H | H | H | H | H | H | C6H13 | H |
| Compound example 257 | S | 0 | 0 | H | C18H37 | H | H | H | H | H | H | C7H15 | H |
| Compound example 258 | S | 0 | 0 | H | C18H37 | H | H | H | H | H | H | C8H17 | H |
| Compound example 259 | S | 0 | 0 | H | C18H37 | H | H | H | H | H | H | C9H19 | H |
| Compound example 260 | S | 0 | 0 | H | C18H37 | H | H | H | H | H | H | C10H21 | H |
| Compound example 261 | S | 0 | 0 | H | C18H37 | H | H | H | H | H | H | C11H23 | H |
| Compound example 262 | S | 0 | 0 | H | C18H37 | H | H | H | H | H | H | C12H25 | H |
| Compound example 263 | S | 0 | 0 | H | C18H37 | H | H | H | H | H | H | C13H27 | H |
| Compound example 264 | S | 0 | 0 | H | C18H37 | H | H | H | H | H | H | C14H29 | H |
| Compound example 265 | S | 0 | 0 | H | C18H37 | H | H | H | H | H | H | C15H31 | H |
| Compound example 266 | S | 0 | 0 | H | C18H37 | H | H | H | H | H | H | C16H33 | H |
| Compound example 267 | S | 0 | 0 | H | C18H37 | H | H | H | H | H | H | C17H35 | H |

[Table 13]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 268 | S | 0 | 0 | H | C8H17 | H | H | H | H | H | H | p-C5H11-Ph-* | H |
| Compound example 269 | S | 0 | 0 | H | C8H17 | H | H | H | H | H | H | p-C6H13-Ph-* | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 270 | S | 0 | 0 | H | C8H17 | H | H | H | H | H | H | p-C7H15 Ph-* | H |
| Compound example 271 | S | 0 | 0 | H | C8H17 | H | H | H | H | H | H | p-C8H17-Ph-* | H |
| Compound example 272 | S | 0 | 0 | H | C8H17 | H | H | H | H | H | H | p-C9H19-Ph-* | H |
| Compound example 273 | S | 0 | 0 | H | C8H17 | H | H | H | H | H | H | p-C10H21-Ph-* | H |
| Compound example 274 | S | 0 | 0 | H | C8H17 | H | H | H | H | H | H | p-C11H23 Ph-* | H |
| Compound example 275 | S | 0 | 0 | H | C8H17 | H | H | H | H | H | H | p-C12H25-Ph-* | H |
| Compound example 276 | S | 0 | 0 | H | C8H17 | H | H | H | H | H | H | p-C13H27-Ph-* | H |
| Compound example 277 | S | 0 | 0 | H | C8H17 | H | H | H | H | H | H | p-C14H29-Ph-* | H |
| Compound example 278 | S | 0 | 0 | H | C8H17 | H | H | H | H | H | H | p-C15H31-Ph-* | H |
| Compound example 279 | S | 0 | 0 | H | C8H17 | H | H | H | H | H | H | p-C16H33-Ph-* | H |
| Compound example 280 | S | 0 | 0 | H | C8H17 | H | H | H | H | H | H | p-C17H35-Ph-* | H |
| Compound example 281 | S | 0 | 0 | H | C8H17 | H | H | H | H | H | H | p-C18H37-Ph-* | H |
| Compound example 282 | S | 0 | 0 | H | C8H17 | H | H | H | H | H | H | 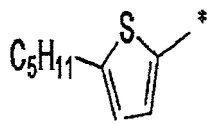 | H |
| Compound example 283 | S | 0 | 0 | H | C8H17 | H | H | H | H | H | H | 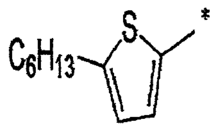 | H |
| Compound example 284 | S | 0 | 0 | H | C8H17 | H | H | H | H | H | H | 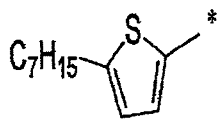 | H |
| Compound example 285 | S | 0 | 0 | H | C8H17 | H | H | H | H | H | H | 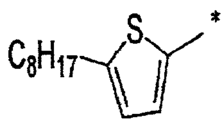 | H |

[Table 14]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 286 | S | 0 | 0 | H | C8H17 | H | H | H | H | H | H | 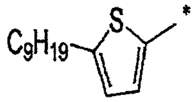 | H |
| Compound example 287 | S | 0 | 0 | H | C8H17 | H | H | H | H | H | H | 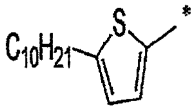 | H |
| Compound example 288 | S | 0 | 0 | H | C8H17 | H | H | H | H | H | H | 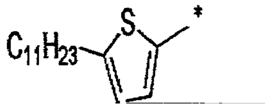 | H |
| Compound example 289 | S | 0 | 0 | H | C8H17 | H | H | H | H | H | H | 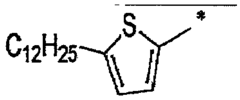 | H |
| Compound example 290 | S | 0 | 0 | H | C8H17 | H | H | H | H | H | H | 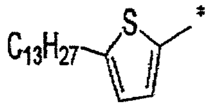 | H |
| Compound example 291 | S | 0 | 0 | H | C8H17 | H | H | H | H | H | H | 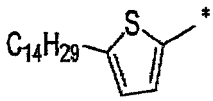 | H |
| Compound example 292 | S | 0 | 0 | H | C8H17 | H | H | H | H | H | H | 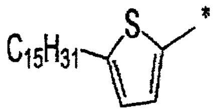 | H |
| Compound example 293 | S | 0 | 0 | H | C8H17 | H | H | H | H | H | H | 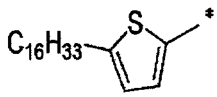 | H |
| Compound example 294 | S | 0 | 0 | H | C8H17 | H | H | H | H | H | H | 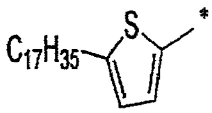 | H |
| Compound example 295 | S | 0 | 0 | H | C8H17 | H | H | H | H | H | H | 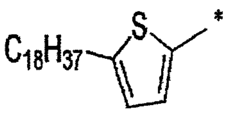 | H |

[Table 15]

| | X | p | q | R1 | R2 | R3 | R4 | R5 | R6 | R7 | R8 | R9 | R10 |
|---|---|---|---|---|---|---|---|---|---|---|---|---|---|
| Compound example 296 | S | 0 | 0 | H | 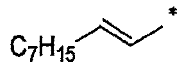 | H | H | H | H | H | H | 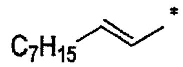 | H |
| Compound example 297 | S | 0 | 0 | H | 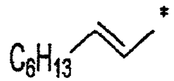 | H | H | H | H | H | H | 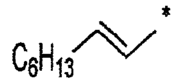 | H |
| Compound example 298 | S | 0 | 0 | H | 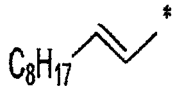 | H | H | H | H | H | H | 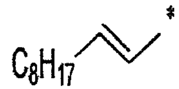 | H |
| Compound example 299 | S | 0 | 0 | H | 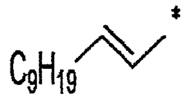 | H | H | H | H | H | H | 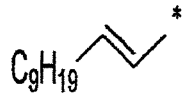 | H |
| Compound example 300 | S | 0 | 0 | H | 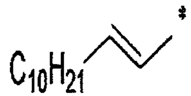 | H | H | H | H | H | H | 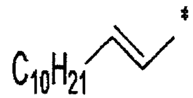 | H |
| Compound example 301 | S | 0 | 0 | H | 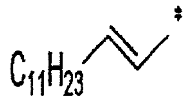 | H | H | H | H | H | H | 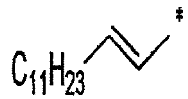 | H |
| Compound example 302 | S | 0 | 0 | H | 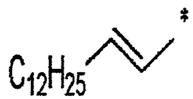 | H | H | H | H | H | H | 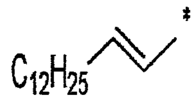 | H |
| Compound example 303 | S | 0 | 0 | H | 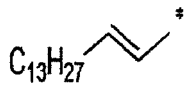 | H | H | H | H | H | H | 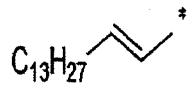 | H |
| Compound example 304 | S | 0 | 0 | H | 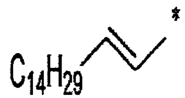 | H | H | H | H | H | H | 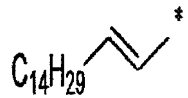 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 305 | S | 0 | 0 | H | 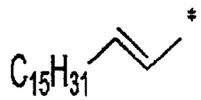 | H | H | H | H | H | H | 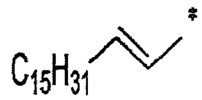 | H |

[Table 16]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 306 | S | 0 | 0 | H | 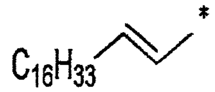 | H | H | H | H | H | H | 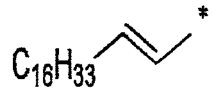 | H |
| Compound example 307 | S | 0 | 0 | H | 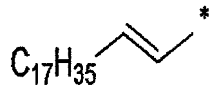 | H | H | H | H | H | H | 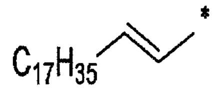 | H |
| Compound example 308 | S | 0 | 0 | H | 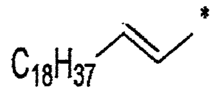 | H | H | H | H | H | H | 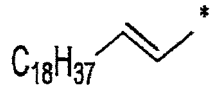 | H |
| Compound example 309 | S | 0 | 0 | H | 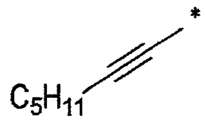 | H | H | H | H | H | H | 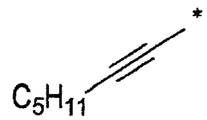 | H |
| Compound example 310 | S | 0 | 0 | H | 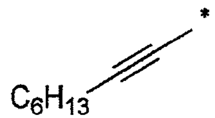 | H | H | H | H | H | H | 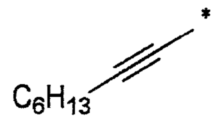 | H |
| Compound example 311 | S | 0 | 0 | H | 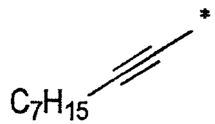 | H | H | H | H | H | H | 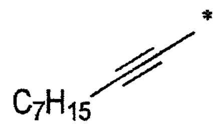 | H |
| Compound example 312 | S | 0 | 0 | H | 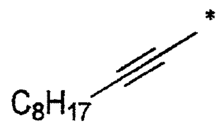 | H | H | H | H | H | H | 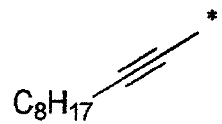 | H |
| Compound example 313 | S | 0 | 0 | H | 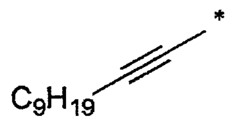 | H | H | H | H | H | H | 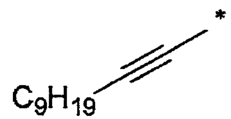 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 314 | S | 0 | 0 | H | 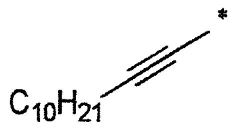 | H | H | H | H | H | H | 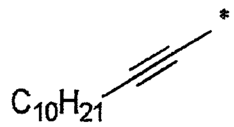 | H |
| Compound example 315 | S | 0 | 0 | H | 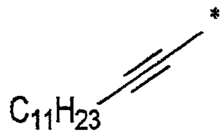 | H | H | H | H | H | H | 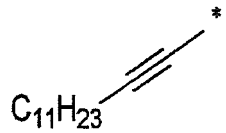 | H |

[Table 17]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 316 | S | 0 | 0 | H | 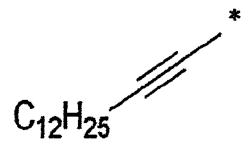 | H | H | H | H | H | H | 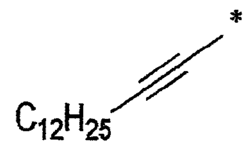 | H |
| Compound example 317 | S | 0 | 0 | H | 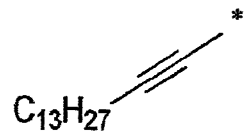 | H | H | H | H | H | H | 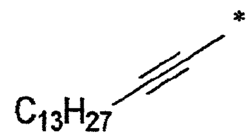 | H |
| Compound example 318 | S | 0 | 0 | H | 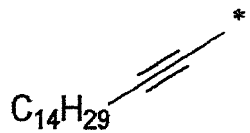 | H | H | H | H | H | H | 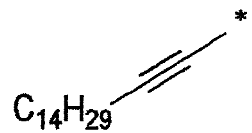 | H |
| Compound example 319 | S | 0 | 0 | H | 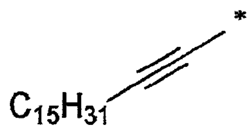 | H | H | H | H | H | H | 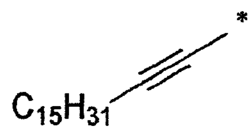 | H |
| Compound example 320 | S | 0 | 0 | H | 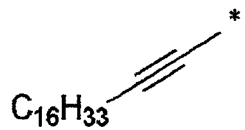 | H | H | H | H | H | H | 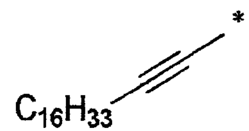 | H |
| Compound example 321 | S | 0 | 0 | H | 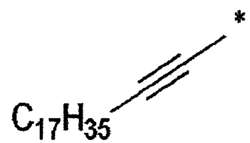 | H | H | H | H | H | H | 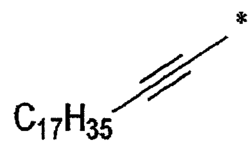 | H |
| Compound example 322 | S | 0 | 0 | H | 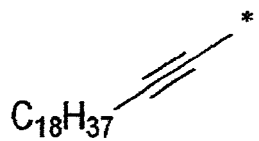 | H | H | H | H | H | H | 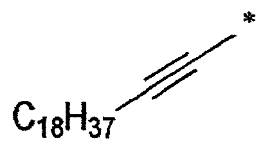 | H |

[Table 18]

| | X | p | q | $R^1$ | $R^2$ | $R^3$ | $R^4$ | $R^5$ | $R^b$ | $R^7$ | $R^8$ | $R^9$ | $R^{10}$ |
|---|---|---|---|---|---|---|---|---|---|---|---|---|---|
| Compound example 323 | S | 0 | 0 | H | $C_5H_{11}O$ | H | H | H | H | H | H | $C_5H_{11}O$ | H |
| Compound example 324 | S | 0 | 0 | H | $C_6H_{13}O$ | H | H | H | H | H | H | $C_6H_{13}0$ | H |
| Compound example 325 | S | 0 | 0 | H | $C_7H_{15}O$ | H | H | H | H | H | H | $C_7H_{13}O$ | H |
| Compound example 326 | S | 0 | 0 | H | $C_8H_{17}O$ | H | H | H | H | H | H | $C_8H_{17}O$ | H |
| Compound example 327 | S | 0 | 0 | H | $C_9H_{19}O$ | H | H | H | H | H | H | $C_9H_{19}O$ | H |
| Compound example 328 | S | 0 | 0 | H | $C_{10}H_{21}O$ | H | H | H | H | H | H | $C_{10}H_{21}0$ | H |
| Compound example 329 | S | 0 | 0 | H | $C_{11}H_{23}O$ | H | H | H | H | H | H | $C_{11}H_{23}O$ | H |
| Compound example 330 | S | 0 | 0 | H | $C_{12}H_{25}O$ | H | H | H | H | H | H | $C_{12}H_{25}O$ | H |
| Compound example 331 | S | 0 | 0 | H | $C_{13}H_{27}O$ | H | H | H | H | H | H | $C_{13}H_{27}O$ | H |
| Compound example 332 | S | 0 | 0 | H | $C_{14}H_{29}O$ | H | H | H | H | H | H | $C_{14}H_{29}O$ | H |
| Compound example 333 | S | 0 | 0 | H | $C_{15}H_{31}O$ | H | H | H | H | H | H | $C_{13}H_{31}O$ | H |
| Compound example 334 | S | 0 | 0 | H | $C_{16}H_{33}O$ | H | H | H | H | H | H | $C_{16}H_{33}O$ | H |
| Compound example 335 | S | 0 | 0 | H | $C_{17}H_{35}0$ | H | H | H | H | H | H | $C_{17}H_{35}O$ | H |
| Compound example 336 | S | 0 | 0 | H | $C_{18}H_{37}O$ | H | H | H | H | H | H | $C_{18}H_{37}O$ | H |
| Compound example 337 | S | 0 | 1 | H | C5H11 | H | H | H | H | H | H | C5H11 | H |
| Compound example 338 | S | 0 | 1 | H | C6H13 | H | H | H | H | H | H | C6H13 | H |
| Compound example 339 | S | 0 | 1 | H | C7H15 | H | H | H | H | H | H | C7H15 | H |
| Compound example 340 | S | 0 | 1 | H | C8H17 | H | H | H | H | H | H | C8H17 | H |
| Compound example 341 | S | 0 | 1 | H | C9H19 | H | H | H | H | H | H | C9H19 | H |
| Compound example 342 | S | 0 | 1 | H | C10H21 | H | H | H | H | H | H | C10H21 | H |
| Compound example 343 | S | 0 | 1 | H | C11H23 | H | H | H | H | H | H | C11H23 | H |
| Compound example 344 | S | 0 | 1 | H | C12H25 | H | H | H | H | H | H | C12H25 | H |

(continued)

| | X | p | q | $R^1$ | $R^2$ | $R^3$ | $R^4$ | $R^5$ | $R^b$ | $R^7$ | $R^8$ | $R^9$ | $R^{10}$ |
|---|---|---|---|---|---|---|---|---|---|---|---|---|---|
| Compound example 345 | S | 0 | 1 | H | C13H27 | H | H | H | H | H | H | C13H27 | H |
| Compound example 346 | S | 0 | 1 | H | C14H29 | H | H | H | H | H | H | C14H29 | H |
| Compound example 347 | S | 0 | 1 | H | C15H31 | H | H | H | H | H | H | C15H31 | H |
| Compound example 348 | S | 0 | 1 | H | C16H33 | H | H | H | H | H | H | C16H33 | H |
| Compound example 349 | S | 0 | 1 | H | C17H35 | H | H | H | H | H | H | C17H35 | H |
| Compound example 350 | S | 0 | 1 | H | C18H37 | H | H | H | H | H | H | C18H37 | H |

[Table 19]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 351 | S | 0 | 0 | H | H | H | H | H | H | H | H | C5H11 | H |
| Compound example 352 | S | 0 | 0 | H | H | H | H | H | H | H | H | C6H13 | H |
| Compound example 353 | S | 0 | 0 | H | H | H | H | H | H | H | H | C7H15 | H |
| Compound example 354 | S | 0 | 0 | H | H | H | H | H | H | H | H | C8H17 | H |
| Compound example 355 | S | 0 | 0 | H | H | H | H | H | H | H | H | C9H19 | H |
| Compound example 356 | S | 0 | 0 | H | H | H | H | H | H | H | H | C10H21 | H |
| Compound example 357 | S | 0 | 0 | H | H | H | H | H | H | H | H | C11H23 | H |
| Compound example 358 | S | 0 | 0 | H | H | H | H | H | H | H | H | C12H25 | H |
| Compound example 359 | S | 0 | 0 | H | H | H | H | H | H | H | H | C13H27 | H |
| Compound example 360 | S | 0 | 0 | H | H | H | H | H | H | H | H | C14H29 | H |
| Compound example 361 | S | 0 | 0 | H | H | H | H | H | H | H | H | C15H31 | H |
| Compound example 362 | S | 0 | 0 | H | H | H | H | H | H | H | H | C16ΣI33 | H |
| Compound example 363 | S | 0 | 0 | H | H | H | H | H | H | H | H | C17H35 | H |
| Compound example 364 | S | 0 | 0 | H | H | H | H | H | H | H | H | C18H37 | H |

(continued)

| | X | p | q | R1 | R2 | R3 | R4 | R5 | R6 | R7 | R8 | R9 | R10 |
|---|---|---|---|---|---|---|---|---|---|---|---|---|---|
| Compound example 365 | S | 0 | 0 | H | H | H | H | H | H | H | H | p-CSH11-Ph-* | H |
| Compound example 366 | S | 0 | 0 | H | H | H | H | H | H | H | H | p-C6H13-Ph-* | H |
| Compound example 367 | S | 0 | 0 | H | H | H | H | H | H | H | H | p-C7H15-Ph-* | H |
| Compound example 368 | S | 0 | 0 | H | H | H | H | H | H | H | H | p-C8H17-Ph-* | H |
| Compound example 369 | S | 0 | 0 | H | H | H | H | H | H | H | H | p-C9H19-Ph-* | H |
| Compound example 370 | S | 0 | 0 | H | H | H | H | H | H | H | H | p-C10H21-Ph-* | H |
| Compound example 371 | S | 0 | 0 | H | H | H | H | H | H | H | H | p-C11H23-Ph-* | H |
| Compound example 372 | S | 0 | 0 | H | H | H | H | H | H | H | H | p-C12H25-Ph-* | H |
| Compound example 373 | S | 0 | 0 | H | H | H | H | H | H | H | H | p-C13H27-Ph-* | H |
| Compound example 374 | S | 0 | 0 | H | H | H | H | H | H | H | H | p-C14H29-Ph-* | H |
| Compound example 375 | S | 0 | 0 | H | H | H | H | H | H | H | H | p-C15H31Ph-* | H |
| Compound example 376 | S | 0 | 0 | H | H | H | H | H | H | H | H | p-C16H33-Ph-* | H |
| Compound example 377 | S | 0 | 0 | H | H | H | H | H | H | H | H | p-C17H35-Ph-* | H |
| Compound example 378 | S | 0 | 0 | H | H | H | H | H | H | H | H | p-C18H37-Ph-* | H |

[Table 20]

| | X | p | q | R1 | R2 | R3 | R4 | R5 | R6 | R7 | R8 | R9 | R10 |
|---|---|---|---|---|---|---|---|---|---|---|---|---|---|
| Compound example 379 | S | 0 | 0 | H | H | H | H | H | H | H | H | 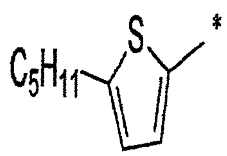 | H |
| Compound example 380 | S | 0 | 0 | H | H | H | H | H | H | H | H | 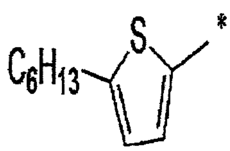 | H |
| Compound example 381 | S | 0 | 0 | H | H | H | H | H | H | H | H | 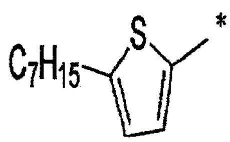 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 382 | S | 0 | 0 | H | H | H | H | H | H | H | H | 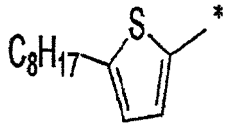 | H |
| Compound example 383 | S | 0 | 0 | H | H | H | H | H | H | H | H | 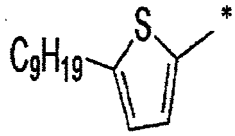 | H |
| Compound example 384 | S | 0 | 0 | H | H | H | H | H | H | H | H | 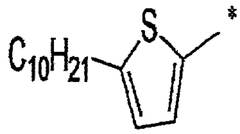 | H |
| Compound example 385 | S | 0 | 0 | H | H | H | H | H | H | H | H | 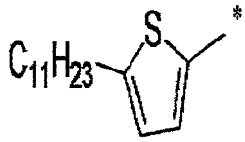 | H |
| Compound example 386 | S | 0 | 0 | H | H | H | H | H | H | H | H | 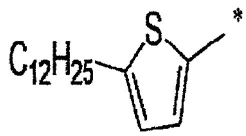 | H |

[Table 21]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 387 | S | 0 | 0 | H | H | H | H | H | H | H | H | 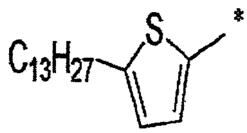 | H |
| Compound example 388 | S | 0 | 0 | H | H | H | H | H | H | H | H | 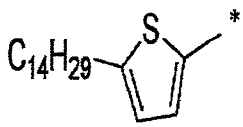 | H |
| Compound example 389 | S | 0 | 0 | H | H | H | H | H | H | H | H | 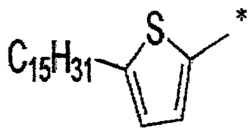 | H |
| Compound example 390 | S | 0 | 0 | H | H | H | H | H | H | H | H | 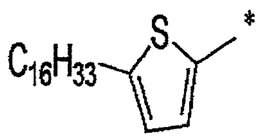 | H |
| Compound example 391 | S | 0 | 0 | H | H | H | H | H | H | H | H | 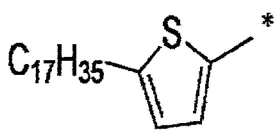 | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 392 | S | 0 | 0 | H | H | H | H | H | H | H | H | 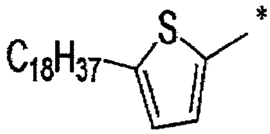 | H |

[Table 22]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 393 | S | 0 | 0 | H | H | C5H11 | H | H | H | H | C5H11 | H | H |
| Compound example 394 | S | 0 | 0 | H | H | C6H13 | H | H | H | H | C6H13 | H | H |
| Compound example 395 | S | 0 | 0 | H | H | C7H15 | H | H | H | H | C7H15 | H | H |
| Compound example 396 | S | 0 | 0 | H | H | C8H17 | H | H | H | H | C8H17 | H | H |
| Compound example 397 | S | 0 | 0 | H | H | C9H19 | H | H | H | H | C9H19 | H | H |
| Compound example 398 | S | 0 | 0 | H | H | C10H21 | H | H | H | H | C10H21 | H | H |
| Compound example 399 | S | 0 | 0 | H | H | C11H23 | H | H | H | H | C11H23 | H | H |
| Compound example 400 | S | 0 | 0 | H | H | C12H25 | H | H | H | H | C12H25 | H | H |
| Compound example 401 | S | 0 | 0 | H | H | C13H27 | H | H | H | H | C13H27 | H | H |
| Compound example 402 | S | 0 | 0 | H | H | C14H29 | H | H | H | H | C14H29 | H | H |
| Compound example 403 | S | 0 | 0 | H | H | C15H31 | H | H | H | H | C15H31 | H | H |
| Compound example 404 | S | 0 | 0 | H | H | C16H33 | H | H | H | H | C16H33 | H | H |
| Compound example 405 | S | 0 | 0 | H | H | C17H35 | H | H | H | H | C17H35 | H | H |
| Compound example 406 | S | 0 | 0 | H | H | C18H37 | H | H | H | H | C18H37 | H | H |
| Compound example 407 | S | 0 | 0 | H | 3,7-Dimethyloctyl | H | H | H | H | H | H | 3,7-Dimethyloctyl | H |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 408 | S | 0 | 0 | H | 2-Ethylhexyl | H | H | H | H | H | H | 2-Ethylhexyl | H |
| Compound example 409 | S | 0 | 0 | H | 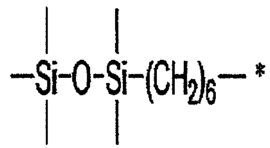 | H | H | H | H | H | H | 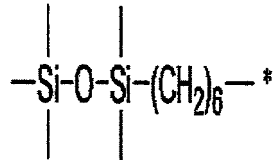 | H |
| Compound example 410 | S | 0 | 0 | H | H | H | H | H | H | H | H | 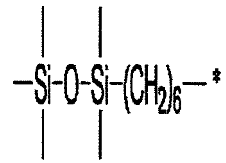 | H |
| Compound example 411 | S | 0 | 0 | H | 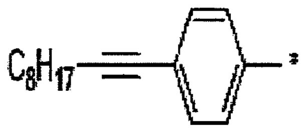 | H | H | H | H | H | H | 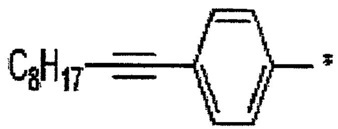 | H |
| Compound example 412 | S | 0 | 0 | H | 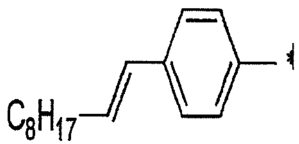 | H | H | H | H | H | H | 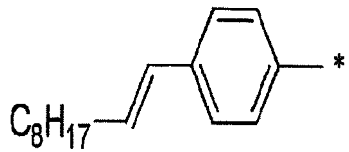 | H |

[Table 23]

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 413 | O | 0 | 0 | H | C5H11 | H | H | H | H | H | H | C5H11 | H |
| Compound example 414 | O | 0 | 0 | H | C6H13 | H | H | H | H | H | H | C6H 13 | H |
| Compound example 415 | O | 0 | 0 | H | C7H15 | H | H | H | H | H | H | C7H 15 | H |
| Compound example 416 | O | 0 | 0 | H | C8H17 | H | H | H | H | H | H | C8H 17 | H |
| Compound example 417 | O | 0 | 0 | H | C9H19 | H | H | H | H | H | H | C9H19 | H |
| Compound example 418 | O | 0 | 0 | H | C10H21 | H | H | H | H | H | H | C10H21 | H |
| Compound example 419 | O | 0 | 0 | H | C11H23 | H | H | H | H | H | H | C11H23 | H |
| Compound example 420 | O | 0 | 0 | H | C12H25 | H | H | H | H | H | H | C12H25 | H |
| Compound example 421 | O | 0 | 0 | H | C13H27 | H | H | H | H | H | H | C13H27 | H |
| Compound example 422 | O | 0 | 0 | H | C14H29 | H | H | H | H | H | H | C14H29 | H |
| Compound example 423 | O | 0 | 0 | H | C15H31 | H | H | H | H | H | H | C15H31 | H |
| Compound example 424 | O | 0 | 0 | H | C16H33 | H | H | H | H | H | H | C16H33 | H |
| Compound example 425 | O | 0 | 0 | H | C17H35 | H | H | H | H | H | H | C17H35 | H |
| Compound example 426 | O | 0 | 0 | H | C18H37 | H | H | H | H | H | H | C18H37 | H |
| Compound example 427 (representative) | S | 0 | 0 | H | 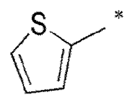 | H | H | 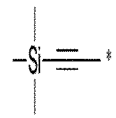 | 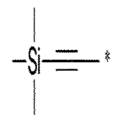 | H | H | 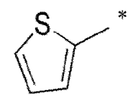 | H |
| Compound example 428 (representative) | S | 0 | 0 | H | Ph | H | H | 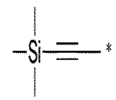 | 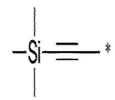 | H | H | Ph | H |
| Compound example 429 | S | 0 | 0 | C5H11 | H | H | H | H | H | H | H | H | C5H11 |
| Compound example 430 | S | 0 | 0 | C6H13 | H | H | H | H | H | H | H | H | C6H13 |
| Compound example 431 | S | 0 | 0 | C7H 15 | H | H | H | H | H | H | H | H | C7H15 |
| Compound example 432 | S | 0 | 0 | C8H 17 | H | H | H | H | H | H | H | H | C8H17 |
| Compound example 433 | S | 0 | 0 | C9H19 | H | H | H | H | H | H | H | H | C9H19 |

(continued)

| | X | p | q | $R^1$ | $R^2$ | $R^3$ | $R^4$ | $R^5$ | $R^6$ | $R^7$ | $R^8$ | $R^9$ | $R^{10}$ |
|---|---|---|---|---|---|---|---|---|---|---|---|---|---|
| Compound example 434 | S | 0 | 0 | C10H21 | H | H | H | H | H | H | H | H | C10H21 |
| Compound example 435 | S | 0 | 0 | C11H23 | H | H | H | H | H | H | H | H | C11H23 |
| Compound example 436 | S | 0 | 0 | C12H25 | H | H | H | H | H | H | H | H | C12H25 |
| Compound example 437 | S | 0 | 0 | C13H27 | H | H | H | H | H | H | H | H | C13H27 |
| Compound example 438 | S | 0 | 0 | C14H29 | H | H | H | H | H | H | H | H | C14H29 |
| Compound example 439 | S | 0 | 0 | C15H31 | H | H | H | H | H | H | H | H | C15H31 |
| Compound example 440 | S | 0 | 0 | C16H33 | H | H | H | H | H | H | H | H | C16H33 |
| Compound example 441 | S | 0 | 0 | C17H35 | H | H | H | H | H | H | H | H | C17H35 |
| Compound example 442 | S | 0 | 0 | C18H37 | H | H | H | H | H | H | H | H | C18H37 |

The compound represented by the Formula (1) may have a repeating structure and may be a low-molecular weight compound or a polymer compound. In a case where the compound represented by Formula (1) is a low-molecular weight compound, the molecular weight thereof is preferably equal to or less than 3,000, more preferably equal to or less than 2,000, even more preferably equal to or less than 1,000, and particularly preferably equal to or less than 850. It is preferable that the molecular weight is equal to or less than the upper limit described above because then the solubility in a solvent can be improved.

In contrast, from the viewpoint of the stability of the film quality, the molecular weight is preferably equal to or greater than 400, more preferably equal to or greater than 450, and even more preferably equal to or greater than 500.

Furthermore, in a case where the compound represented by Formula (1) is a polymer compound having a repeating structure, the weight average molecular weight thereof is preferably equal to or greater than 30,000, more preferably equal to or greater than 50,000, and even more preferably equal to or greater than 100,000. In a case where the compound represented by Formula (1) is a polymer compound having a repeating structure, it is preferable that the weight average molecular weight thereof is equal to or greater than the lower limit described above because then the intermolecular interaction can be enhanced, and hence high mobility is obtained.

Examples of the polymer compound having a repeating structure include a $\pi$-conjugated polymer having a repeating structure in which the compound represented by Formula (1) represents at least one or more arylene groups or heteroarylene groups (thiophene or bithiophene), and a pendant-type polymer in which the compound represented by Formula (1) is bonded to the main chain of the polymer via the side chain. As the main chain of the polymer, polyacrylate, polyvinyl, polysiloxane, or the like is preferable, and as the side chain, an alkylene group, a polyethylene oxide group, or the like is preferable.

The compound represented by Formula (1) can be synthesized according to EP2251342A1, EP2301926A1, EP2301921A1, KR10-2012-0120886A, and Scheme 1 which will be described later.

In synthesizing the compound of the present invention, any of reaction conditions may be used. As a reaction solvent, any solvent may be used. Furthermore, in order to accelerate a ring forming reaction, an acid or a base is preferably used, and a base is particularly preferably used. The optimal reaction conditions vary with the structure of the intended compound.

<Intermediate compound>

The synthetic intermediate having various substituents can be synthesized using known reactions in combination. Furthermore, various substituents may be introduced into the intermediate at any stage. After the intermediate is synthesized, it is preferable to purify the intermediate by column chromatography, recrystallization, or the like and then further purify it by sublimation. By the sublimation purification, it is possible to separate organic impurities and to effectively remove an inorganic salt, a residual solvent, and the like.

The present disclosure also relates to a compound represented by the following Formula (M1) and a compound represented by the following Formula (M2).

Formula (M1)

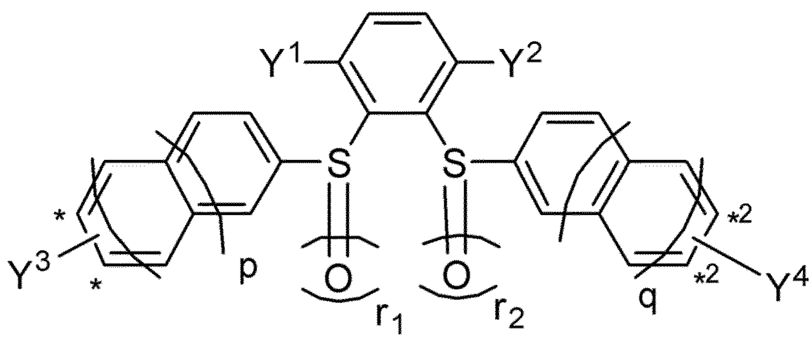

In Formula (M1),

each of $Y^1$ to $Y^4$ independently represents a halogen atom, an alkoxy group, an acyloxy group, a hydroxy group, or a sulfonyl group,
each of $*$ and $*2$ independently represents a position in which each of $Y^3$ and $Y^4$ can be substituted,
each of p and q independently represents an integer of 0 to 2, and
each of r1 and r2 independently represents an integer of 0 to 2.

Formula (M2)

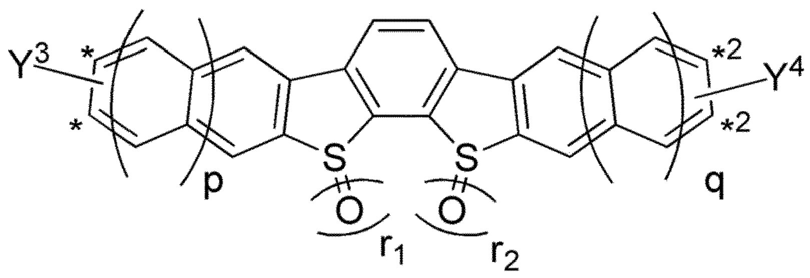

In Formula (M2),

each of $Y^3$ and $Y^4$ independently represents a halogen atom, an alkoxy group, an acyloxy group, a hydroxy group, or a sulfonyl group,
each of ∗ and ∗2 independently represents a position in which each of $Y^3$ and $Y^4$ can be substituted,
each of p and q independently represents an integer of 0 to 2, and
each of r1 and r2 independently represents an integer of 0 to 2.

It is preferable that each of the compound represented by Formula (M1) described above and the compound represented by Formula (M2) described above is the intermediate compound of the compound represented by Formula (1) described above. The compound represented by Formula (1) described above can be synthesized according to Scheme 1, which will be described later, through a process in which the compound represented by Formula (M1) described above and the compound represented by Formula (M2) described above are synthesized as synthetic intermediate compounds.
In Formula (M1), each of $Y^1$ to $Y^4$ is independently a halogen atom, an alkoxy group, an acyloxy group, a hydroxy group, or a sulfonyl group.
The halogen atom represented by $Y^1$ and $Y^2$ in Formula (M1) is preferably a chlorine atom, a bromine atom, or an iodine atom, more preferably a bromine atom or an iodine atom, and particularly preferably a bromine atom.
The halogen atom represented by $Y^3$ and $Y^4$ is preferably a chlorine atom, a bromine atom, or an iodine atom, more preferably a chlorine atom or a bromine atom, and particularly preferably a chlorine atom.
The alkoxy group represented by $Y^1$ to $Y^4$ in Formula (M1) is preferably an alkoxy group having 1 to 5 carbon atoms, more preferably an alkoxy group having 1 to 3 carbon atoms, and particularly preferably a methoxy group.
The acyloxy group represented by $Y^1$ to $Y^4$ in Formula (M1) is preferably an acyloxy group having 1 to 5 carbon atoms, more preferably an acyloxy group having 1 to 3 carbon atoms, and particularly preferably an acetyloxy group.
The sulfonyl group means a group represented by $-SO_2R^{Tf}$, and $R^{Tf}$ represents a substituent. $R^{Tf}$ that the sulfonyl group in Formula (M1) has is preferably a p-tolyl group or a substituted or unsubstituted alkyl group, more preferably a haloalkyl group, and particularly preferably a trifluoromethyl group. The sulfonyl group represented by $Y^1$ to $Y^4$ in Formula (M1) is particularly preferably a trifluoromethanesulfonyl group.
The compound represented by Formula (M1) is preferably a compound in which each of $Y^1$ and $Y^2$ in the Formula (M1) is independently a bromine atom or an iodine atom, and each of $Y^3$ and $Y^4$ is independently a chlorine atom or an alkoxy group.
In Formula (M2), each of $Y^3$ and $Y^4$ independently represents a halogen atom, an alkoxy group, an acyloxy group, a hydroxy group, or a sulfonyl group.
The halogen atom represented by $Y^3$ and $Y^4$ in Formula (M2) is preferably a bromine atom or a chlorine atom, and more preferably a chlorine atom.
The alkoxy group represented by $Y^3$ and $Y^4$ in Formula (M2) is preferably an alkoxy group having 1 to 5 carbon atoms, more preferably an alkoxy group having 1 to 3 carbon atoms, and particularly preferably a methoxy group.
The acyloxy group represented by $Y^3$ and $Y^4$ in Formula (M2) is preferably an acyloxy group having 1 to 5 carbon atoms, more preferably an acyloxy group having 1 to 3 carbon atoms, and particularly preferably an acetyloxy group.
$R^{Tf}$ that the sulfonyl group in Formula (M2) has is preferably a p-tolyl group or a substituted or unsubstituted alkyl group, more preferably a haloalkyl group, and particularly preferably a trifluoromethyl group. The sulfonyl group represented by $Y^3$ and $Y^4$ in Formula (M2) is particularly preferably a trifluoromethanesulfonyl group.
The compound represented by Formula (M2) is preferably a compound in which each of $Y^3$ and $Y^4$ in the Formula (M2) is independently a chlorine atom, an alkoxy group, a hydroxy group, or a sulfonyl group.
In Formulae (M1) and (M2), each of p and q independently represents an integer of 0 to 2. The preferred range

of p and q in Formulae (M1) and (M2) is the same as the preferred range of p and q in Formula (1).

In Formulae (M1) and (M2), each of r1 and r2 independently represents an integer of 0 to 2. Each of r1 and r2 is preferably independently 0 or 1. More preferably, both of r1 and r2 are 0 or 1.

<Structure of organic transistor>

The organic transistor of the present invention has a semiconductor active layer containing the compound represented by Formula (1).

The organic transistor of the present invention may further have layers other than the semiconductor active layer.

The organic transistor of the present invention is preferably used as an organic field effect transistor (PET), and is more preferably used as an insulated gate-type FET in which the gate is insulated from channels.

Hereinafter, preferred structural aspects of the organic transistor of the present invention will be specifically described by using drawings, but the present invention is not limited to the aspects.

(Lamination structure)

The lamination structure of an organic field effect transistor is not particularly limited, and various known structures can be adopted.

For example, the organic transistor of the present invention can adopt a structure (bottom gate/top contact type) in which an electrode, an insulator layer, a semiconductor active layer (organic semiconductor layer), and two electrodes are arranged in this order on the upper surface of a substrate which is a lowermost layer. In this structure, the electrode on the upper surface of the substrate as the lowermost layer is provided in a portion of the substrate, and the insulator layer is so disposed that it comes into contact with the substrate in a portion other than the electrode. The two electrodes provided on the upper surface of the semiconductor active layer are arranged in a state of being separated from each other.

Fig. 1 shows the constitution of a bottom gate/top contact-type element. Fig. 1 is a schematic view showing a section of an exemplary structure of the organic transistor of the present invention. In the organic transistor shown in Fig. 1, a substrate 11 is disposed as a lowermost layer, an electrode 12 is provided in a portion of the upper surface thereof, and an insulator layer 13 is provided such that it covers the electrode 12 and comes into contact with the substrate 11 in a portion other than the electrode 12. On the upper surface of the insulator layer 13, a semiconductor active layer 14 is provided, and in a portion of the upper surface thereof, two electrodes 15a and 15b separated from each other are arranged.

In the organic transistor shown in Fig. 1, the electrode 12 is a gate, and the electrode 15a and the electrode 15b are a drain and a source respectively. The organic transistor shown in Fig. 1 is an insulated gate-type FET in which a channel as a path of electric currents between the drain and the source is insulated from the gate.

As an example of the structure of the organic transistor of the present invention, a bottom gate/bottom contact-type element can be exemplified.

Fig. 2 shows the constitution of the bottom gate/bottom contact-type element. Fig. 2 is a schematic view showing a section of the structure of an organic transistor manufactured as a substrate for measuring FET characteristics in examples of the present invention. In the organic transistor shown in Fig. 2, a substrate 31 is disposed as a lowermost layer, an electrode 32 is provided in a portion of the upper surface thereof, and an insulator layer 33 is provided such that it covers the electrode 32 and comes into contact with the substrate 31 in a portion other than the electrode 32. Furthermore, a semiconductor active layer 35 is provided on the upper surface of the insulator layer 33, and electrodes 34a and 34b are in a lower portion of the semiconductor active layer 35.

In the organic transistor shown in Fig. 2, the electrode 32 is a gate, and the electrode 34a and the electrode 34b are a drain and a source respectively. The organic transistor shown in Fig. 2 is an insulated gate-type FET in which a channel as a path of electric currents between the drain and the source is insulated from the gate.

As the structure of the organic transistor of the present invention, a top gate/top contact-type element in which an insulator and a gate electrode are in the upper portion of a semiconductor active layer or a top gate/bottom contact-type element can also be preferably used.

(Thickness)

In a case where the organic transistor of the present invention needs to be a thinner transistor, the total thickness of the transistor is preferably, for example, 0.1 μm to 0.5 μm.

(Sealing)

In order to improve the preservation stability of the organic transistor element by blocking the organic transistor element from the atmosphere or moisture, the entirety of the organic transistor element may be sealed with a metal sealing can, glass, an inorganic material such as silicon nitride, a polymer material such as parylene, a low-molecular weight material, or the like.

Hereinafter, preferred aspects of the respective layers of the organic transistor of the present invention will be described, but the present invention is not limited to the aspects.

<Substrate>

(Material)

The organic transistor of the present invention preferably includes a substrate.

The material of the substrate is not particularly limited, and known materials can be used. Examples of the material include a polyester film such as polyethylene naphthalate (PEN) or polyethylene terephthalate (PET), a cycloolefin polymer film, a polycarbonate film, a triacetylcellulose (TAC) film, a polyimide film, a material obtained by bonding these polymer films to extremely thin glass, ceramics, silicon, quartz, glass, and the like. Among these, silicon is preferable.

<Electrode>

(Material)

The organic transistor of the present invention preferably includes an electrode.

As the material constituting the electrode, known conductive materials such as a metal material like Cr, Al, Ta, Mo, Nb, Cu, Ag, Au, Pt, Pd, In, Ni, or Nd, an alloy material of these, a carbon material, and a conductive polymer can be used without particular limitation.

(Thickness)

The thickness of the electrode is not particularly limited, but is preferably 10 nm to 50 nm.

A gate width (or a channel width) W and a gate length (or a channel length) L are not particularly limited. However, a ratio of W/L is preferably equal to or greater than 10, and more preferably equal to or greater than 20.

<Insulating layer>

(Material)

The material constituting the insulating layer is not particularly limited as long as an insulating effect is obtained as required. Examples of the material include silicon dioxide, silicon nitride, a fluorine polymer-based insulating material such as PTFE or CYTOP, a polyester insulating material, a polycarbonate insulating material, an acryl polymer-based insulating material, an epoxy resin-based insulating material, a polyimide insulating material, a polyvinyl phenol resin-based insulating material, a poly p-xylylene resin-based insulating material, and the like.

A surface treatment may be performed on the upper surface of the insulating layer. For example, it is possible to preferably use an insulating layer in which the silicon dioxide surface thereof is subjected to the surface treatment by being coated with hexamethyldisilazane (HMDS) or octadecyltrichlorosilane (OTS).

(Thickness)

The thickness of the insulating layer is not particularly limited. However, in a case where the film needs to be thinned, the thickness of the insulating layer is preferably 10 nm to 400 nm, more preferably 20 nm to 200 nm, and particularly preferably 50 nm to 200 nm.

<Semiconductor active layer>

(Material)

In the organic transistor of the present invention, the semiconductor active layer contains a compound represented by Formula (1), that is, the compound of the present invention.

The semiconductor active layer may be a layer consisting of the compound of the present invention or a layer further containing a polymer binder, which will be described later, in addition to the compound of the present invention. Furthermore, the semiconductor active layer may contain a residual solvent used at the time of forming a film.

The content of the polymer binder in the semiconductor active layer is not particularly limited. However, the content of the polymer binder is preferably within a range of 0% by mass to 95% by mass, more preferably within a range of 10% by mass to 90% by mass, even more preferably within a range of 20% by mass to 80% by mass, and particularly preferably within a range of 30% by mass to 70% by mass.

(Thickness)

The thickness of the semiconductor active layer is not particularly limited. However, in a case where the film needs to be thinned, the thickness of the semiconductor active layer is preferably 10 nm to 400 nm, more preferably 10 nm to 200 nm, and particularly preferably 10 nm to 100 nm.

[Organic semiconductor material for non-light-emitting organic semiconductor device] The present disclosure also relates to an organic semiconductor material for a non-light-emitting organic semiconductor device containing the compound represented by Formula (1).

(Non-light-emitting organic semiconductor device)

In the present specification, a "non-light-emitting organic semiconductor device" refers to a device which is not used for the purpose of emitting light. The non-light-emitting organic semiconductor device preferably uses an electronic element having a layered structure consisting of films. The non-light-emitting organic semiconductor device includes an organic transistor, an organic photoelectric conversion element (a solid-state imaging element used for a photosensor, a solar cell used for energy conversion, or the like), a gas sensor, an organic rectifying element, an organic inverter, an information recording element, and the like. The organic photoelectric conversion element can be used for both a photosensor (solid-state imaging element) and for energy conversion (a solar cell). Among these, an organic photoelectric conversion element and an organic transistor are preferable, and an organic transistor is more preferable. That is, the organic semiconductor material for a non-light-emitting organic semiconductor device of the present invention is preferably a material for an organic transistor as described above.

(Organic semiconductor material)

In the present specification, the "organic semiconductor material" is an organic material showing characteristics of a semiconductor. Just as the semiconductor composed of an inorganic material, the organic semiconductor is classified into a p-type (hole-transporting) organic semiconductor material conducting holes as carriers and an n-type (electron-transporting) organic semiconductor material conducting electrons as carriers.

The compound of the present invention may be used as any of the p-type organic semiconductor material and the n-type organic semiconductor material, but is preferably used as the p-type. The ease with which the carriers flow in the organic semiconductor is represented by a carrier mobility $\mu$. The higher the carrier mobility $\mu$, the better. The carrier mobility $\mu$ is preferably equal to or greater than $1 \times 10^{-4}$ $cm^2/Vs$, more preferably equal to or greater than $1 \times 10^{-2}$ $cm^2/Vs$, particularly preferably equal to or greater than $5 \times 10^{-2}$ $cm^2/Vs$, more particularly preferably equal to or greater than $1 \times 10^{-1}$ $cm^2/Vs$, and even more particularly preferably equal to or greater than $2 \times 10^{-1}$ $cm^2/Vs$. The carrier mobility $\mu$ can be determined by the characteristics of the prepared field effect transistor (FET) element or by a time-of-flight (TOF) measurement method.

[Organic semiconductor film for non-light-emitting organic semiconductor device]

(Material)

The present invention also relates to an organic semiconductor film for a non-light-emitting organic semiconductor device containing the compound represented by Formula (1).

As the organic semiconductor film for a non-light-emitting organic semiconductor device of the present invention,

an aspect is also preferable in which the organic semiconductor film contains the compound represented by Formula (1) and does not contain a polymer binder.

Furthermore, the organic semiconductor film for a non-light-emitting organic semiconductor device of the present invention may contain the compound represented by Formula (1) and a polymer binder.

Examples of the polymer binder include an insulating polymer such as polystyrene, polycarbonate, polyarylate, polyester, polyamide, polyimide, polyurethane, polysiloxane, polysulfone, polymethyl methacrylate, polymethyl acrylate, cellulose, polyethylene, or polypropylene, a copolymer of these, a photoconductive polymer such as polyvinylcarbazole or polysilane, a conductive polymer such as polythiophene, polypyrrole, polyaniline, or poly p-phenylenevinylene, and a semiconductor polymer.

One kind of the aforementioned polymer binder may be used singly, or plural kinds thereof may be used concurrently.

The organic semiconductor material may be uniformly mixed with the polymer binder. Alternatively, the organic semiconductor material and the polymer binder may be totally or partially in a phase separation state. From the viewpoint of the charge mobility, a structure, in which the organic semiconductor and the binder are in a phase separation state along the film thickness direction in the film, is the most preferable because then the binder does not hinder the organic semiconductor from moving a charge.

Considering the mechanical strength of the film, a polymer binder having a high glass transition temperature is preferable. Furthermore, considering the charge mobility, a polymer binder having a structure not containing a polar group, a photoconductive polymer, and a conductive polymer are preferable.

The amount of the polymer binder used is not particularly limited. However, in the organic semiconductor film for a non-light-emitting organic semiconductor device of the present invention, the amount of the polymer binder used is preferably within a range of 0% by mass to 95% by mass, more preferably within a range of 10% by mass to 90% by mass, even more preferably within a range of 20% by mass to 80% by mass, and particularly preferably within a range of 30% by mass to 70% by mass.

In the present invention, by adopting the aforementioned structure as the structure of the compound, an organic film having excellent film quality can be obtained. Specifically, because the compound obtained in the present invention has excellent crystallinity, a sufficient film thickness can be obtained, and the obtained organic semiconductor film for a non-light-emitting organic semiconductor device of the present invention has excellent quality.

(Film forming method)

The compound of the present invention may be formed into a film on a substrate by any method.

At the time of forming the film, the substrate may be heated or cooled. By varying the temperature of the substrate, it is possible to control the film quality or the packing of molecules in the film. The temperature of the substrate is not particularly limited. However, it is preferably between 0°C to 200°C, more preferably between 15°C to 100°C, and particularly preferably between 20°C to 95°C.

The compound of the present invention can be formed into a film on a substrate by a vacuum process or a solution process, and both of the processes are preferable.

Specific examples of the film forming method by a vacuum process include a physical vapor deposition method such as a vacuum vapor deposition method, a sputtering method, an ion plating method, or a molecular beam epitaxy (MBE) method and a chemical vapor deposition (CVD) method such as plasma polymerization, and it is particularly preferable to use a vacuum vapor deposition method.

Herein, the film forming method by a solution process refers to a method of dissolving an organic compound in a solvent which can dissolve the compound and forming a film by using the solution. Specifically, it is possible to use general methods like a coating method such as a casting method, a dip coating method, a die coater method, a roll coater method, a bar coater method, or a spin coating method, various printing methods such as an ink jet method, a screen printing method, a gravure printing method, a flexographic printing method, an offset printing method, or a micro-contact printing method, and a Langmuir-Blodgett (LB) method. It is particularly preferable to use a casting method, a spin coating method, an ink jet method, a gravure printing method, a flexographic printing method, an offset printing method, or a micro-contact printing method.

The organic semiconductor film for a non-light-emitting organic semiconductor device of the present invention is preferably prepared by a solution coating method. In a case where the organic semiconductor film for a non-light-emitting organic semiconductor device of the present invention contains a polymer binder, it is preferable to prepare a coating solution by dissolving or dispersing a material, which will be formed into a layer, and a polymer binder in an appropriate solvent and to form the organic semiconductor film by various coating methods.

Hereinafter, a coating solution for a non-light-emitting organic semiconductor device of the present invention that can be used for forming a film by a solution process will be described.

[Coating solution for non-light-emitting organic semiconductor device]

The present invention also relates to a coating solution for a non-light-emitting organic semiconductor device that contains the compound represented by Formula (1).

In a case where a film is formed on a substrate by using a solution process, a material which will be formed into a layer is dissolved or dispersed in either or both of an appropriate organic solvent (for example, a hydrocarbon-based solvent such as hexane, octane, decane, toluene, xylene, mesitylene, ethylbenzene, decalin, or 1-methylnaphthalene, a ketone-based solvent such as acetone, methyl ethyl ketone, methyl isobutyl ketone, or cyclohexanone, a halogenated hydrocarbon-based solvent such as dichloromethane, chloroform, tetrachloromethane, dichloroethane, trichloroethane, tetrachloroethane, chlorobenzene, dichlorobenzene, or chlorotoluene, an ester-based solvent such as ethyl acetate, butyl acetate, or amyl acetate, an alcohol-based solvent such as methanol, propanol, butanol, pentanol, hexanol, cyclohexanol, methyl cellosolve, ethyl cellosolve, or ethylene glycol, an ether-based solvent such as dibutylether, tetrahydrofuran, dioxane, or anisole, an amide-imide-based solvent such as N,N-dimethylformamide, N,N-dimethylacetamide, 1-methyl-2-pyrrolidone, or 1-methyl-2-imidazolidinone, a sulfoxide-based solvent such as dimethyl sulfoxide, or a nitrile-based solvent such as acetonitrile) and water so as to obtain a coating solution, and a film can be formed by various coating methods by using the coating solution. One kind of the solvent may be used singly, or plural kinds thereof may be used in combination. Among these, a hydrocarbon-based solvent, a halogenated hydrocarbon-based solvent, and an ether-based solvent are preferable, toluene, xylene, mesitylene, tetralin, dichlorobenzene, and anisole are more preferable, and toluene, xylene, tetralin, and anisole are particularly preferable. The concentration of the compound represented by Formula (1) in the coating solution is preferably 0.1% by mass to 80% by mass, more preferably 0.1% by mass to 10% by mass, and particularly preferably 0.5% by mass to 10% by mass. In this way, a film having an arbitrary thickness can be formed.

In order to form a film by a solution process, the material needs to dissolve in the solvent exemplified above, but simply dissolving in a solvent is not good enough. Generally, even the material formed into a film by a vacuum process can dissolve in a solvent to some extent. The solution process includes a step of coating a substrate with a material by dissolving the material in a solvent and then forming a film by evaporating the solvent, and many of the materials not suitable for being formed into a film by the solution process have high crystallinity. Therefore, the material is inappropriately crystallized (aggregated) in the aforementioned step, and hence it is difficult to form an excellent film. The compound represented by Formula (1) is also excellent in the respect that it is not easily crystallized (aggregated).

As the coating solution for a non-light-emitting organic semiconductor device of the present invention, an aspect is also preferable in which the coating solution contains the compound represented by Formula (1) and does not contain a polymer binder.

Furthermore, the coating solution for a non-light-emitting organic semiconductor device of the present invention may contain the compound represented by Formula (1) and a polymer binder. In this case, a material, which will be formed into a layer, and a polymer binder are dissolved or dispersed in an appropriate solvent described above so as to prepare a coating solution, and by using the coating solution, a film can be formed by various coating methods. The polymer binder can be selected from those described above.

Examples

Hereinafter, the characteristics of the present invention will be more specifically explained by describing examples and comparative examples. The materials, the amount thereof used, the proportion thereof, the content of treatment, the treatment procedure, and the like described in the following examples can be appropriately modified within a range that does not depart from the gist of the present invention. Accordingly, the scope of the present invention is not limited to the following specific examples.

[Examples 1 to 21 and Comparative examples 1 to 6]

<Synthesis example>

According to a specific synthesis procedure shown in the following Scheme 1, compound examples 3, 6, 10, 407, 20, 24, 298, 310, 326, 34, 62, 1, 16, 396, 386, 372, 358, 344, 416, 427, and 429 as compounds represented by Formula (1) and intermediate compounds (A) to (D) were synthesized.

Scheme 1

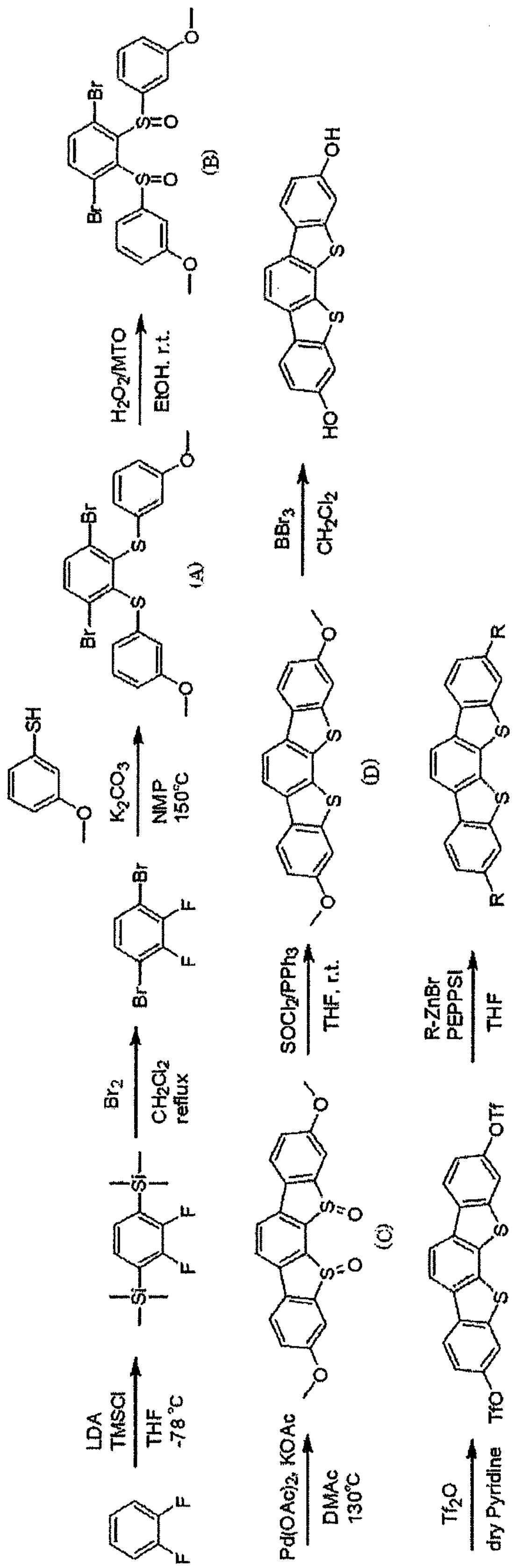
LDA
TMSCl
THF
-78 °C
Br2
CH2Cl2
reflux
K2CO3
NMP
150°C
(A)
H2O2/MTO
EtOH. r.t.
(B)
Pd(OAc)2, KOAc
DMAc
130°C
(C)
SOCl2/PPh3
THF, r.t.
(D)
BBr3
CH2Cl2
Tf2O
dry Pyridine
R-ZnBr
PEPPSI
THF

Synthesis of intermediate compound (A)

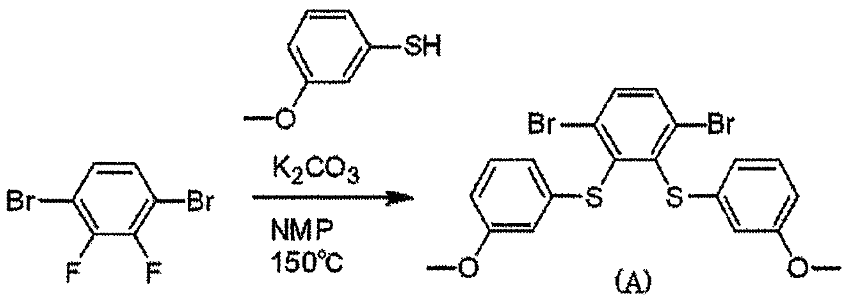


From 1,4-dibromo-2,3-difluorobenzene, an intermediate compound (A) was synthesized by the following procedure.

31 g of 1,4-dibromo-2,3-difluorobenzene, 37.9 g of potassium carbonate, and 31.9 g of 3-methoxyphenylthiol were added to 600 ml of N-methylpyrrolidone, followed by stirring for 2 hours at room temperature in a nitrogen atmosphere. The reaction mixture was filtered using celite, ethyl acetate and water were added to the filtrate for extraction, and oil layers were washed three times with dilute hydrochloric acid. Then, magnesium sulfate was added to the oil layers put together so as to dry the oil layers, followed by filtration, and ethyl acetate was distilled away under reduced pressure, thereby obtaining 45 g (yield: 77%) of an oil-like intermediate compound (A).

The structure of the intermediate compound (A) was identified by $^1$H-NMR.

$^1$H-NMR (400 MHz, DMSO)
δ: 7.88 (s, 2H), 7.17 (t, 2H), 6.73 (d, 2H), 6.51 (s, 2H), 6.50 (d, 2H), 3.68 (s, 6H)

Synthesis of intermediate compound (B)

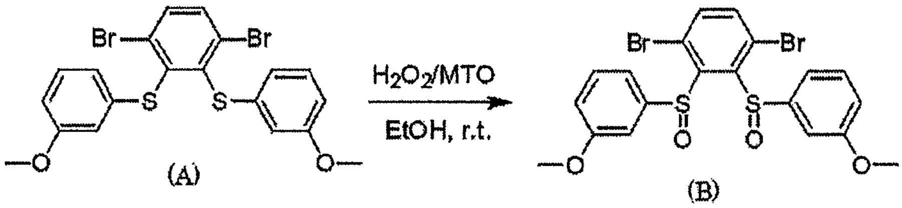


From the intermediate compound (A), an intermediate compound (B) was synthesized by the following procedure.

23.1 g of the intermediate compound (A) and 0.548 g of methyltrioxorhenium were dissolved in a mixed solvent of 176 ml of acetonitrile and 44 ml of dichloromethane, and 25.8 ml of 30% aqueous hydrogen peroxide was added dropwise thereto at room temperature. After the dropwise addition, the resulting mixture was stirred for 2 hours at room temperature, and the generated precipitate was collected by filtration, thereby obtaining 14.3 g (yield: 59%) of an intermediate compound (B) in the form of a white solid.

The structure of the intermediate compound (B) was identified by $^1$H-NMR.

$^1$H-NMR (400 MHz, DMSO)
δ: 7.89 (s, 2H), 7.31 (t, 2H), 6.97 (d, 2H), 6.78 (s, 2H), 6.69 (d, 2H), 3.68 (s, 6H)

Synthesis of intermediate compound (C)

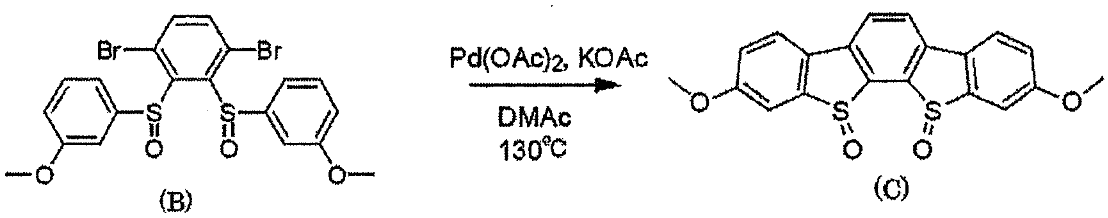


(B) (C)

From the intermediate compound (B), an intermediate compound (C) was synthesized by the following procedure.

7.8 g of the intermediate (B), 96.5 mg of palladium acetate, and 2.8 g of potassium acetate were added to 86 ml of dimethyl acetamide, followed by vacuum deaeration, and then the resulting solution was heated and stirred for 2 hours at 130°C in a nitrogen atmosphere. The reaction solution as cooled to room temperature and then poured into water, and the generated precipitate was collected by filtration. The solid separated by filtration was washed by ultrasonic dispersion in ethanol, thereby obtaining 4.2 g (yield: 76%) of an intermediate (C) in the form of a white solid.

The structure of the intermediate compound (C) was identified by $^1$H-NMR.

$^1$H-NMR (400 MHz, $CDCl_3$)
δ: 7.85 (s, 2H), 7.75 (d, 2H), 7.59 (s, 2H), 7.17 (d, 2H), 3.94 (s, 6H)

Synthesis of intermediate compound (D)

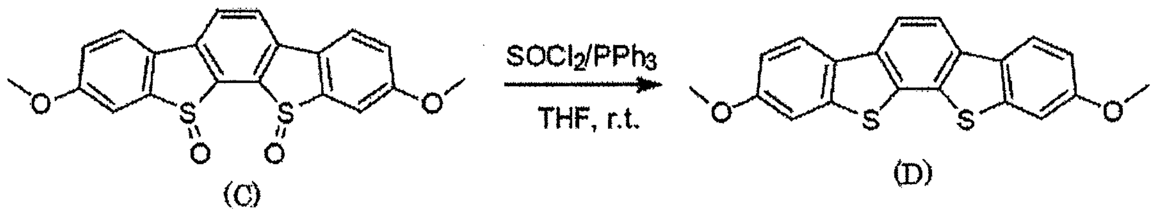


(C) (D)

From the intermediate compound (C), an intermediate compound (D) was synthesized by the following procedure.

3.8 g of the intermediate compound (C), 5.63 g of potassium iodide, and 4.9 g of p-toluenesulfonic acid monohydrate were mixed together and irradiated with ultrasonic waves for 1 hour at 40°C. The reaction mixture was put into an aqueous sodium hydrogen sulfite solution, and the insoluble matter was collected by filtration. The solid separated by filtration was washed with toluene, thereby obtaining 2.7 g (yield: 78%) of an intermediate compound (D) in the form of a white solid.

The structure of the intermediate compound (D) was identified by $^1$H-NMR.

$^1$H-NMR (400 MHz, $CDCl_3$)
δ: 8.10-8.07 (m, 4H), 7.40 (s, 2H), 7.10 (d, 2H), 3.94 (s, 6H)

Through the intermediate compound (D), compound examples 3, 6, 10, 407, 20, 24, 298, 310, 326, 34, 62, 1, 16, 396, 386, 368, 358, 344, 416, 427, and 429 as compounds represented by Formula (1) were synthesized. The obtained compounds were identified by elementary analysis, NMR, and MASS spectrometry.

The structures of comparative compounds 1 to 6 used in a semiconductor active layer (organic semiconductor layer) of comparative elements are shown below.

The comparative compound 1 is a compound described in Tetrahedron Lett., 1973 (17), 1561, and was synthesized according to the method described in the document.

The comparative compound 2 is a compound described in Rao, Tilak, Journal of Scientific and Industrial Research, 1958, vol. 17 B, pp. 260-265 and Kudo, Hirotaka; Tedjamulia, Marvin L.; Castle, Raymond N.; Lee, Milton L., Journal of Heterocyclic Chemistry, 1984, vol. 21, pp. 185-192, and was synthesized according to the method described in these documents.

The comparative compounds 3 and 4 are compounds 232 and 236 of EP2301926A1, and were synthesized according to the method described in the document.

The comparative compound 5 was synthesized according to the method described in EP2251342A1.

The comparative compound 6 is a compound 30 of KR2012-0120886A, and was synthesized according to the method described in the document.

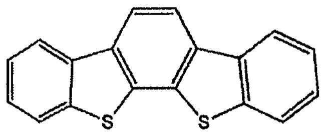

Comparative compound 1

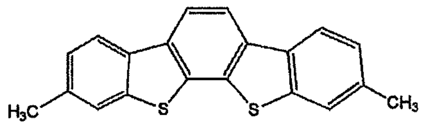

Comparative compound 2

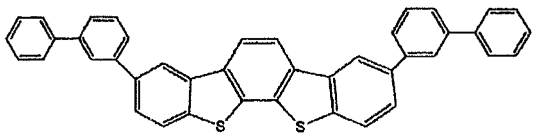

Comparative compound 3

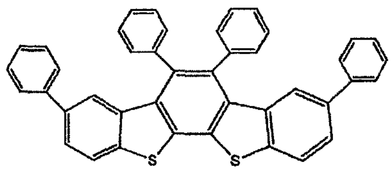

Comparative compound 4

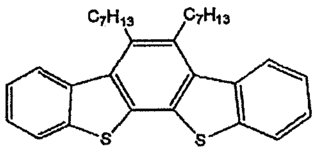

Comparative compound 5

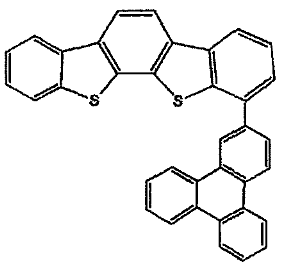

Comparative compound 6

<Preparation/evaluation of element>

All of the materials used for preparing elements were purified by sublimation. Through high-performance liquid chromatography (TSKgel ODS-100Z manufactured by TOSOH CORPORATION), it was confirmed that the materials had purity (area ratio for absorption intensity at 254 nm) of equal to or higher than 99.5%.

<Formation of semiconductor active layer (organic semiconductor layer) by using compound alone>

Each of the compounds of the present invention or the comparative compounds (1 mg each) was mixed with toluene (1 mL) and heated to 100°C, thereby obtaining a coating solution for a non-light-emitting organic semiconductor device. In a nitrogen atmosphere, the coating solution was cast onto a substrate for measuring FET characteristics that was heated to 90°C, thereby forming an organic semiconductor film for a non-light-emitting organic semiconductor device. In this way, an organic transistor element of Example 2 for measuring FET characteristics was obtained. As the substrate for measuring FET characteristics, a silicon substrate having a bottom gate/bottom contact structure was used which comprised chromium/gold (gate width W = 100 mm, gate length L = 100 $\mu$m) arranged to form a comb pattern as

source and drain electrodes and comprised $SiO_2$ (film thickness: 200 nm) as an insulating layer (the structure is schematically shown in Fig. 2).

[Evaluation]

(a) Carrier mobility

By using a semiconductor parameter analyzer (4156C manufactured by Agilent Technologies) connected to a semi-automatic prober (AX-2000 manufactured by Vector Semiconductor Co., Ltd.), the carrier mobility as one of the FET characteristics of the organic transistor element of each of the examples and comparative examples were evaluated under a normal pressure/nitrogen atmosphere.

Between the source electrode and the drain electrode of each organic film transistor element (FET element), a voltage of -80 V was applied, and the gate voltage was varied within a range of 20 V to -100 V. In this way, a carrier mobility $\mu$ was calculated using the following equation showing a drain current $I_d$.

$$I_d = (w/2L)\mu C_i(V_g - V_{th})^2$$

In the equation, L represents a gate length, W represents a gate width, $C_i$ represents a capacity of the insulating layer per unit area, $V_g$ represents a gate voltage, and $V_{th}$ represents a threshold voltage.

(b) Solubility

Each of the compounds of the present invention or comparative compounds (2% by mass, 1% by mass, 0.5% by mass, or 0.1% by mass each) was mixed with toluene (1 mL), heated to 100°C, and then left to stand for 30 minutes at room temperature. Thereafter, the concentration at which precipitation did not occur was determined, and the solubility in toluene was evaluated into 4 levels as below. For practical use, the compound needs to be evaluated to be A, B, C, or D. The compound preferably needs to be evaluated to be A, B, or C, more preferably needs to be evaluated to be A or B, and particularly preferably needs to be evaluated to be A.

A: Precipitation did not occur at 2% by mass.
B: Precipitation did not occur at 1% by mass but occurred at 2% by mass.
C: Precipitation did not occur at 0.5% by mass but occurred at 1% by mass.
D: Precipitation did not occur at 0.1% by mass but occurred at 0.5% by mass.
E: Precipitation occurred at 0.1% by mass.

(c) Threshold voltage shift after repeated driving (threshold shift)

Between the source electrode and the drain electrode of each organic transistor element (FET element), a voltage of -80 V was applied, and the element was repeatedly driven 100 times by varying the gate voltage within a range of +20 V to -100 V In this way, the element was measured in the same manner as in the section (a), and a difference between a threshold voltage $V_{before}$ before the repeated driving and a threshold voltage $V_{after}$ after the repeated driving ($|V_{after} - V_{before}|$) was evaluated into 5 levels as below. The smaller the difference, the higher the stability of the element against repeated driving. Therefore, the smaller the difference, the more preferable.

$$A: |V_{after} - V_{before}| \leq 1\ V$$

$$B: 1\ V < |V_{after} - V_{before}| \leq 5\ V$$

$$C: 5\ V < |V_{after} - V_{before}| \leq 10\ V$$

$$D: 10\ V < |V_{after} - V_{before}| \leq 15\ V$$

$$E: |V_{after} - V_{before}| > 15\ V$$

(d) Quality of coating film

The quality of the coating film was evaluated by the following method.

For the semiconductor active layer of the organic transistor element of Example 2, a domain size was calculated within a range of 1 $mm^2$ by using a polarization microscope, and the average domain size was calculated. The obtained result was evaluated into 3 levels as below. There is no problem with the practical use of an organic transistor element evaluated to be D. However, the organic transistor element preferably needs to be evaluated to be A, B, or C, more preferably needs to be evaluated to be A or B, and particularly preferably needs to be evaluated to be A.

A: The average domain size was greater than 10 μm.
B: The average domain size was greater than 5 μm but equal to or less than 10 μm.
C: The average domain size was greater than 1 μm but equal to or less than 5 μm.
D: The average domain size was greater than 0.5 μm but equal to or less than 1 μm.
E: The average domain size was equal to or less than 0.5 μm.

(e) Variation

Each element was prepared by 30, the mobility of the prepared 30 elements was measured, and the coefficient of variation was calculated. The result was evaluated into 5 levels as below.

For practical use, an element evaluated to be C is also acceptable. However, the element preferably needs to be evaluated to be A or B, and more preferably needs to be evaluated to be A.

A: Less than 30%
B: Equal to or greater than 30% but less than 50%
C: Equal to or greater than 50% but less than 100%
D: Equal to or greater than 100% but less than 200%
E: Equal to or greater than 200%

The respective results are summarized in the following table.

[Table 24]

Examples 12 and 20 are not part of the invention.

| | Used compound | Mobility | Solubility | Threshold shift | Quality of coating film | Variation |
|---|---|---|---|---|---|---|
| Example 1 | Compound example 3 | 0.58 | A | A | A | A |
| Example 2 | Compound example 6 | 0.77 | A | A | A | A |
| Example 3 | Compound example 10 | 0.32 | A | A | A | A |
| Example 4 | Compound example 407 | 0.58 | A | A | A | A |
| Example 5 | Compound example 20 | 0.7 | B | A | A | A |
| Example 6 | Compound example 24 | 1.1 | B | A | A | A |
| Example 7 | Compound example 298 | 0.42 | B | B | A | A |
| Example 8 | Compound example 310 | 0.28 | B | B | A | A |

(continued)

| Examples 12 and 20 are not part of the invention. | | | | | | |
|---|---|---|---|---|---|---|
| | Used compound | Mobility | Solubility | Threshold shift | Quality of coating film | Variation |
| Example 9 | Compound example 326 | 0.11 | B | B | A | A |
| Example 10 | Compound example 34 | 0.13 | B | B | B | A |
| Example 11 | Compound example 62 | 0.18 | B | B | B | A |
| Example 12 (representative) | Compound example 1 | 0.13 | B | B | C | B |
| Example 13 | Compound example 16 | 0.1 | B | B | C | B |
| Example 14 | Compound example 396 | 0.01 | B | B | C | B |
| Example 15 | Compound example 386 | 0.08 | B | C | C | C |
| Example 16 | Compound example 372 | 0.05 | B | C | C | C |
| Example 17 | Compound example 358 | $6.4 \times 10^{-3}$ | B | C | C | C |
| Example 18 | Compound example 344 | $8.6 \times 10^{-3}$ | B | C | C | C |
| Example 19 | Compound example 416 | $2.1 \times 10^{-3}$ | C | C | C | C |
| Example 20 (representative) | Compound example 427 | $2.4 \times 10^{-3}$ | D | C | C | C |
| Example 21 | Compound example 429 | $1.0 \times 10^{-4}$ | B | C | D | C |
| Comparative example 1 | Comparative compound 1 | $6.8 \times 10^{-5}$ | E | C | E | E |
| Comparative example 2 | Comparative compound 2 | $3.7 \times 10^{-4}$ | E | C | E | E |
| Comparative example 3 | Comparative compound 3 | No characteristics | E | C | C | D |
| Comparative example 4 | Comparative compound 4 | No characteristics | E | C | D | D |
| Comparative example 5 | Comparative compound 5 | No characteristics | D | C | D | D |
| Comparative example 6 | Comparative compound 6 | No characteristics | E | D | E | E |

From the results shown in the above table, it was understood that the compound of the present invention exhibits excellent solubility in an organic solvent, and the organic transistor element using the compound of the present invention has high carrier mobility. Therefore, it was understood that the compound of the present invention can be preferably used as an organic semiconductor material for a non-light-emitting organic semiconductor device.

Particularly, Examples 1 to 6 using a compound represented by Formula (1), in which X is a S atom, each of $R^2$ and $R^9$ is an alkyl group, and each of $R^1$, $R^3$ to $R^8$, and $R^{10}$ is a hydrogen atom, showed excellent results in each of the carrier mobility, solubility, threshold voltage shift (threshold shift) after repeated driving, quality of coating film, and variation.

In contrast, the organic transistor elements using the comparative compounds 3 to 6 had poor carrier mobility that could not be evaluated. Furthermore, the comparative compounds 1 to 4 and 6 exhibited poor solubility in an organic solvent.

The organic transistor element using the compound of the present invention showed only a slight threshold voltage shift (threshold shift) after repeated driving, was excellent in the quality of the coating film, showed a small variation, and had excellent driving stability.

[Examples 101 to 121 and Comparative examples 101 to 106]

<Formation of semiconductor active layer (organic semiconductor layer) by using compound and binder together>

Each of the compounds of the present invention or the comparative compounds (1 mg each) was mixed with 1 mg of PαMS (poly(α-methylstyrene, Mw = 300,000), manufactured by Sigma-Aldrich Co. LLC.) and toluene (1 mL), and the mixture was heated to 100°C, thereby obtaining a coating solution. Organic transistor elements of Examples 101 to 121 and Comparative examples 101 to 106 for measuring FET characteristics were prepared in the same manner as in Examples 1 to 21 and Comparative examples 1 to 6 except that the coating solution obtained as above was used, and evaluated in the same manner as in Examples 1 to 21 and Comparative examples 1 to 6.

As a result, it was understood that the organic transistor elements show the same trend as in Examples 1 to 21 and Comparative examples 1 to 6.

[Examples 201 to 221 and Comparative examples 201 to 206]

<Formation of semiconductor active layer (organic semiconductor layer)>

The surface of a silicon wafer, which comprised $SiO_2$ (film thickness: 370 nm) as a gate insulating film, was treated with octyltrichlorosilane.

Each of the compounds of the present invention or the comparative compounds (1 mg each) was mixed with toluene (1 mL), and the mixture was heated to 100°C, thereby preparing a coating solution for a non-light-emitting organic semiconductor device. In a nitrogen atmosphere, the coating solution was cast onto the silicon wafer which had been heated to 90°C and undergone surface treatment with octylsilane, thereby forming an organic semiconductor film for a non-light-emitting organic semiconductor device.

Furthermore, gold was deposited onto the surface of the film by using a mask so as to prepare source and drain electrodes, thereby obtaining organic transistor elements of Examples 201 to 221 and Comparative examples 201 to 206 having a bottom gate/top contact structure with a gate width W = 5 mm and a gate length L = 80 μm (the structure is schematically shown in Fig. 1).

By using a semiconductor parameter analyzer (4156C manufactured by Agilent Technologies) connected to a semi-automatic prober (AX-2000 manufactured by Vector Semiconductor Co., Ltd.), the FET characteristics of the organic transistor elements of Examples 201 to 221 and Comparative examples 201 to 206 were evaluated in the same manner as in Examples 1 to 21 and Comparative examples 1 to 6 under a normal pressure/nitrogen atmosphere.

As a result, it was understood that the organic transistor elements show the same trend as in Examples 1 to 21 and Comparative examples 1 to 6.

Explanation of References

11: substrate
12: electrode
13: insulator layer
14: semiconductor active layer (organic substance layer, organic semiconductor layer)
15a, 15b: electrode
31: substrate
32: electrode
33: insulator layer

34a, 34b: electrode
35: semiconductor active layer (organic substance layer, organic semiconductor layer)

## Claims

**1.** An organic transistor, **characterised in that** the organic transistor comprises

a compound represented by the following Formula (1) in a semiconductor active layer wherein said compound is a low-molecular weight compound or a polymer compound having a repeating structure in which the compound represented by Formula (1) represents at least one or more arylene groups or heteroarylene groups, or a pendant-type polymer in which the compound represented by Formula (1) is bonded to the main chain of the polymer via the side chain;

Formula (1)

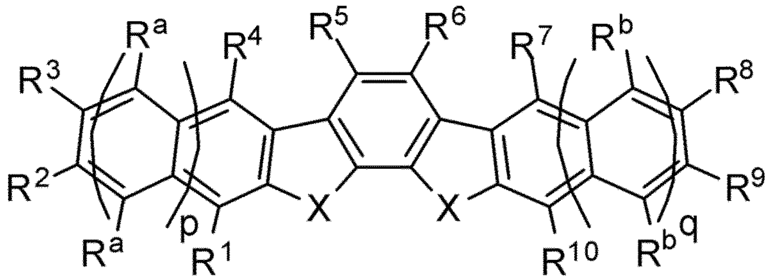

in Formula (1),
each X independently represents an O atom, a S atom, or a Se atom,
each of p and q independently represents an integer of 0 to 2,
each of $R^1$ to $R^{10}$, $R^a$, and $R^b$ independently represents a hydrogen atom, a halogen atom, or a substituent represented by the following Formula (W), at least one of $R^1$, $R^2$, $R^3$, $R^4$, $R^5$, $R^6$, $R^7$, $R^8$, $R^9$, $R^{10}$, $R^a$, or $R^b$ is a substituent represented by Formula (W), and in a case where at least one of $R^5$ or $R^6$ is a substituent represented by Formula (W), L in Formula (W) represented by $R^5$ and $R^6$ is a divalent linking group represented by the following Formula (L-2) or (L-3);

-L-R Formula (W)

in Formula (W),
L represents a divalent linking group represented by any one of the following Formulae (L-1) to (L-5), (L-13), (L-17) and (L-18) or a divalent linking group in which two or more divalent linking groups represented by any one of the following Formulae (L-1) to (L-5), (L-13), (L-17) and (L-18) are bonded to each other, and
R represents a substituted or unsubstituted alkyl group, a cyano group, a vinyl group, an ethynyl group, an oxyethylene group, an oligo-oxyethylene group in which a repetition number v of an oxyethylene unit is equal to or greater than 2, a siloxane group, an oligosiloxane group having two or more silicon atoms, or a substituted or unsubstituted trialkylsilyl group;

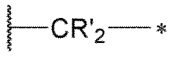 (L-1)

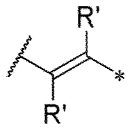 (L-2)

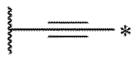 (L-3)

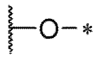 (L-4)

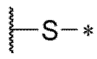 (L-5)

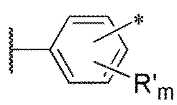 (L-13)

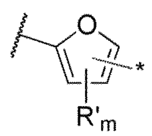 (L-17)

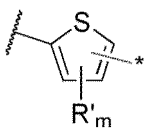 (L-18)

in Formulae (L-1) to (L-5), (L-13), (L-17) and (L-18),
the portion of a wavy line represents a position of bonding to the condensed cyclic skeleton,
m in Formula (L-13) represents 4, m in Formulae (L-17) and (L-18) represents 2,
each R' in Formulae (L-1), (L-2), (L-13), (L-17) and (L-18) independently represents a hydrogen atom, a halogen atom, an alkyl group, an alkenyl group, an alkynyl group, an aryl group, a hetero ring group, a cyano group, a hydroxyl group, a nitro group, an acyl group, an alkoxy group, an aryloxy group, a silyloxy group, a heterocyclic oxy group, an acyloxy group, a carbamoyloxy group, an amino group, an acylamino group, an aminocarbonylamino group, alkoxy- and aryloxycarbonylamino groups, alkyl- and aryl sulfonylamino groups, a mercapto group, alkyl- and arylthio groups, a heterocyclic thio group, a sulfamoyl group, a sulfo group, alkyl- and aryl sulfinyl groups, alkyl- and aryl sulfonyl groups, alkyloxy- and aryloxycarbonyl groups, a carbamoyl group, aryl- and heterocyclic azo group, an imide group, a phosphino group, a phosphinyl group, a phosphinyloxy group, a phosphinylamino group, a phosphono group, a silyl group, a hydrazino group, a ureide group, a boronic acid group ($-B(OH)_2$), a phosphate group ($-OPO(OH)_2$), a sulfate group ($-OSO_3H$),
in a case where p=q=0 and a substituent represented by Formula (W) is an alkyl group, the substituent represented by Formula (W) is a linear alkyl group having 4 to 18 carbon atoms or a branched alkyl group having 4 or more carbon atoms, and
* represents a position of bonding to any portion of a wavy line and R in a divalent linking group represented by any of the Formulas (L-1) to (L-5), (L-13), (L-17) and (L-18) ; in a case where L represents a divalent linking group in which two or more divalent linking groups represented by any one of Formulae (L-1) to (L-5), (L-13), (L-17) and (L-18) are bonded to each other, * represents a position of bonding to the portion of a wavy line of a divalent linking group represented by any of Formulae (L-1) to (L-5), (L-13), (L-17) and (L-18).

**2.** The organic transistor according to claim 1,

wherein in the Formula (1), each of $R^1$, $R^4$ to $R^7$, $R^{10}$, $R^a$, and $R^b$ is independently a hydrogen atom or a halogen atom,
each of $R^2$, $R^3$, $R^8$, and $R^9$ is independently a hydrogen atom, a halogen atom, or a substituent represented by the Formula (W), and at least one of $R^2$, $R^3$, $R^8$, or $R^9$ is a substituent represented by the Formula (W).

**3.** The organic transistor according to claim 1 or 2,
wherein in the Formula (1), p and q satisfy p = q = 0 or p = q = 1.

**4.** The organic transistor according to any one of claims 1 to 3,

wherein in the compound represented by the Formula (1) p = q = 0,
each of $R^1$, $R^3$ to $R^8$, and $R^{10}$ independently represents a hydrogen atom or a halogen atom,
each of $R^2$ and $R^9$ is independently a hydrogen atom, a halogen atom, or a substituent represented by the Formula (W), at least one of $R^2$ or $R^9$ is a substituent represented by Formula (W), and in a case where a substituent represented by Formula (W) is an alkyl group, the substituent represented by Formula (W) is limited to a linear alkyl group having 4 to 18 carbon atoms or a branched alkyl group having 4 or more carbon atoms;

-L-R Formula (W)

wherein in Formula (W) L and R are as defined in claim 1.

**5.** The organic transistor according to any one of claims 1 to 3,

wherein in the compound represented by the Formula (1) p = q = 1,
each of $R^1$, $R^4$ to $R^7$, $R^{10}$, $R^a$, and $R^b$ is independently a hydrogen atom or a halogen atom,
each of $R^2$, $R^3$, $R^8$, and $R^9$ independently represents a hydrogen atom, a halogen atom, or a substituent represented by the Formula (W), and at least one of $R^2$, $R^3$, $R^8$, or $R^9$ represents a substituent represented by Formula (W);

-L-R Formula (W)

wherein in Formula (W) L and R are as defined in claim 1.

**6.** A compound represented by the following Formula (2-1) or the following Formula (2-2):

Formula (2-1)

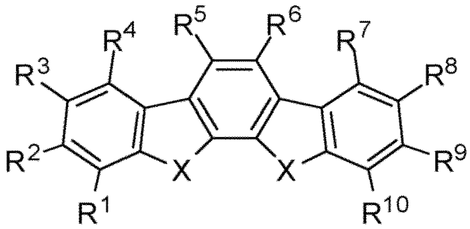

Formula (2-2)

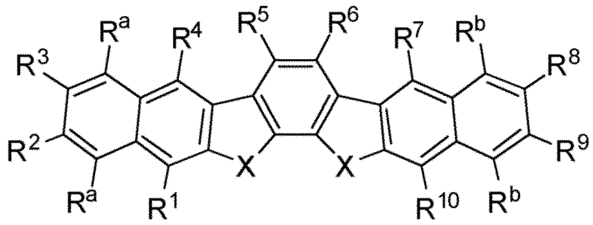

in Formula (2-1),
each X independently represents an O atom, a S atom, or a Se atom,
each of $R^1$, $R^3$ to $R^8$, and $R^{10}$ independently represents a hydrogen atom or a halogen atom,
each of $R^2$ and $R^9$ is independently a hydrogen atom, a halogen atom, or a substituent represented by the following Formula (W), at least one of $R^2$ or $R^9$ is a substituent represented by Formula (W), and in a case where a substituent represented by Formula (W) is an alkyl group, the substituent represented by Formula (W) is limited to a linear alkyl group having 4 to 18 carbon atoms or a branched alkyl group having 4 or more carbon atoms;

-L-R Formula (W)

wherein in Formula (W) L and R are as defined in claim 1,
in Formula (2-2),
each X independently represents an O atom, a S atom, or a Se atom,
each of $R^1$, $R^4$ to $R^7$, $R^{10}$, $R^a$, and $R^b$ is independently a hydrogen atom or a halogen atom,
each of $R^2$, $R^3$, $R^8$, and $R^9$ independently represents a hydrogen atom, a halogen atom, or a substituent represented by the following Formula (W), and at least one of $R^2$, $R^3$, $R^8$, or $R^9$ represents a substituent represented by Formula (W),

-L-R Formula (W)

wherein in Formula (W) L and R are as defined in claim 1.

**7.** The compound according to claim 6,
wherein in the Formulae (2-1) and (2-2), X is a S atom.

**8.** The compound according to any one of claims 6 to 7,

wherein in the Formulae (2-1) and (2-2), both of $R^2$ and $R^9$ are a substituent represented by the Formula (W), and both of $R^3$ and $R^8$ are a hydrogen atom or a halogen atom, or

both of $R^3$ and $R^8$ are a substituent represented by the Formula (W), and both of $R^2$ and $R^9$ are a hydrogen atom or a halogen atom.

**9.** An organic semiconductor material for a non-light-emitting organic semiconductor device or a material for an organic transistor or a coating solution for a non-light-emitting organic semiconductor device or an organic semiconductor film for a non-light-emitting organic semiconductor device, comprising the compound represented by the Formula (1) as defined in claim 1.

**Patentansprüche**

**1.** Organischer Transistor, **dadurch gekennzeichnet, dass** der organische Transistor eine durch die folgende Formel (1) dargestellte Verbindung in einer Halbleiter-Aktivschicht umfasst, worin die Verbindung eine niedermolekulare Verbindung oder eine Polymerverbindung mit einer sich wiederholenden Struktur ist, worin die durch die Formel (1) dargestellte Verbindung mindestens eine oder mehr Arylengruppen oder Heteroarylengruppen darstellt, oder ein Polymer vom anhängigen Typ ist, worin die durch die Formel (1) dargestellte Verbindung an die Hauptkette des Polymers über die Seitenkette gebunden ist;

Formel (1)

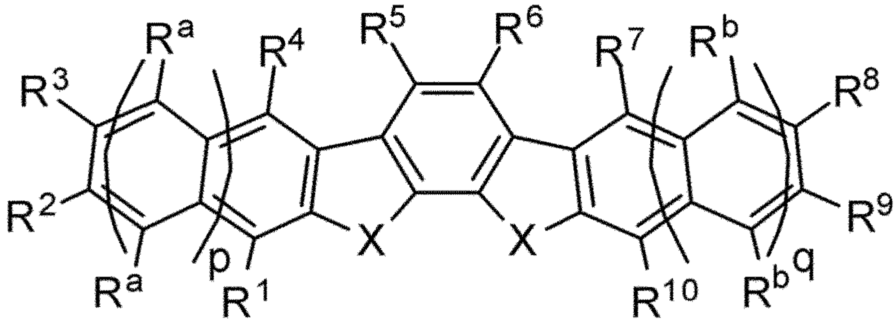

worin in Formel (1)
jedes X unabhängig ein O-Atom, ein S-Atom oder ein Se-Atom darstellt,
jedes von p und q unabhängig eine ganze Zahl von 0 bis 2 darstellt,
jedes von $R^1$ bis $R^{10}$, $R^a$ und $R^b$ unabhängig ein Wasserstoffatom, ein Halogenatom oder einen durch die folgende Formel (W) dargestellten Substituenten darstellt, wobei zumindest eines von $R^1$, $R^2$, $R^3$, $R^4$, $R^5$, $R^6$, $R^7$, $R^8$, $R^9$, $R^{10}$, $R^a$ oder $R^b$ ein durch die Formel (W) dargestellter Substituent ist, und wenn zumindest eines von $R^5$ oder $R^6$ ein durch die Formel (W) dargestellter Substituent ist, L in der durch $R^5$ und $R^6$ dargestellten Formel (W) eine divalente Verknüpfungsgruppe ist, die durch die folgende Formel (L-2) oder (L-3) dargestellt wird;

-L-R Formel (W)

worin in Formel (W)
L eine divalente Verknüpfungsgruppe, die durch irgendeine der folgenden Formeln (L-1) bis (L-5), (L-13), (L-17) und (L-18) dargestellt wird, oder eine divalente Verknüpfungsgruppe, worin zwei oder mehr der durch irgendeine der folgenden Formeln (L-1) bis (L-5), (L-13), (L-17) und (L-18) miteinander verbunden sind, darstellt, und
R eine substituierte oder unsubstituierte Alkylgruppe, eine Cyanogruppe, eine Vinylgruppe, eine Ethinylgruppe, eine Oxyethylengruppe, eine Oligo-Oxyethylengruppe, worin die Wiederholungszahl v einer Oxyethyleneinheit gleich oder größer als 2 ist, eine Siloxangruppe, eine Oligosiloxangruppe mit zwei oder mehr Siliciumatomen oder eine substituierte oder unsubstituierte Trialkylsilylgruppe darstellt;

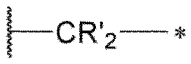

(L-1)

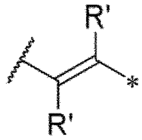

(L-2)

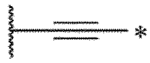

(L-3)

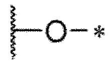

(L-4)

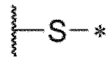

(L-5)

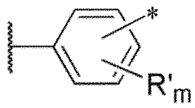

(L-13)

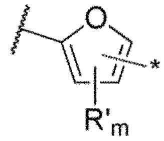

(L-17)

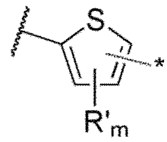

(L-18)

**worin in den Formeln** (L-1) **bis** (L-5), (L-13), (L-17) **und** (L-18) **der Teil einer gewellten Linie eine Bindungsposition an das kondensierte cyclische Gerüst darstellt,**
**m in Formel** (L-13) 4 **darstellt, m in Formel** (L-17) **und** (L-18) 2 **darstellt,**
**jedes R' in den Formeln** (L-1), (L-2), (L-13), (L-17) **und** (L-18) unabhängig en Wasserstoffatom, ein Halogenatom, eine Alkylgruppe, eine Alkenylgruppe , eine Alkinylgruppe, eine Arylgruppe, eine Heteroringgruppe, eine Cyanogruppe, eine Hydroxylgruppe, eine Nitrogruppe, eine Acylgruppe, eine Alkoxygruppe, eine Aryloxygruppe, eine Silyloxygruppe, eine heterocyclische Oxygruppe, eine Acyloxygruppe, eine Carbamoyloxygruppe, eine Aminogruppe, eine Acylaminogruppe, eine Aminocarbonylaminogruppe, Alkoxy- und Aryloxycarbonylaminogruppen, Alkyl- und Arylsulfonylaminogruppen, eine Mercaptogruppe, Alkyl- und Arylthiogruppen, eine heterocyclische Thiogruppe, eine Sulfamoylgruppe, eine Sulfogruppe, Alkyl- und Arylsulfinylgruppen, Alkyl- und Arylsulfonylgruppen, Alkyloxy- und Aryloxycarbonylgruppen, eine Carbamoylgruppe, Aryl- und heterocyclische Azogruppe, eine Imidgruppe, eine Phosphinogruppe, eine Phosphinylgruppe, eine Phosphinyloxygruppe, eine Phosphinylaminogruppe, eine Phosphonogruppe, eine Silylgruppe, eine Hydrazinogruppe, eine Ureidgruppe, eine Boronsäuregruppe ($-B(OH)_2$), eine Phosphatgruppe ($-OPO(OH)_2$), eine Sulfatgruppe ($-OSO_3H$) darstellt, wenn p=q=0 ist und ein durch die Formel (W) dargestellter Substituent eine Alkylgruppe ist, der durch die Formel (W) dargestellte Substituent eine lineare Alkylgruppe mit 4 bis 18 Kohlenstoffatomen oder eine verzweigte Alkylgruppe mit 4 oder mehr Kohlenstoffatomen ist, und
* eine Bindungsposition an irgendeinen Teil einer gewellten Linie und R in einer divalenten Verknüpfungsgruppe darstellt, die durch irgendeine der Formeln (L-1) bis (L-5), (L-13), (L-17) und (L-18) dargestellt wird; wenn L eine divalente Verknüpfungsgruppe darstellt, worin zwei oder mehr divalente Verknüpfungsgruppen miteinander verbunden sind, die durch irgendeine der Formeln (L-1) bis (L-5), (L-13), (L-17) und (L-18) dargestellt werden,
* eine Bindungsposition an den Teil einer gewellten Linie einer divalenten Verknüpfungsgruppe darstellt, die durch irgendeine der Formeln (L-1) bis (L-5), (L-13), (L-17) und (L-18) dargestellt wird.

**2.** Organischer Transistor gemäß Anspruch 1,

worin in der Formel 1 jedes von $R^1$, $R^4$ bis $R^7$,$R^{10}$, $R^a$ und $R^b$ unabhängig ein Wasserstoffatom oder ein Halogenatom ist,
jedes von $R^2$, $R^3$, $R^8$ und $R^9$ unabhängig ein Wasserstoffatom, ein Halogenatom oder ein durch die Formel (W) dargestellter Substituent ist, und zumindest eines von $R^2$, $R^3$, $R^8$ oder $R^9$ ein durch die Formel (W) dargestellter Substituent ist.

**3.** Organischer Transistor gemäß Anspruch 1 oder 2,
worin in der Formel (1) p und q p=q=0 oder p=q=1 erfüllen.

**4.** Organischer Transistor gemäß irgendeinem der Ansprüche 1 bis 3,

worin in der durch die Formel (1) dargestellten Verbindung p=q=0 ist,
jedes von $R^1$, $R^3$ bis $R^8$ und $R^{10}$ unabhängig ein Wasserstoffatom oder ein Halogenatom darstellt,
jedes von $R^2$ und $R^9$ unabhängig ein Wasserstoffatom, ein Halogenatom oder einen durch die Formel (W)

dargestellten Substituenten darstellt, wobei zumindest eines von $R^2$ oder $R^9$ ein durch Formel (W) dargestellter Substituent ist, und wenn ein durch die Formel (W) dargestellter Substituent eine Alkylgruppe ist, der durch die Formel (W) dargestellte Substituent auf eine lineare Alkylgruppe mit 4 bis 18 Kohlenstoffatomen oder eine verzweigte Alkylgruppe mit 4 oder mehr Kohlenstoffatomen beschränkt ist;

-L-R Formel (W)

worin in der Formel (W) L und R wie in Anspruch 1 definiert sind.

**5.** Organischer Transistor gemäß irgendeinem der Ansprüche 1 bis 3,

worin in der durch die Formel (1) dargestellte Verbindung p=q=1 ist,
jedes von $R^1$, $R^4$ bis $R^7$, $R^{10}$, $R^a$ und $R^b$ unabhängig ein Wasserstoffatom oder ein Halogenatom ist,
jedes von $R^2$, $R^3$, $R^8$ und $R^9$ unabhängig ein Wasserstoffatom, ein Halogenatom oder einen durch die Formel (W) dargestellten Substituent darstellt und zumindest eines von $R^2$, $R^3$, $R^8$ oder $R^9$ einen durch die Formel (W) dargestellten Substituent darstellt;

-L-R Formel (W)

worin in der Formel (W) L und R wie in Anspruch 1 definiert sind.

**6.** Verbindung, dargestellt durch die folgende Formel (2-1) oder die folgende Formel (2-2):

Formel (2-1)

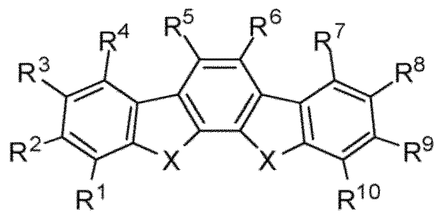

Formel (2-2)

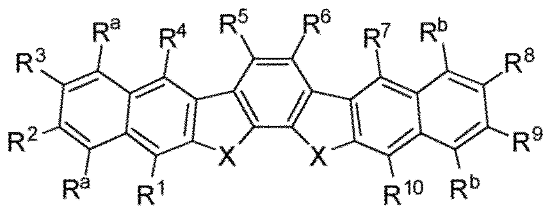

worin in Formel (2-1)
jedes X unabhängig ein O-Atom, ein S-Atom oder ein Se-Atom darstellt,
jedes von $R^1$, $R^3$ bis $R^8$ und $R^{10}$ unabhängig ein Wasserstoffatom, ein Halogenatom darstellt,
jedes von $R^2$ und $R^9$ unabhängig ein Wasserstoffatom, ein Halogenatom oder einen durch die folgende Formel (W) dargestellten Substituenten darstellt, wobei zumindest eines von $R^2$ und $R^9$ ein durch die Formel (W) dargestellter Substituent ist, und wenn ein durch die Formel (W) dargestellter Substituent eine Alkylgruppe ist, der durch die Formel (W) dargestellte Substituent auf eine lineare Alkylgruppe mit 4 bis 18 Kohlenstoffatomen oder eine verzweigte Alkylgruppe mit 4 oder mehr Kohlenstoffatomen beschränkt ist;

-L-R Formel (W)

worin in Formel (W) L und R wie in Anspruch 1 definiert sind,
wobei in Formel (2-2)
jedes X unabhängig ein O-Atom, ein S-Atom oder ein Se-Atom darstellt,
jedes von $R^1$, $R^4$ bis $R^7$, $R^{10}$, $R^a$ und $R^b$ unabhängig ein Wasserstoffatom oder ein Halogenatom ist,
jedes von $R^2$, $R^3$, $R^8$ und $R^9$ unabhängig ein Wasserstoffatom, ein Halogenatom oder einen durch die folgende Formel (W) dargestellten Substituenten darstellt, und zumindest eines von $R^2$, $R^3$, $R^8$ oder $R^9$ einen durch die Formel (W) dargestellten Substituenten darstellt,

-L-R Formel (W)

worin in Formel (W) L und R wie in Anspruch 1 definiert sind.

**7.** Verbindung gemäß Anspruch 6,

worin in der Formel (2-1) und (2-2) X ein S-atom ist.

**8.** Verbindung gemäß irgendeinem der Ansprüche 6 bis 7,

worin in den Formeln (2-1) und (2-2) beide von $R^2$ und $R^9$ ein durch die Formel (W) dargestellter Substituent sind und beide von $R^3$ und $R^8$ ein Wasserstoffatom oder ein Halogenatom sind;
oder
beide von $R^3$ und $R^8$ ein durch die Formel (W) dargestellter Substituent sind und beide von $R^2$ und $R^9$ ein Wasserstoffatom oder ein Halogenatom sind.

**9.** Organisches Halbleitermaterial für eine nicht-lichtemittierende organische Halbleitervorrichtung oder Material für einen organischen Transistor oder Beschichtungslösung für eine nicht-lichtemittierende organische Halbleiervorrichtung oder einen organischen Halbleiterfilm für eine nicht-lichtemittierende organische Halbleitervorrichtung, umfassend die wie in Anspruch 1 definierte, durch die Formel (1) dargestellte Verbindung.

## Revendications

**1.** Transistor organique, **caractérisé en ce que** le transistor organique comprend un composé représenté par la formule (1) suivante dans une couche active semi-conductrice, dans lequel ledit composé est un composé à faible poids moléculaire ou un composé polymère présentant une structure de répétition dans laquelle le composé représenté par la formule (1) représente au moins un ou plusieurs groupes arylène ou groupes hétéroarylène, ou un polymère de type pendant dans lequel le composé représenté par la formule (1) est lié à la chaîne principale du polymère via la chaîne latérale ;

Formule (1)

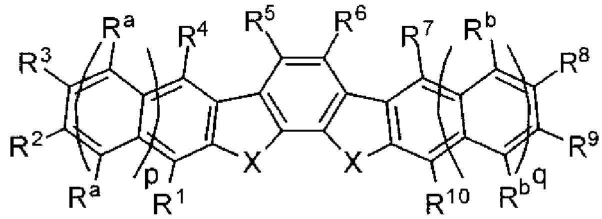

dans la formule (1),
chaque X représente indépendamment un atome O, un atome S ou un atome Se,
chacun des p et q représente indépendamment un nombre entier de 0 à 2,
chacun des $R^1$ à $R^{10}$, $R^a$ et $R^b$ représente indépendamment un atome d'hydrogène, un atome d'halogène ou un substituant représenté par la formule (W) suivante, au moins un parmi $R^1$, $R^2$, $R^3$, $R^4$, $R^5$, $R^6$, $R^7$, $R^8$, $R^9$, $R^{10}$, $R^a$ ou $R^b$ est un substituant représenté par la formule (W), et dans un cas où au moins un parmi $R^5$ ou $R^6$ est un substituant représenté par la formule (W), L dans la formule (W) représenté par $R^5$ et $R^6$ est un groupe de liaison bivalente représenté par la formule (L-2) ou (L-3) suivante ;

-L-R formule (W)

dans la formule (W),
L représente un groupe de liaison bivalente représenté par l'une quelconque des formules (L-1) à (L-5), (L-13), (L-17) et (L-18) suivante ou un groupe de liaison bivalente dans lequel deux groupes de liaison bivalente ou plus représentés par l'une quelconque des formules (L-1) à (L-5), (L-13), (L-17) et (L-18) suivantes sont liés entre eux, et
R représente un groupe alkyle substitué ou non substitué, un groupe cyano, un groupe vinyle, un groupe éthynyle, un groupe oxyéthylène, un groupe oligo-oxyéthylène dans lequel un nombre de répétitions v d'une unité d'oxyéthylène est supérieur ou égal à 2, un groupe siloxane, un groupe oligosiloxane présentant deux atomes de silicium ou plus, ou un groupe trialkylsilyle substitué ou non substitué ;

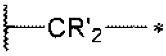
(L-1)

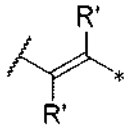
(L-2)

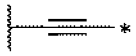
(L-3)

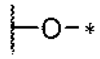
(L-4)

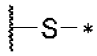
(L-5)

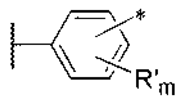
(L-13)

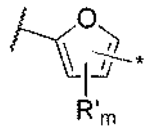
(L-17)

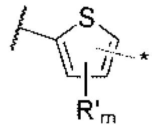
(L-18)

dans les formules (L-1) à (L-5), (L-13), (L-17) et (L-18),
la portion d'une ligne ondulée représente une position de liaison au squelette cyclique condensé,
m dans la formule (L-13) représente 4, m dans les formules (L-17) et (L-18) représente 2,
chaque R' dans les formules (L-1), (L-2), (L-13), (L-17) et (L-18) représente indépendamment un atome d'hydrogène, un atome d'halogène, un groupe alkyle, un groupe alkényle, un groupe alkynyle, un groupe aryle, un groupe à hétérochaîne, un groupe cyano, un groupe hydroxyle, un groupe nitro, un groupe acyle, un groupe alcoxy, un groupe aryloxy, un groupe silyloxy, un groupe oxy hétérocyclique, un groupe acyloxy, un groupe carbamoyloxy, un groupe amino, un groupe acylamino, un groupe aminocarbonylamino, des groupes alcoxy- et aryloxycarbonylamino, des groupes alkyle- et aryle sulfonylamino, un groupe mercapto, des groupes alkyle- et arylthio, un groupe thio hétérocyclique, un groupe sulfamoyle, un groupe sulfo, des groupes alkyle- et aryle sulfinyle, des groupes alkyle- et aryle sulfonyle, des groupes alkyloxy- et aryloxycarbonyle, un groupe carbamoyle, un groupe aryle- et azo hétérocyclique, un groupe imide, un groupe phosphino, un groupe phosphinyle, un groupe phosphinyloxy, un groupe phosphinylamino, un groupe phosphono, un groupe silyle, un groupe hydrazino, un groupe uréide, un groupe acide boronique ($-B(OH)_2$), un groupe phosphate ($-OPO(OH)_2$), un groupe sulfate ($-OSO_3H$),
dans un cas où p=q=0 et un substituant représenté par la formule (W) est un groupe alkyle, le substituant représenté par la formule (W) est un groupe alkyle linéaire présentant 4 à 18 atomes de carbone ou un groupe alkyle ramifié présentant 4 atomes de carbone ou plus,
* représente une position de liaison à toute portion d'une ligne ondulée et R dans un groupe de liaison bivalente représenté par l'une quelconque des formules (L-1) à (L-5), (L-13), (L-17) et (L-18) ; dans un cas où L représente un groupe de liaison bivalente dans lequel deux groupes de liaison bivalente ou plus représentés par l'une quelconque des formules (L-1) à (L-5), (L-13), (L-17) et (L-18) sont liés entre eux, * représente une position de liaison à la portion d'une ligne ondulée d'un groupe de liaison bivalente représenté par l'une quelconque des formules (L-1) à (L-5), (L-13), (L-17) et (L-18).

**2.** Transistor organique selon la revendication 1,

dans lequel dans la formule (1), chacun des $R^1$, $R^4$ à $R^7$, $R^{10}$, $R^a$ et $R^b$ est indépendamment un atome d'hydrogène ou un atome d'halogène,
chacun des $R^2$, $R^3$, $R^8$ et $R^9$ est indépendamment un atome d'hydrogène, un atome d'halogène ou un substituant représenté par la formule (W), et au moins un parmi $R^2$, $R^3$, $R^8$ ou $R^9$ est un substituant représenté par la formule (W).

**3.** Transistor organique selon la revendication 1 ou revendication 2,
dans lequel dans la formule (1), p et q satisfont p = q = 0 ou p = q = 1.

**4.** Transistor organique selon l'une quelconque des revendications 1 à 3,

dans lequel dans le composé représenté par la formule (1) p = q = 0,
chacun des $R^1$, $R^3$ à $R^8$ et $R^{10}$ représente indépendamment un atome d'hydrogène ou un atome d'halogène,
chacun des $R^2$ et $R^9$ est indépendamment un atome d'hydrogène, un atome d'halogène ou un substituant

représenté par la formule (W), au moins un parmi $R^2$ ou $R^9$ est un substituant représenté par la formule (W), et dans un cas où un substituant représenté par la formule (W) est un groupe alkyle, le substituant représenté par la formule (W) est limité à un groupe alkyle linéaire présentant 4 à 18 atomes de carbone ou un groupe alkyle ramifié présentant 4 atomes de carbone ou plus ;

-L-R formule (W)

dans lequel dans la formule (W) L et R sont tels que définis dans la revendication 1.

**5.** Transistor organique selon l'une quelconque des revendications 1 à 3,

dans lequel dans le composé représenté par la formule (1) p = q = 1,
chacun des $R^1$, $R^4$ à $R^7$, $R^{10}$, $R^a$ et $R^b$ est indépendamment un atome d'hydrogène ou un atome d'halogène,
chacun des $R^2$, $R^3$, $R^8$ et $R^9$ représente indépendamment un atome d'hydrogène, un atome d'halogène ou un substituant représenté par la formule (W), et au moins un parmi $R^2$, $R^3$, $R^8$ ou $R^9$ représente un substituant représenté par la formule (W) ;

-L-R formule (W)

dans lequel dans la formule (W) L et R sont tels que définis dans la revendication 1.

**6.** Composé représenté par la formule (2-1) suivante ou la formule (2-2) suivante :

Formule (2-1)

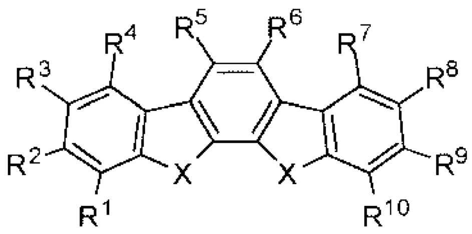

Formule 2-2)

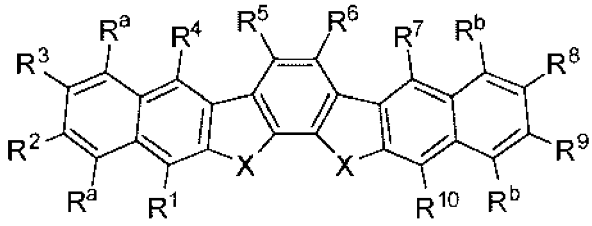

dans la formule (2-1),
chaque X représente indépendamment un atome O, un atome S ou un atome Se,
chacun des $R^1$, $R^3$ à $R^8$ et $R^{10}$ représente indépendamment un atome d'hydrogène ou un atome d'halogène,
chacun des $R^2$ et $R^9$ est indépendamment un atome d'hydrogène, un atome d'halogène ou un substituant représenté par la formule (W) suivante, au moins un parmi $R^2$ ou $R^9$ est un substituant représenté par la formule (W), et dans un cas où un substituant représenté par la formule (W) est un groupe alkyle, le substituant représenté par la formule (W) est limité à un groupe alkyle linéaire présentant 4 à 18 atomes de carbone ou un groupe alkyle ramifié présentant 4 atomes de carbone ou plus ;

-L-R formule (W)

dans lequel dans la formule (W) L et R sont tels que définis dans la revendication 1,
dans la formule (2-2),
chaque X représente indépendamment un atome O, un atome S ou un atome Se,
chacun des $R^1$, $R^4$ à $R^7$, $R^{10}$, $R^a$ et $R^b$ est indépendamment un atome d'hydrogène ou un atome d'halogène,
chacun des $R^2$, $R^3$, $R^8$ et $R^9$ représente indépendamment un atome d'hydrogène, un atome d'halogène ou un substituant représenté par la formule (W) suivante, et au moins un parmi $R^2$, $R^3$, $R^8$ ou $R^9$ représente un substituant représenté par la formule (W),

-L-R formule (W)

dans lequel dans la formule (W) L et R sont tels que définis dans la revendication 1.

**7.** Composé selon la revendication 6,

dans lequel dans les formules (2-1) et (2-2), X est un atome S.

**8.** Composé selon l'une quelconque des revendications 6 à 7,

dans lequel dans les formules (2-1) et (2-2), $R^2$ et $R^9$ sont tous les deux un substituant représenté par la formule (W), et $R^3$ et $R^8$ sont tous les deux un atome d'hydrogène ou un atome d'halogène, ou
$R^3$ et $R^8$ sont tous les deux un substituant représenté par la formule (W), et $R^2$ et $R^9$ sont tous les deux un atome d'hydrogène ou un atome d'halogène.

**9.** Matériau semi-conducteur organique pour un dispositif à semi-conducteur organique non électroluminescent ou matériau pour un transistor organique ou une solution de revêtement pour un dispositif à semi-conducteur organique non électroluminescent ou un film semi-conducteur organique pour un dispositif à semi-conducteur organique non électroluminescent, comprenant le composé représenté par la formule (1) tel que définie dans la revendication 1.

FIG. 1

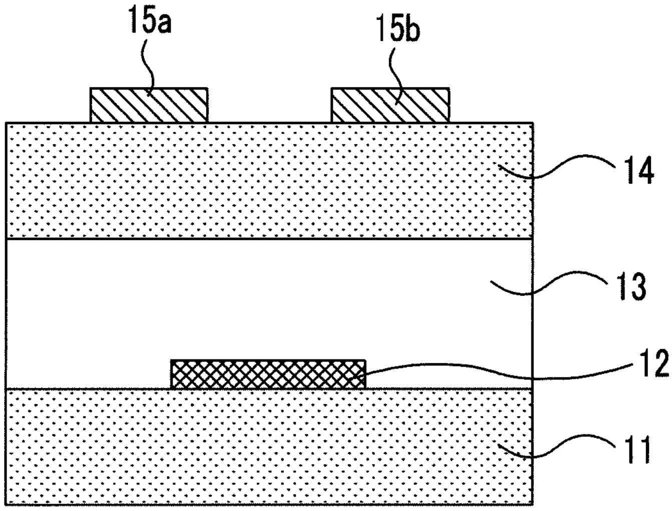


FIG. 2

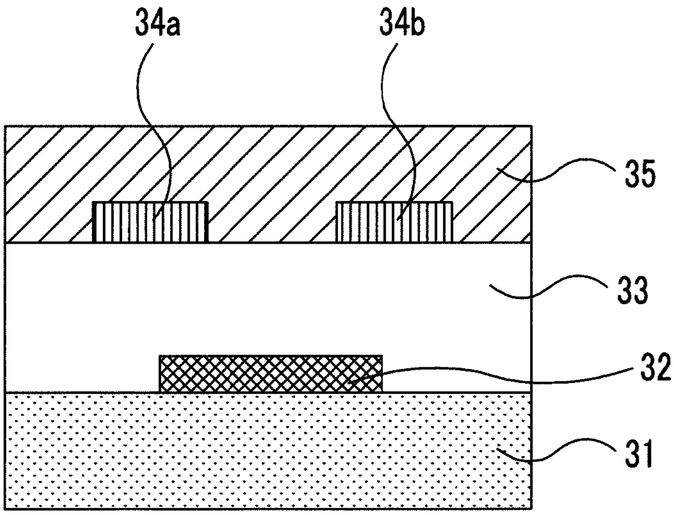

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2251342 A1
- EP 2301926 A1
- EP 2301921 A1
- KR 1020120120886 A
- US 2008220285 A
- US 2012138915 A
- JP 2009190999 A
- KR 20120120886 A


### Non-patent literature cited in the description


- **RAO, TILAK.** *Journal of Scientific and Industrial Research,* 1958, vol. 17 B, 260-265
- **KUDO, HIROTAKA ; TEDJAMULIA, MARVIN L ; CASTLE, RAYMOND N. ; LEE, MILTON L.** *Journal of Heterocyclic Chemistry,* 1984, vol. 21, 185-192
- *Tetrahedron Lett.,* 1973, vol. 17, 1561
- **KUDO, HIROTAKA ; TEDJAMULIA, MARVIN L. ; CASTLE, RAYMOND N. ; LEE, MILTON L.** *Journal of Heterocyclic Chemistry,* 1984, vol. 21, 185-192